# EUROPEAN PATENT APPLICATION

(11) **EP 4 484 414 A1**
(43) Date of publication of application: **01.01.2025**
(21) Application number: 23759167.2
(22) Date of filing: 21.02.2023
(51) Int. Cl.: C07D 267/10, C07D 413/12, A61K 31/553, A61P 11/00

(54) **SALT AND CRYSTAL FORM OF DIPEPTIDYL PEPTIDASE INHIBITOR COMPOUND**

(30) Priority: 22.02.2022 CN 202210160862
(71) Applicant: Haisco Pharmaceuticals Pte. Ltd., Singapore 079903 (SG)
(72) Inventor: FAN, Jiang, Chengdu, Sichuan 611130 (CN); DOU, Ying, Chengdu, Sichuan 611130 (CN); GONG, Zheng, Chengdu, Sichuan 611130 (CN); ZHU, Fengfei, Chengdu, Sichuan 611130 (CN)
(74) Representative: Bianchetti & Minoja with Trevisan & Cuonzo IPS SRL
(86) International application number: PCT/CN2023/077409
(87) International publication number: WO 2023/160542

(57) **Abstract**

Disclosed are a crystal of the compound (S)-N-((S)-1-cyano-2-(2-fluoro-4-(3-methyl-2-oxo-2,3-dihydrobenzo[d]oxazol-5-yl)phenyl)ethyl)-1,4-oxazacycloheptane-2-carboxamide salt or a salt thereof, a preparation method therefor, and the uses thereof in pharmaceutical compositions and in medicine.

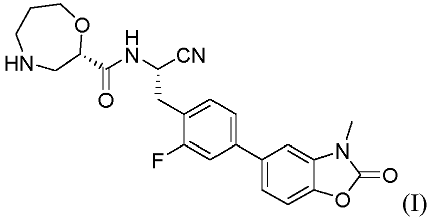

## Description

### Technical field

The present invention relates to a dipeptidyl peptidase inhibitor compound (S)-N-((S)-1-cyano-2-(2-fluoro-4-(3-methyl-2-oxo-2,3-dihydrobenzo[d]oxazol-5-yl)phenyl)ethyl)-1 ,4-oxazepane-2-carboxamide pharmaceutically acceptable salts or hydrates, solvates; their crystalline forms, preparation methods or pharmaceutical compositions thereof and their use in preparing small molecule inhibitor drugs of dipeptidyl peptidase 1 (DPP1).

### Background art

Dipeptidyl peptidase 1 (DPP1), also known as cathepsin C, is a cysteinyl protease of the lysosomal papain family and is involved in intracellular protein degradation. During the maturation process of neutrophils, DPP1 activates neutrophil serine proteases (NSPs), including neutrophil elastase (NE), proteinase 3 (Pr3) and cathepsin G (CatG), by cleaving the N-terminal dipeptides of target proteins. DPP1 is associated with a variety of inflammatory diseases, including Wegener's granulomatosis, rheumatoid arthritis, lung inflammation, and viral infections, etc. Studies have shown that inhibiting DPP1 can have a good therapeutic effect on highly inflammatory lung diseases caused by neutrophils, such as bronchiectasis, chronic obstructive pulmonary disease (COPD), acute lung injury, etc. Therefore, by targeting DPP1 and inhibiting the excessive activation of NSPs, it may have a potential therapeutic effect on bronchiectasis.

### Contents of invention

The present invention provides salts of the following structure (marked as Compound A), and hydrates, solvates of the salts thereof, and crystalline forms of the salts thereof, as well as in the preparation of medicaments or compositions thereof, 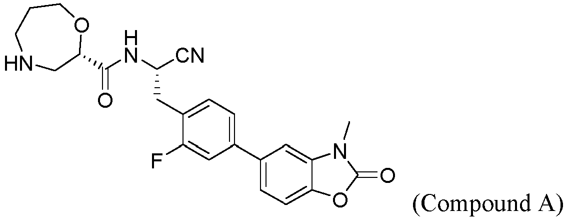 the salts of Compound A and the hydrates of the salts, the solvates of the salts and the crystalline forms of the salts have better solubility and stability than the free base compound, can exist very stably in the diluent (solvent), and are resistant to high temperature, high wet and strong light, suitable for the preparation of pharmaceutical dosage forms. At the same time, they have better pharmacokinetics and bioavailability than the free base compound.

Specifically, the present invention provides salts of the compound represented by formula (I), and hydrates and solvates thereof:

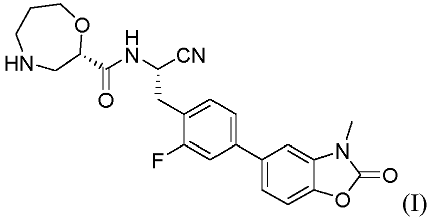

The salt is selected from the group consisting of hydrochloride, sulfate, maleate, phosphate, mucate, tartrate, fumarate, citrate, malate, hippurate, adipate, sebacate, 1,5-naphthalenedisulfonate, methanesulfonate, benzenesulfonate, oxalate, benzoate, hydrobromide, 2-naphthalenesulfonate, p-toluenesulfonate, hemi-1,5-naphthalenedisulfonate, succinate.

Further, the salt of the compound of formula (I) is selected from the group consisting of hydrochloride, sulfate, maleate, phosphate, mucate, tartrate, fumarate, citrate, malate, hippurate, adipate, sebacate, 1,5-naphthalenedisulfonate, methanesulfonate, benzenesulfonate, oxalate, benzoate, hydrobromide, preferably in crystalline form.

Furthermore, the salt of the compound of formula (I) is selected from the group consisting of hydrochloride, malate and adipate, preferably in crystalline form.

The present invention also relates to a hydrochloride of the compound of formula (I), which is in crystalline form (crystalline form A of the hydrochloride), and its X-ray powder diffraction pattern has characteristic diffraction peaks at the following 2θ positions: 9.38°±0.2°, 16.40°±0.2°, 18.69°±0.2°, 22.04°±0.2°, 23.05°±0.2°, 23.90°±0.2°, using Cu-Kα radiation.

Further, the present invention provides the crystalline form A of the hydrochloride of the compound of formula (I), and its X-ray powder diffraction pattern also has characteristic diffraction peaks at the following 2θ positions: 13.99°±0.2°, 14.75°±0.2°, 17.92°±0.2°, 25.70°±0.2°, 30.32°±0.2°, using Cu-Kα radiation.

Furthermore, the present invention provides the crystalline form A of the hydrochloride of the compound of formula (I), and its X-ray powder diffraction pattern also has a characteristic diffraction peak at the following 2θ position: 30.32°±0.2°.

The present invention provides the crystalline form A of the hydrochloride of the compound of formula (I), and the 2θ values and corresponding intensity in its X-ray powder diffraction pattern are shown in Table 1, and the error range of 2θ is ±0.2°.

**Table 1: 2θ values and corresponding intensity in XRPD pattern of the crystalline form A of the hydrochloride of the compound of formula (I)**

| Pos. [°2θ] | Height [cts] | FWHM Left [°2θ] | d-spacing [Å] | Rel. Int. [%] |
|---|---|---|---|---|
| 9.38 | 55.85 | 0.4093 | 9.43 | 52.05 |
| 13.99 | 21.80 | 0.3070 | 6.33 | 20.32 |
| 14.75 | 49.11 | 0.1535 | 6.01 | 45.76 |
| 16.40 | 107.30 | 0.1279 | 5.40 | 100.00 |
| 17.92 | 21.92 | 0.3070 | 4.95 | 20.43 |
| 18.69 | 61.42 | 0.1535 | 4.75 | 57.24 |
| 22.04 | 53.67 | 0.1535 | 4.03 | 50.02 |
| 23.05 | 65.75 | 0.2558 | 3.86 | 61.27 |
| 23.90 | 50.36 | 0.1535 | 3.72 | 46.93 |
| 25.70 | 48.26 | 0.2558 | 3.47 | 44.98 |
| 28.87 | 9.44 | 0.6140 | 3.09 | 8.80 |
| 30.32 | 12.54 | 0.3070 | 2.95 | 11.68 |

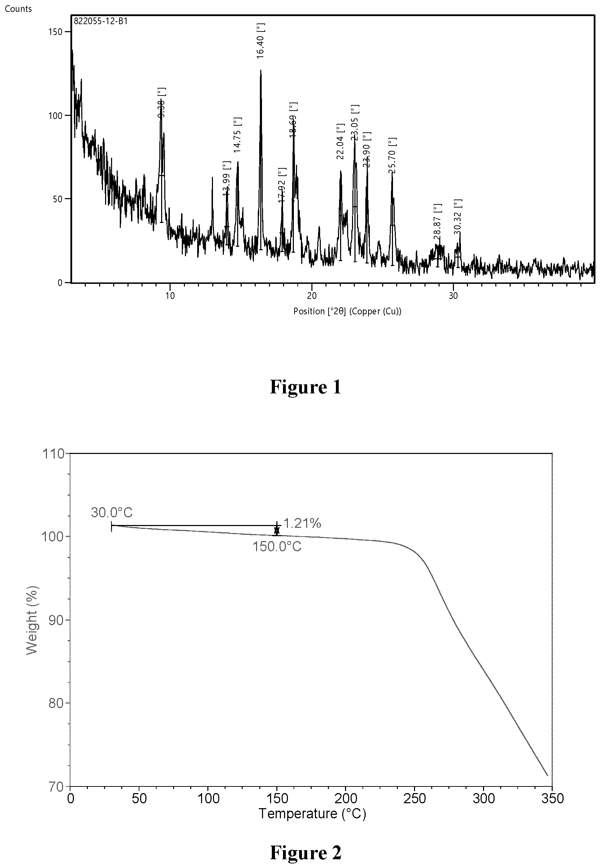

Further, the present invention provides the crystalline form A of the hydrochloride of the compound of formula (I), and its X-ray powder diffraction pattern is substantially shown in Figure 1.

The present invention provides the crystalline form A of the hydrochloride of the compound of formula (I), and its differential scanning calorimetry (DSC) shows an endothermic curve, in which Tₛₜₐᵣₜ = 219.5°C, Tₚₑₐₖ = 225.5°C, and ΔH = 12.50 J/g.

The present invention provides the crystalline form A of the hydrochloride of the compound of formula (I), and its differential scanning calorimetry (DSC) shows that its melting point is 225.5°C.

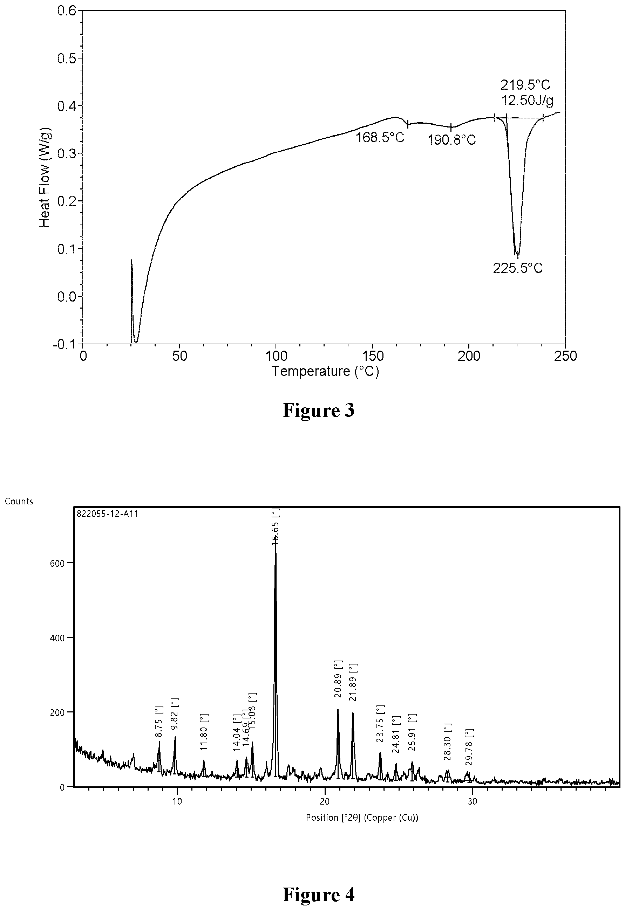

The present invention provides the crystalline form A of the hydrochloride of the compound of formula (I), and its differential scanning calorimetry (DSC) thermogram is shown in Figure 3.

The present invention provides the crystalline form A of the hydrochloride of the compound of formula (I), and its thermogravimetric analysis (TGA) thermogram shows 1.21% weight loss below 150°C, and the decomposition temperature is 250°C.

The present invention provides the crystalline form A of the hydrochloride of the compound of formula (I), and its thermogravimetric analysis (TGA) thermogram is shown in Figure 2.

The present invention provides the crystalline form A of the hydrochloride of the compound of formula (I), which is an anhydrate.

The present invention also provides a sulfate of the compound of formula (I), which is in crystalline form (crystalline form A of the sulfate), and its X-ray powder diffraction pattern has characteristic diffraction peaks at the following 2θ positions: 6.00°±0.2°, 9.72°±0.2°, 14.53°±0.2°, 15.25°±0.2°, using Cu-Kα radiation.

Further, the present invention provides the crystalline form A of the sulfate of the compound of formula (I), and its X-ray powder diffraction pattern also has characteristic diffraction peaks at the following 2θ positions: 12.70°±0.2°, 8.36°±0.2°, using Cu-Kα radiation.

The present invention provides the crystalline form A of the sulfate of the compound of formula (I), and the 2θ values and corresponding intensity in its X-ray powder diffraction pattern are shown in Table 2, and the error range of 2θ is ±0.2°.

**Table 2: 2θ values and corresponding intensity in XRPD pattern of the crystalline form A of the sulfate of the compound of formula (I)**

| Pos. [°2θ] | Height [cts] | FWHM Left [°2θ] | d-spacing [Å] | Rel. Int. [%] |
|---|---|---|---|---|
| 6.00 | 36.11 | 0.3070 | 14.73 | 59.42 |
| 8.36 | 34.26 | 0.3070 | 10.58 | 56.37 |
| 9.72 | 47.85 | 0.2047 | 9.10 | 78.73 |
| 12.70 | 10.89 | 0.8187 | 6.97 | 17.91 |
| 14.53 | 60.77 | 0.1535 | 6.10 | 100.00 |
| 15.25 | 35.45 | 0.4093 | 5.81 | 58.33 |

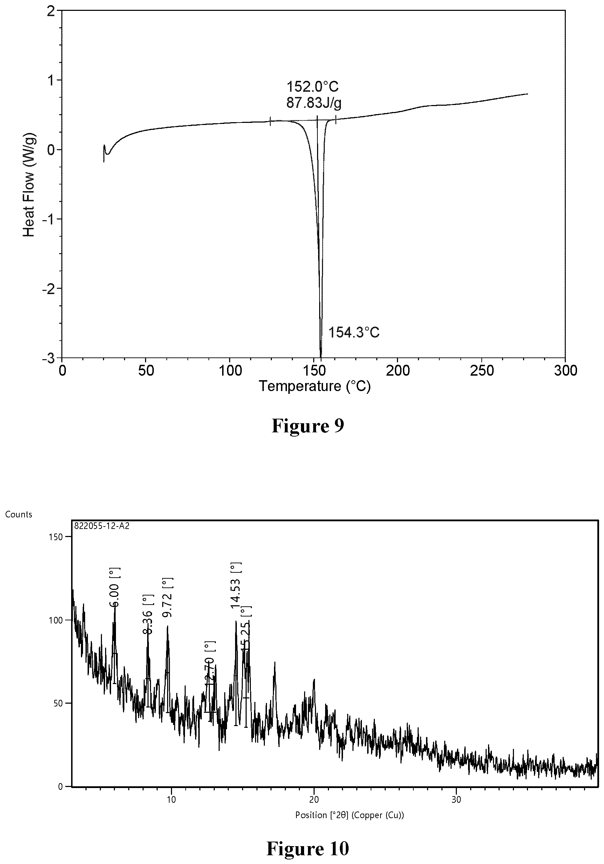

Further, the present invention provides the crystalline form A of the sulfate of the compound of formula (I), and its X-ray powder diffraction pattern is substantially shown in Figure 10.

The present invention provides the crystalline form A of the sulfate of the compound of formula (I), and its differential scanning calorimetry (DSC) does not detect a thermal signal.

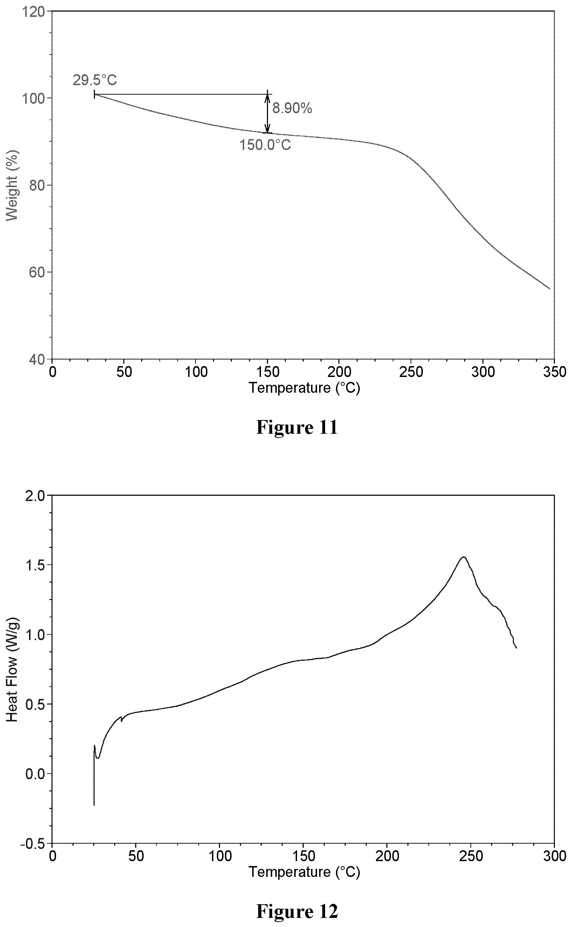

The present invention provides the crystalline form A of the sulfate of the compound of formula (I), and its differential scanning calorimetry (DSC) thermogram is shown in Figure 12.

The present invention provides the crystalline form A of the sulfate of the compound of formula (I), and its thermogravimetric analysis (TGA) thermogram shows 8.90% weight loss below 150°C, and the decomposition temperature is 250°C.

The present invention provides the crystalline form A of the sulfate of the compound of formula (I), and its thermogravimetric analysis (TGA) thermogram is shown in Figure 11.

The present invention provides the crystalline form A of the sulfate of the compound of formula (I), which is a hydrate.

The present invention also provides a sulfate of the compound of formula (I), which is in crystalline form (crystalline form B of the sulfate), and its X-ray powder diffraction pattern has characteristic diffraction peaks at the following 2θ positions: 13.43°±0.2°, 17.35°±0.2°, 18.15°±0.2°, 20.93°±0.2°, 21.37°±0.2°, 24.22°±0.2°, 25.15°±0.2°, using Cu-Kα radiation.

Further, the present invention provides the crystalline form B of the sulfate of the compound of formula (I), and its X-ray powder diffraction pattern also has characteristic diffraction peaks at the following 2θ positions: 8.55°±0.2°, 22.42° ±0.2°, 22.85°±0.2°, 29.20°±0.2°, using Cu-Kα radiation.

The present invention provides the crystalline form B of the sulfate of the compound of formula (I), and the 2θ values and corresponding intensity in its X-ray powder diffraction pattern are shown in Table 3, and the error range of 2θ is ±0.2°.

**Table 3: 2θ values and corresponding intensity in XRPD pattern of the crystalline form B of the sulfate of the compound of formula (I)**

| Pos. [°2θ] | Height [cts] | FWHM Left [°2θ] | d-spacing [Å] | Rel. Int. [%] |
|---|---|---|---|---|
| 8.55 | 48.78 | 0.1535 | 10.34 | 25.47 |
| 13.43 | 92.94 | 0.1023 | 6.59 | 48.53 |
| 17.35 | 125.14 | 0.1023 | 5.11 | 65.35 |
| 18.15 | 128.71 | 0.1279 | 4.89 | 67.21 |
| 20.93 | 144.79 | 0.1279 | 4.25 | 75.61 |
| 21.37 | 109.26 | 0.1535 | 4.16 | 57.05 |
| 22.42 | 61.00 | 0.1535 | 3.96 | 31.86 |
| 22.85 | 48.91 | 0.1535 | 3.89 | 25.54 |
| 24.22 | 154.95 | 0.1279 | 3.68 | 80.92 |
| 25.15 | 191.50 | 0.1279 | 3.54 | 100.00 |
| 28.41 | 18.31 | 0.3070 | 3.14 | 9.56 |
| 29.20 | 23.41 | 0.3070 | 3.06 | 12.23 |
| 31.58 | 18.21 | 0.3070 | 2.83 | 9.51 |

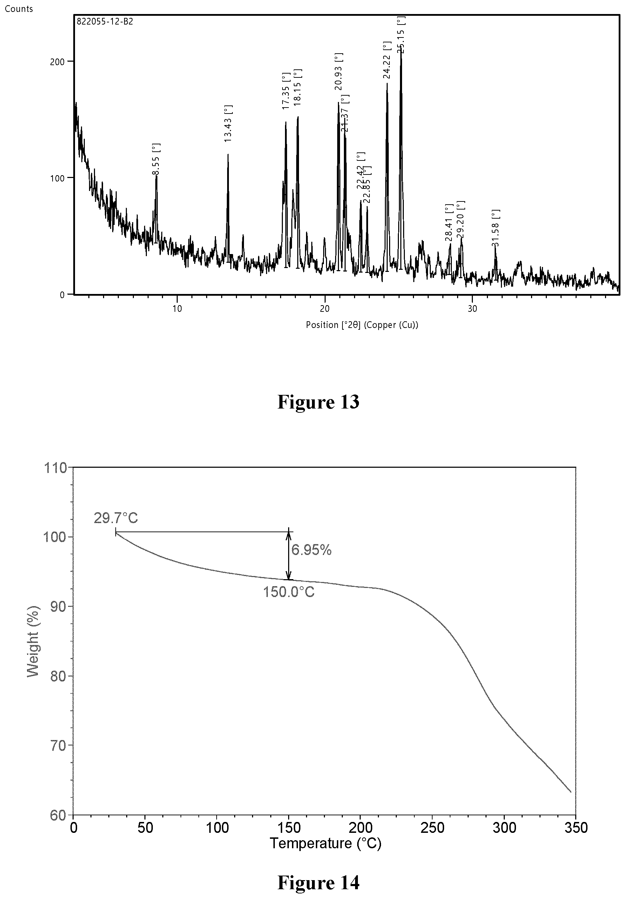

Further, the present invention provides the crystalline form B of the sulfate of the compound of formula (I), and its X-ray powder diffraction pattern is substantially shown in Figure 13.

The present invention provides the crystalline form B of the sulfate of the compound of formula (I), and its differential scanning calorimetry (DSC) shows an endothermic curve, in which Tₛₜₐᵣₜ = 180.4°C, Tₚₑₐₖ = 189.0°C.

The present invention provides the crystalline form B of the sulfate of the compound of formula (I), and its differential scanning calorimetry (DSC) shows that its melting point is 189.0°C_{∘}

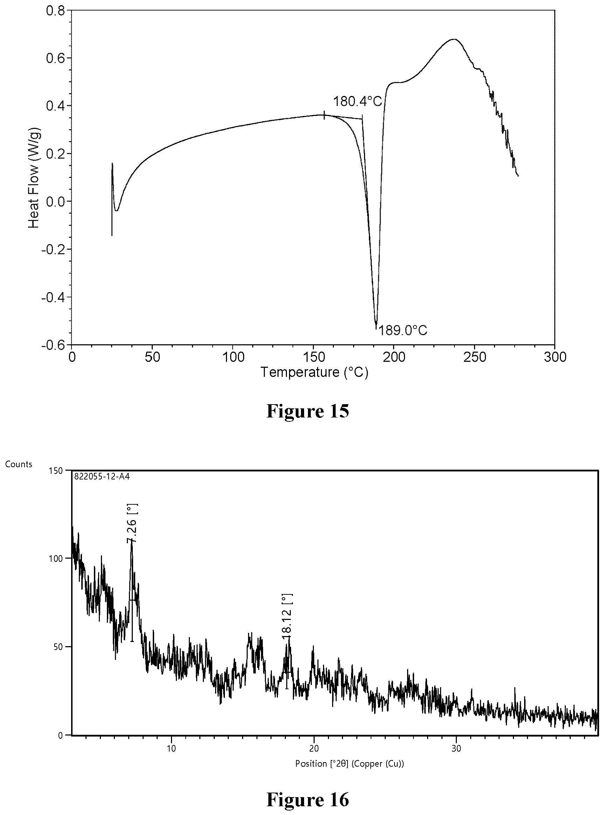

The present invention provides the crystalline form B of the sulfate of the compound of formula (I), and its differential scanning calorimetry (DSC) thermogram is shown in Figure 15.

The present invention provides the crystalline form B of the sulfate of the compound of formula (I), and its thermogravimetric analysis (TGA) thermogram shows 6.95% weight loss below 150°C, and the decomposition temperature is 250°C.

The present invention provides the crystalline form B of the sulfate of the compound of formula (I), and its thermogravimetric analysis (TGA) thermogram is shown in Figure 14.

The present invention provides the crystalline form B of the sulfate of the compound of formula (I), which is a hydrate.

The present invention also provides a maleate of the compound of formula (I), which is in crystalline form (crystalline form A of the maleate), and its X-ray powder diffraction pattern has characteristic diffraction peaks at the following 2θ positions: 7.26°±0.2°, 18.12°±0.2°, using Cu-Kα radiation.

The present invention provides the crystalline form A of the maleate of the compound of formula (I), and the 2θ values and corresponding intensity in its X-ray powder diffraction pattern are shown in Table 4, and the error range of 2θ is ±0.2°.

**Table 4: 2θ values and corresponding intensity in XRPD pattern of the crystalline form A of the maleate of the compound of formula (I)**

| Pos. [°2θ] | Height [cts] | FWHM Left [°2θ] | d-spacing [Å] | Rel. Int. [%] |
|---|---|---|---|---|
| 7.26 | 46.65 | 0.3070 | 12.17 | 100.00 |
| 18.12 | 18.72 | 0.6140 | 4.90 | 40.12 |

Further, the present invention provides the crystalline form A of the maleate of the compound of formula (I), and its X-ray powder diffraction pattern is substantially shown in Figure 16.

The present invention provides the crystalline form A of the maleate of the compound of formula (I), and its differential scanning calorimetry (DSC) shows 4 endothermic peaks at 54.2°C, 79.9°C, 125.4°C and 178.4°C (peak temperature).

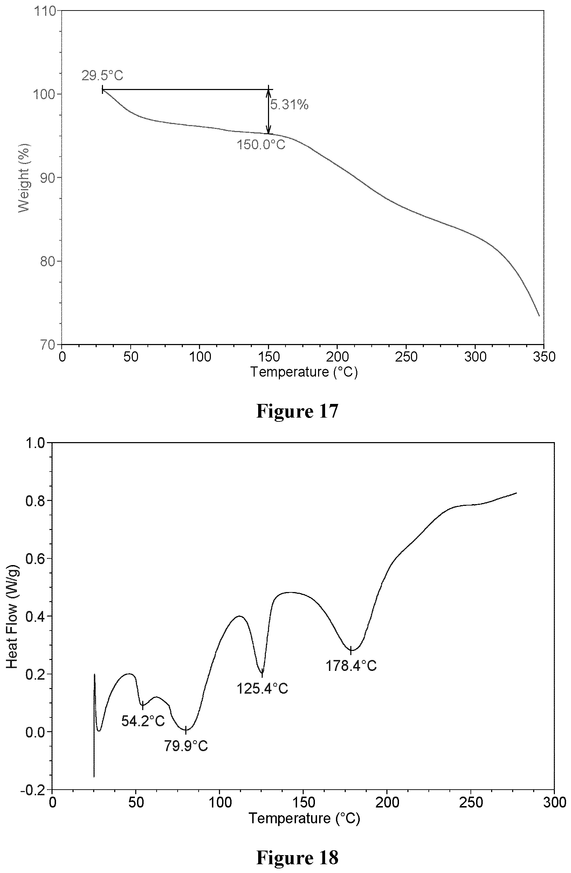

The present invention provides the crystalline form A of the maleate of the compound of formula (I), and its differential scanning calorimetry (DSC) thermogram is shown in Figure 18.

The present invention provides the crystalline form A of the maleate of the compound of formula (I), and its thermogravimetric analysis (TGA) thermogram shows 5.31% weight loss below 150°C.

The present invention provides the crystalline form A of the maleate of the compound of formula (I), and its thermogravimetric analysis (TGA) thermogram is shown in Figure 17.

The present invention provides the crystalline form A of the maleate of the compound of formula (I), which is a hydrate.

The present invention also provides a maleate of the compound of formula (I), which is in crystalline form (crystalline form B of the maleate), and its X-ray powder diffraction pattern has characteristic diffraction peaks at the following 2θ positions: 5.10°±0.2°, 7.65°±0.2°, 9.52°±0.2°, 11.67°±0.2°, 17.34°±0.2°, 21.38°±0.2°, using Cu-Kα radiation.

Further, the present invention provides the crystalline form B of the maleate of the compound of formula (I), and its X-ray powder diffraction pattern also has characteristic diffraction peaks at the following 2θ positions: 6.24°±0.2°, 19.01°±0.2°, 19.93°±0.2°, 22.90°±0.2°, 23.48°±0.2°, 24.33°±0.2°, using Cu-Kα radiation.

The present invention provides the crystalline form B of the maleate of the compound of formula (I), and the 2θ values and corresponding intensity in its X-ray powder diffraction pattern are shown in Table 1, and the error range of 2θ is ±0.2°.

**Table 5: 2θ values and corresponding intensity in XRPD pattern of the crystalline form B of the maleate of the compound of formula (I)**

| Pos. [°2θ] | Height [cts] | FWHM Left [°2θ] | d-spacing [Å] | Rel. Int. [%] |
|---|---|---|---|---|
| 5.10 | 190.22 | 0.1023 | 17.34 | 100.00 |
| 6.24 | 71.59 | 0.1535 | 14.17 | 37.64 |
| 7.65 | 94.50 | 0.1279 | 11.55 | 49.68 |
| 9.52 | 78.38 | 0.1279 | 9.29 | 41.20 |
| 11.67 | 108.58 | 0.1023 | 7.58 | 57.08 |
| 17.34 | 186.93 | 0.1791 | 5.11 | 98.27 |
| 19.01 | 28.88 | 0.3070 | 4.67 | 15.18 |
| 19.93 | 59.78 | 0.1535 | 4.45 | 31.43 |
| 21.38 | 81.21 | 0.1279 | 4.16 | 42.69 |
| 22.90 | 34.87 | 0.2047 | 3.88 | 18.33 |
| 23.48 | 48.10 | 0.1535 | 3.79 | 25.29 |
| 24.33 | 43.02 | 0.2047 | 3.66 | 22.62 |
| 27.59 | 18.85 | 0.3070 | 3.23 | 9.91 |
| 28.95 | 11.86 | 0.6140 | 3.08 | 6.24 |

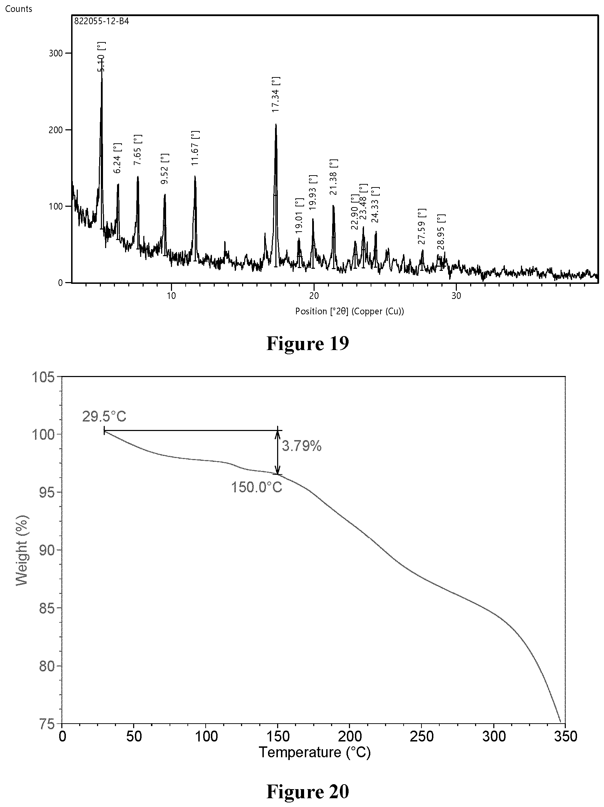

Further, the present invention provides the crystalline form B of the maleate of the compound of formula (I), and its X-ray powder diffraction pattern is substantially shown in Figure 19.

The present invention provides the crystalline form B of the maleate of the compound of formula (I), and its differential scanning calorimetry (DSC) shows 3 endothermic peaks at 77.5°C, 125.4°C and 179.1°C (peak temperature).

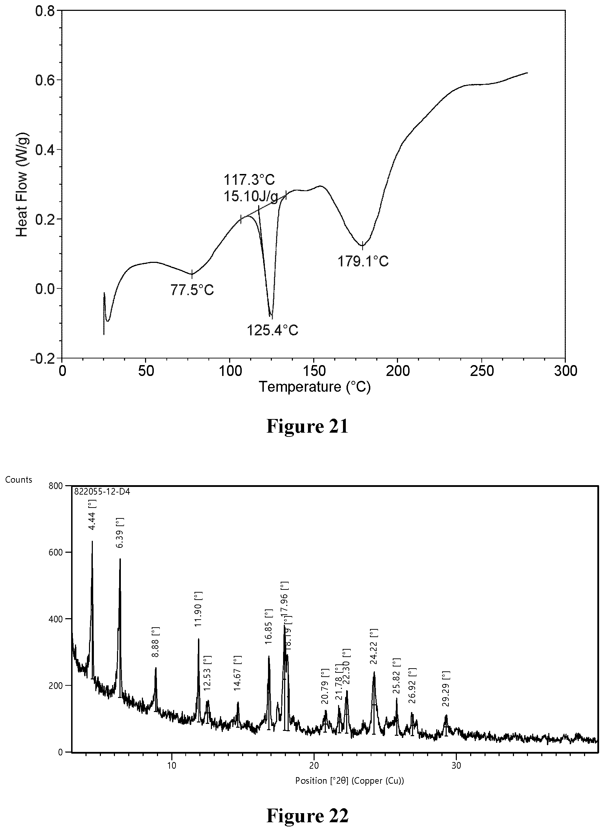

The present invention provides the crystalline form B of the maleate of the compound of formula (I), and its differential scanning calorimetry (DSC) thermogram is shown in Figure 21.

The present invention provides the crystalline form B of the maleate of the compound of formula (I), and its thermogravimetric analysis (TGA) thermogram shows 3.79% weight loss below 150°C.

The present invention provides the crystalline form B of the maleate of the compound of formula (I), and its thermogravimetric analysis (TGA) thermogram is shown in Figure 20.

The present invention provides the crystalline form B of the maleate of the compound of formula (I), which is a hydrate.

The present invention also provides a maleate of the compound of formula (I), which is in crystalline form (crystalline form C of the maleate), and its X-ray powder diffraction pattern has characteristic diffraction peaks at the following 2θ positions: 4.44°±0.2°, 6.39°±0.2°, 11.90°±0.2°, 16.85°±0.2°, 17.96°±0.2°, 18.19°±0.2°, 22.30°±0.2°, 24.22°±0.2°, using Cu-Kα radiation.

Further, the present invention provides the crystalline form C of the maleate of the compound of formula (I), and its X-ray powder diffraction pattern also has characteristic diffraction peaks at the following 2θ positions: 8.88°±0.2°, 14.67°±0.2°, 21.78°±0.2°, 22.30°±0.2°, 25.82°±0.2°, 26.92°±0.2°, using Cu-Kα radiation.

The present invention provides the crystalline form C of the maleate of the compound of formula (I), and the 2θ values and corresponding intensity in its X-ray powder diffraction pattern are shown in Table 6, and the error range of 2θ is ±0.2°.

**Table 6: 2θ values and corresponding intensity in XRPD pattern of the crystalline form C of the maleate of the compound of formula (I)**

| Pos. [°2θ] | Height [cts] | FWHM Left [°2θ] | d-spacing [Å] | Rel. Int. [%] |
|---|---|---|---|---|
| 4.44 | 413.95 | 0.0669 | 19.92 | 99.83 |
| 6.39 | 414.67 | 0.0836 | 13.83 | 100.00 |
| 8.88 | 130.47 | 0.1004 | 9.96 | 31.46 |
| 11.90 | 249.92 | 0.0669 | 7.44 | 60.27 |
| 12.53 | 61.43 | 0.2676 | 7.07 | 14.82 |
| 14.67 | 73.47 | 0.1338 | 6.04 | 17.72 |
| 16.85 | 222.19 | 0.1338 | 5.26 | 53.58 |
| 17.96 | 306.46 | 0.1171 | 4.94 | 73.90 |
| 18.19 | 203.98 | 0.1004 | 4.88 | 49.19 |
| 20.79 | 50.11 | 0.2007 | 4.27 | 12.08 |
| 21.78 | 64.98 | 0.2007 | 4.08 | 15.67 |
| 22.30 | 112.73 | 0.2007 | 3.99 | 27.19 |
| 24.22 | 174.98 | 0.2007 | 3.68 | 42.20 |
| 25.82 | 90.43 | 0.1338 | 3.45 | 21.81 |
| 26.92 | 63.01 | 0.2007 | 3.31 | 15.20 |
| 29.29 | 54.87 | 0.2676 | 3.05 | 13.23 |

Further, the present invention provides the crystalline form C of the maleate of the compound of formula (I), and its X-ray powder diffraction pattern is substantially shown in Figure 22.

The present invention provides the crystalline form C of the maleate of the compound of formula (I), and its differential scanning calorimetry (DSC) shows 2 endothermic peaks at 126.6°C and 183.9°C (peak temperature).

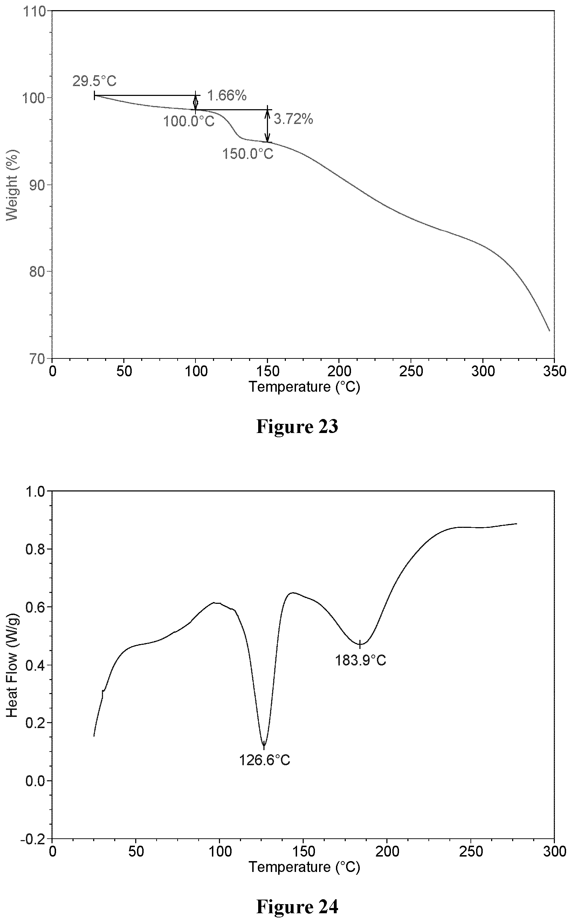

The present invention provides the crystalline form C of the maleate of the compound of formula (I), and its differential scanning calorimetry (DSC) thermogram is shown in Figure 24.

The present invention provides the crystalline form C of the maleate of the compound of formula (I), and its thermogravimetric analysis (TGA) thermogram shows 1.7% weight loss when the sample is heated to 100°C, and 3.7% weight loss when the sample is continued to be heated to 150°C.

The present invention provides the crystalline form C of the maleate of the compound of formula (I), and its thermogravimetric analysis (TGA) thermogram is shown in Figure 23.

The present invention provides the crystalline form C of the maleate of the compound of formula (I), which is a hydrate.

The present invention also provides a phosphate of the compound of formula (I), which is in crystalline form (crystalline form A of the phosphate), and its X-ray powder diffraction pattern has characteristic diffraction peaks at the following 2θ positions: 13.61°±0.2°, 16.67°±0.2°, 21.04°±0.2°, using Cu-Kα radiation.

The present invention provides the crystalline form A of the phosphate of the compound of formula (I), and its X-ray powder diffraction pattern also has characteristic diffraction peaks at the following 2θ positions: 15.45°±0.2°, 18.45°±0.2°, using Cu-Kα radiation.

The present invention provides the crystalline form A of the phosphate of the compound of formula (I), and the 2θ values and corresponding intensity in its X-ray powder diffraction pattern are shown in Table 7, and the error range of 2θ is ±0.2°.

**Table 7: 2θ values and corresponding intensity in XRPD pattern of the crystalline form A of the phosphate of the compound of formula (I)**

| Pos. [°2θ] | Height [cts] | FWHM Left [°2θ] | d-spacing [Å] | Rel. Int. [%] |
|---|---|---|---|---|
| 13.61 | 43.66 | 0.2047 | 6.51 | 100.00 |
| 15.45 | 16.82 | 0.8187 | 5.74 | 38.52 |
| 16.67 | 31.26 | 0.4093 | 5.32 | 71.60 |
| 18.45 | 19.19 | 0.3070 | 4.81 | 43.96 |
| 21.04 | 27.39 | 0.3070 | 4.22 | 62.73 |

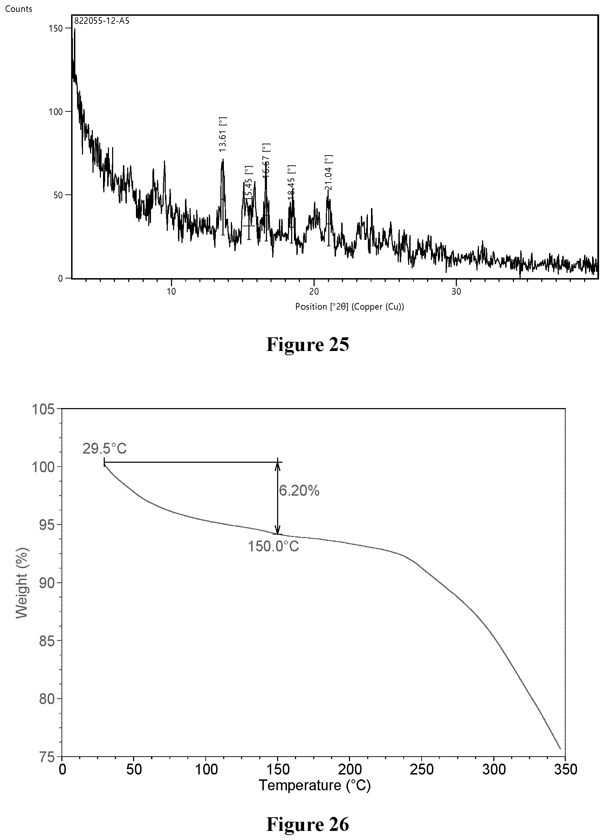

Further, the present invention provides the crystalline form A of the phosphate of the compound of formula (I), and its X-ray powder diffraction pattern is substantially shown in Figure 25.

The present invention provides the crystalline form A of the phosphate of the compound of formula (I), and its differential scanning calorimetry (DSC) shows 2 endothermic peaks at 61.4°C and 150.9°C (peak temperature).

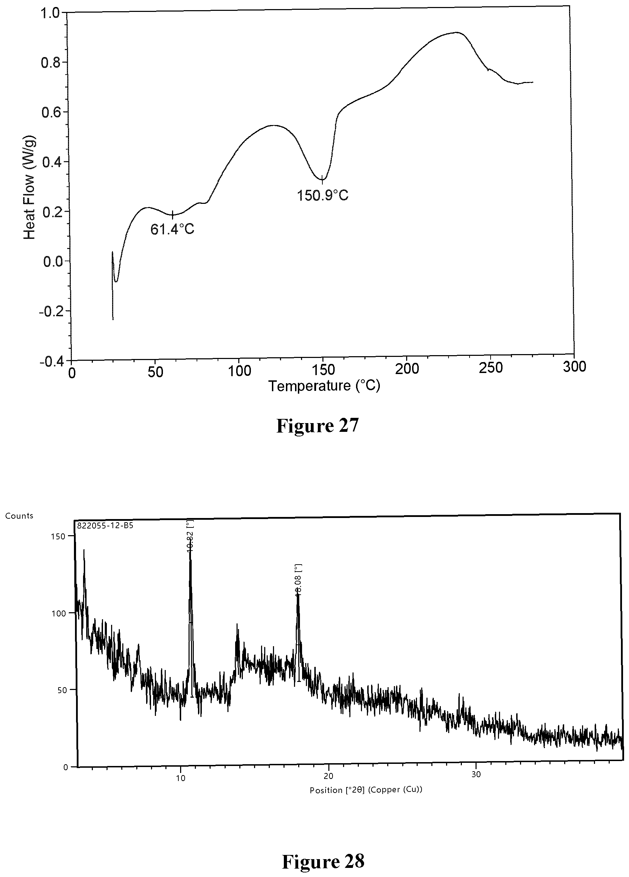

The present invention provides the crystalline form A of the phosphate of the compound of formula (I), and its differential scanning calorimetry (DSC) thermogram is shown in Figure 27.

The present invention provides the crystalline form A of the phosphate of the compound of formula (I), and its thermogravimetric analysis (TGA) thermogram shows 6.20% weight loss below 150°C.

The present invention provides the crystalline form A of the phosphate of the compound of formula (I), and its thermogravimetric analysis (TGA) thermogram is shown in Figure 26.

The present invention provides the crystalline form A of the phosphate of the compound of formula (I), which is a hydrate.

The present invention also provides a phosphate of the compound of formula (I), which is in crystalline form (crystalline form B of the phosphate), and its X-ray powder diffraction pattern has characteristic diffraction peaks at the following 2θ positions: 10.82°±0.2°, 18.08°±0.2°, using Cu-Kα radiation.

The present invention provides the crystalline form B of the phosphate of the compound of formula (I), and the 2θ values and corresponding intensity in its X-ray powder diffraction pattern are shown in Table 8, and the error range of 2θ is ±0.2°.

**Table 8: 2θ values and corresponding intensity in XRPD pattern of the crystalline form B of the phosphate of the compound of formula (I)**

| Pos. [°2θ] | Height [cts] | FWHM Left [°2θ] | d-spacing [Å] | Rel. Int. [%] |
|---|---|---|---|---|
| 10.82 | 97.03 | 0.1791 | 8.18 | 100.00 |
| 18.08 | 48.18 | 0.3070 | 4.91 | 49.66 |

Further, the present invention provides the crystalline form B of the phosphate of the compound of formula (I), and its X-ray powder diffraction pattern is substantially shown in Figure 28.

The present invention provides the crystalline form B of the phosphate of the compound of formula (I), and its differential scanning calorimetry (DSC) shows 2 endothermic peaks at 74.8°C and 142.6°C (peak temperature).

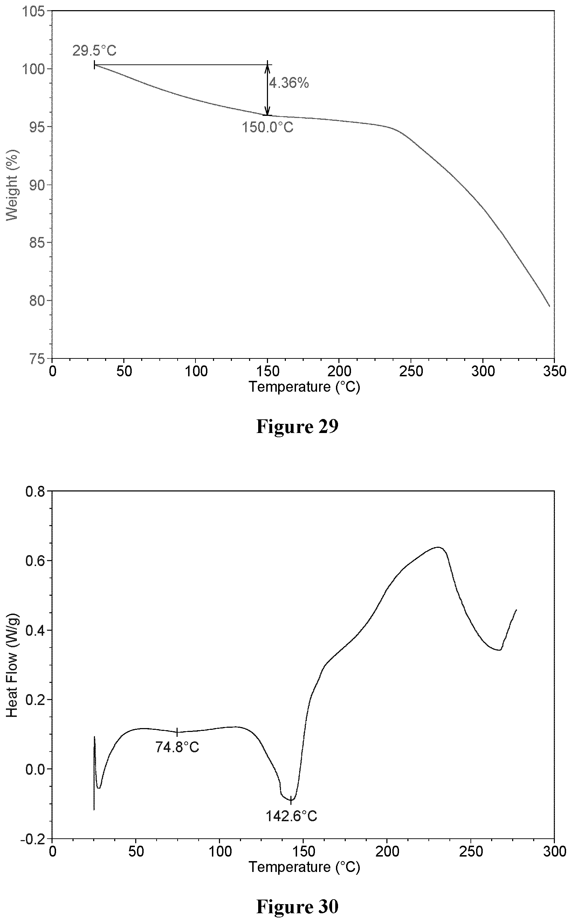

The present invention provides the crystalline form B of the phosphate of the compound of formula (I), and its differential scanning calorimetry (DSC) thermogram is shown in Figure 30.

The present invention provides the crystalline form B of the phosphate of the compound of formula (I), and its thermogravimetric analysis (TGA) thermogram shows 4.36% weight loss below 150°C.

The present invention provides the crystalline form B of the phosphate of the compound of formula (I), and its thermogravimetric analysis (TGA) thermogram is shown in Figure 29.

The present invention provides the crystalline form B of the phosphate of the compound of formula (I), which is a hydrate.

The present invention also provides a phosphate of the compound of formula (I), which is in crystalline form (crystalline form C of the phosphate), and its X-ray powder diffraction pattern has characteristic diffraction peaks at the following 2θ positions: 3.45°±0.2°, 7.85°±0.2°, 13.70°±0.2°, 24.79°±0.2°, using Cu-Kα radiation.

The present invention provides the crystalline form C of the phosphate of the compound of formula (I), and its X-ray powder diffraction pattern also has characteristic diffraction peaks at the following 2θ positions: 10.50°±0.2°, 26.85°±0.2°, using Cu-Kα radiation.

The present invention provides the crystalline form C of the phosphate of the compound of formula (I), and the 2θ values and corresponding intensity in its X-ray powder diffraction pattern are shown in Table 9, and the error range of 2θ is ±0.2°.

**Table 9: 2θ values and corresponding intensity in XRPD pattern of the crystalline form C of the phosphate of the compound of formula (I)**

| Pos. [°2θ] | Height [cts] | FWHM Left [°2θ] | d-spacing [Å] | Rel. Int. [%] |
|---|---|---|---|---|
| 3.45 | 137.54 | 0.2676 | 25.59 | 100.00 |
| 7.85 | 53.27 | 0.2676 | 11.27 | 38.73 |
| 10.50 | 27.69 | 0.4015 | 8.43 | 20.13 |
| 13.70 | 39.38 | 0.4015 | 6.47 | 28.63 |
| 24.79 | 54.20 | 0.4684 | 3.59 | 39.40 |
| 26.85 | 24.88 | 0.4015 | 3.32 | 18.09 |

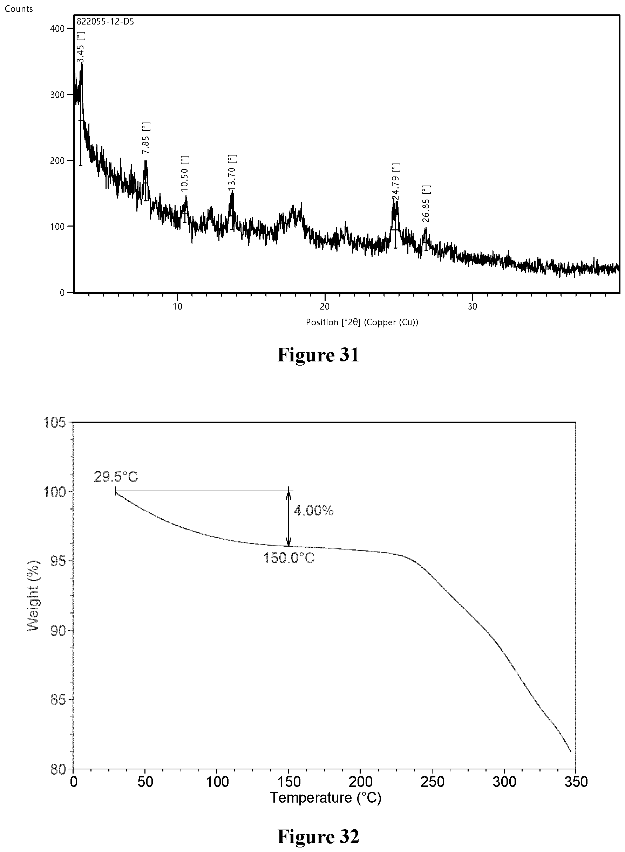

Further, the present invention provides the crystalline form C of the phosphate of the compound of formula (I), and its X-ray powder diffraction pattern is substantially shown in Figure 31.

The present invention provides the crystalline form C of the phosphate of the compound of formula (I), and its differential scanning calorimetry (DSC) shows 2 endothermic peaks at 81.7°C and 159.4°C (peak temperature).

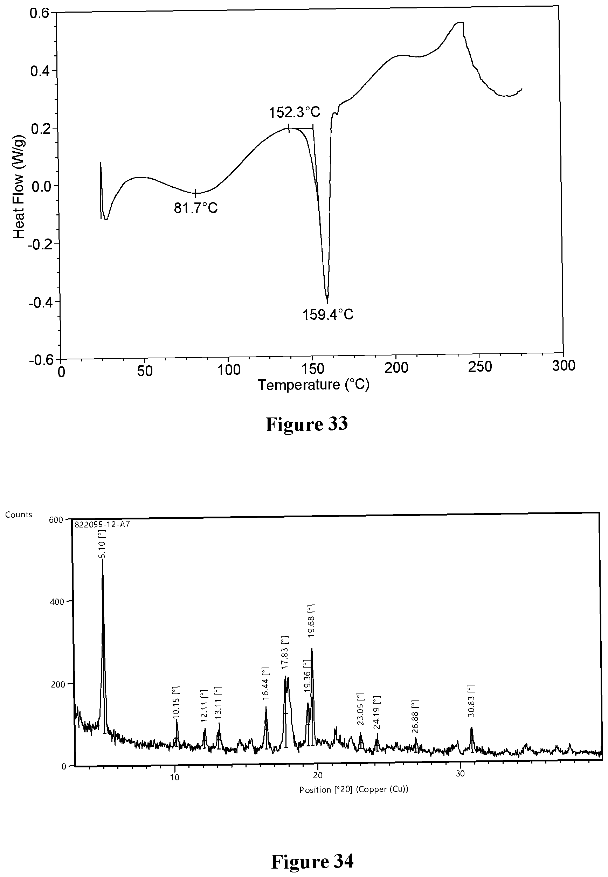

The present invention provides the crystalline form C of the phosphate of the compound of formula (I), and its differential scanning calorimetry (DSC) thermogram is shown in Figure 33.

The present invention provides the crystalline form C of the phosphate of the compound of formula (I), and its thermogravimetric analysis (TGA) thermogram shows 4.00% weight loss below 150°C

The present invention provides the crystalline form C of the phosphate of the compound of formula (I), and its thermogravimetric analysis (TGA) thermogram is shown in Figure 32.

The present invention provides the crystalline form C of the phosphate of the compound of formula (I), which is a hydrate.

The present invention also provides a mucate of the compound of formula (I), which is in crystalline form (crystalline form A of the mucate), and its X-ray powder diffraction pattern has characteristic diffraction peaks at the following 2θ positions: 5.10°±0.2°, 16.44°±0.2°, 17.83°±0.2°, 19.36°±0.2°, 19.68°±0.2°, using Cu-Kα radiation.

The present invention provides the crystalline form A of the mucate of the compound of formula (I), and its X-ray powder diffraction pattern also has characteristic diffraction peaks at the following 2θ positions: 10.15°±0.2°, 13.11°±0.2 °, 23.05°±0.2°, 30.83°±0.2°, using Cu-Kα radiation.

The present invention provides the crystalline form A of the mucate of the compound of formula (I), and the 2θ values and corresponding intensity in its X-ray powder diffraction pattern are shown in Table 10, and the error range of 2θ is ±0.2°.

**Table 10: 2θ values and corresponding intensity in XRPD pattern of the crystalline form A of the mucate of the compound of formula (I)**

| Pos. [°2θ] | Height [cts] | FWHM Left [°2θ] | d-spacing [Å] | Rel. Int. [%] |
|---|---|---|---|---|
| 5.10 | 399.88 | 0.1023 | 17.33 | 100.00 |
| 10.15 | 40.56 | 0.3070 | 8.71 | 10.14 |
| 12.11 | 36.21 | 0.3070 | 7.31 | 9.06 |
| 13.11 | 45.46 | 0.2558 | 6.75 | 11.37 |
| 16.44 | 92.22 | 0.2047 | 5.39 | 23.06 |
| 17.83 | 165.12 | 0.2558 | 4.98 | 41.29 |
| 19.36 | 104.08 | 0.2047 | 4.59 | 26.03 |
| 19.68 | 236.77 | 0.1279 | 4.51 | 59.21 |
| 23.05 | 30.79 | 0.2558 | 3.86 | 7.70 |
| 24.19 | 27.75 | 0.3070 | 3.68 | 6.94 |
| 26.88 | 19.46 | 0.8187 | 3.32 | 4.87 |
| 30.83 | 59.15 | 0.2047 | 2.90 | 14.79 |

Further, the present invention provides the crystalline form A of the mucate of the compound of formula (I), and its X-ray powder diffraction pattern is substantially shown in Figure 34.

The present invention provides the crystalline form A of the mucate of the compound of formula (I), and its differential scanning calorimetry (DSC) shows an endothermic curve, in which Tₛₜₐᵣₜ = 186.2°C, Tₚₑₐₖ = 191.5°C, and ΔH = 177.7 J/g.

The present invention provides the crystalline form A of the mucate of the compound of formula (I), and its differential scanning calorimetry (DSC) shows that its melting point is 191.5°C.

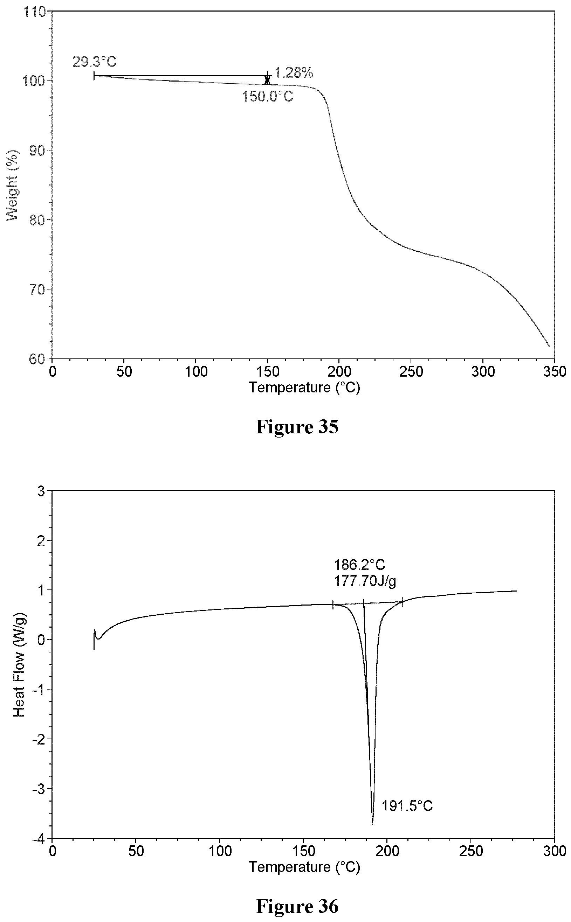

The present invention provides the crystalline form A of the mucate of the compound of formula (I), and its differential scanning calorimetry (DSC) thermogram is shown in Figure 36.

The present invention provides the crystalline form A of the mucate of the compound of formula (I), and its thermogravimetric analysis (TGA) thermogram shows 1.28% weight loss below 150°C.

The present invention provides the crystalline form A of the mucate of the compound of formula (I), and its thermogravimetric analysis (TGA) thermogram is shown in Figure 35.

The present invention provides the crystalline form A of the mucate of the compound of formula (I), which is an anhydrate.

The present invention also provides a tartrate of the compound of formula (I), which is in crystalline form (crystalline form A of the tartrate), and its X-ray powder diffraction pattern has characteristic diffraction peaks at the following 2θ positions: 8.21°±0.2°, 15.16°±0.2°, using Cu-Kα radiation.

The present invention provides the crystalline form A of the tartrate of the compound of formula (I), and its X-ray powder diffraction pattern also has characteristic diffraction peaks at the following 2θ positions: 16.46°±0.2°, 21.02°±0.2°, using Cu-Kα radiation.

The present invention provides the crystalline form A of the tartrate of the compound of formula (I), and the 2θ values and corresponding intensity in its X-ray powder diffraction pattern are shown in Table 11, and the error range of 2θ is ±0.2°.

**Table 11: 2θ values and corresponding intensity in XRPD pattern of the crystalline form A of the tartrate of the compound of formula (I)**

| Pos. [°2θ] | Height [cts] | FWHM Left [°2θ] | d-spacing [Å] | Rel. Int. [%] |
|---|---|---|---|---|
| 8.21 | 84.50 | 0.2047 | 10.77 | 100.00 |
| 15.16 | 50.40 | 0.1535 | 5.84 | 59.65 |
| 16.46 | 31.50 | 0.8187 | 5.38 | 37.27 |
| 21.02 | 20.54 | 0.6140 | 4.23 | 24.31 |

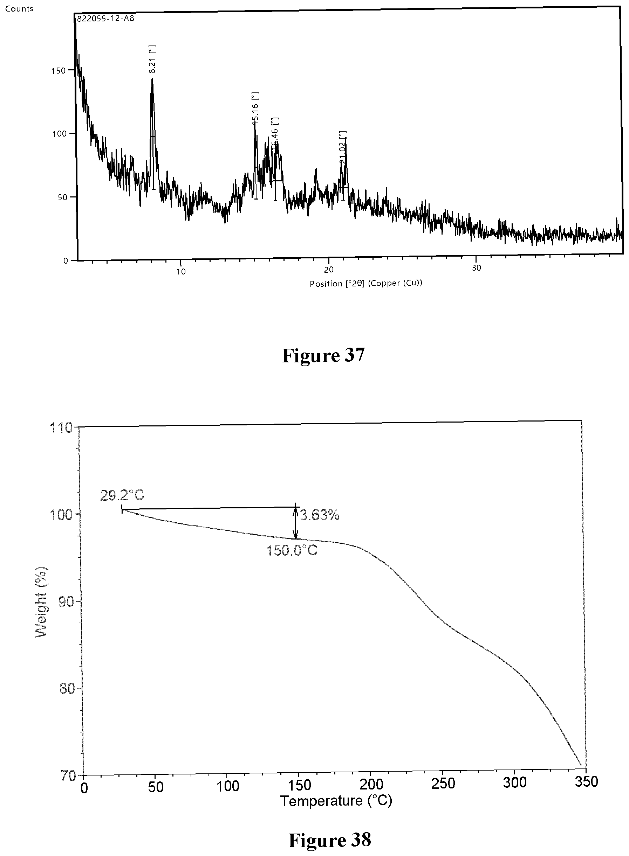

Further, the present invention provides the crystalline form A of the tartrate of the compound of formula (I), and its X-ray powder diffraction pattern is substantially shown in Figure 37.

The present invention provides the crystalline form A of the tartrate of the compound of formula (I), and its differential scanning calorimetry (DSC) shows 3 endothermic peaks at 67.6°C, 178.0°C and 204.6°C (peak temperature).

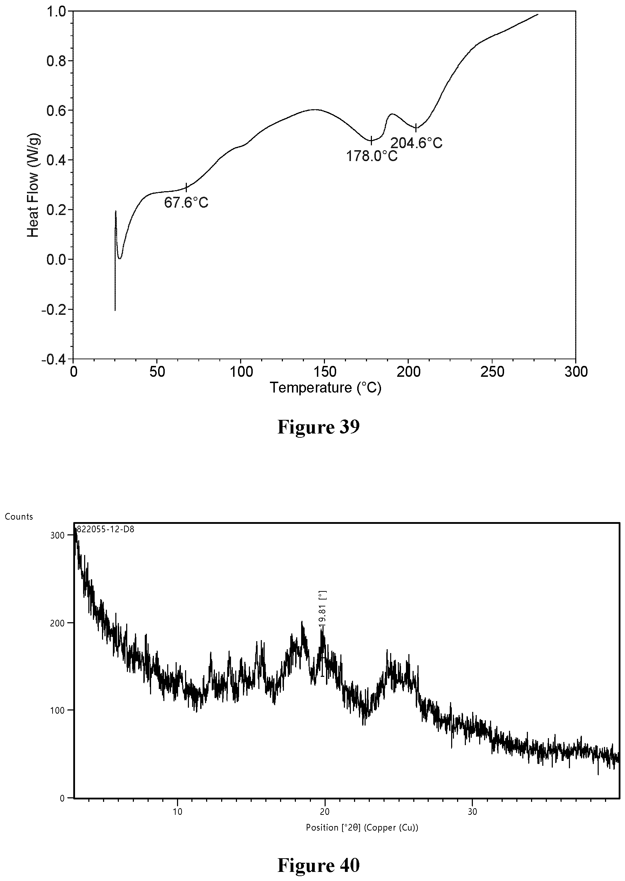

The present invention provides the crystalline form A of the tartrate of the compound of formula (I), and its differential scanning calorimetry (DSC) thermogram is shown in Figure 39.

The present invention provides the crystalline form A of the tartrate of the compound of formula (I), and its thermogravimetric analysis (TGA) thermogram shows 3.63% weight loss below 150°C.

The present invention provides the crystalline form A of the tartrate of the compound of formula (I), and its thermogravimetric analysis (TGA) thermogram is shown in Figure 38.

The present invention provides the crystalline form A of the tartrate of the compound of formula (I), which is a hydrate.

The present invention also provides a tartrate of the compound of formula (I), which is in crystalline form (crystalline form B of the tartrate), and its X-ray powder diffraction pattern has a characteristic diffraction peak at the following 2θ position: 19.81°±0.2°, using Cu-Kα radiation.

The present invention provides the crystalline form B of the tartrate of the compound of formula (I), and the 2θ values and corresponding intensity in its X-ray powder diffraction pattern are shown in Table 12, and the error range of 2θ is ±0.2°.

**Table 12: 2θ values and corresponding intensity in XRPD pattern of the crystalline form B of the tartrate of the compound of formula (I)**

| Pos. [°2θ] | Height [cts] | FWHM Left [°2θ] | d-spacing [Å] | Rel. Int. [%] |
|---|---|---|---|---|
| 19.81 | 43.53 | 0.4896 | 4.48 | 100.00 |

Further, the present invention provides the crystalline form B of the tartrate of the compound of formula (I), and its X-ray powder diffraction pattern is substantially shown in Figure 40.

The present invention provides the crystalline form B of the tartrate of the compound of formula (I), and its differential scanning calorimetry (DSC) shows 3 endothermic peaks at 67.3°C, 128.8°C and 193.3°C (peak temperature).

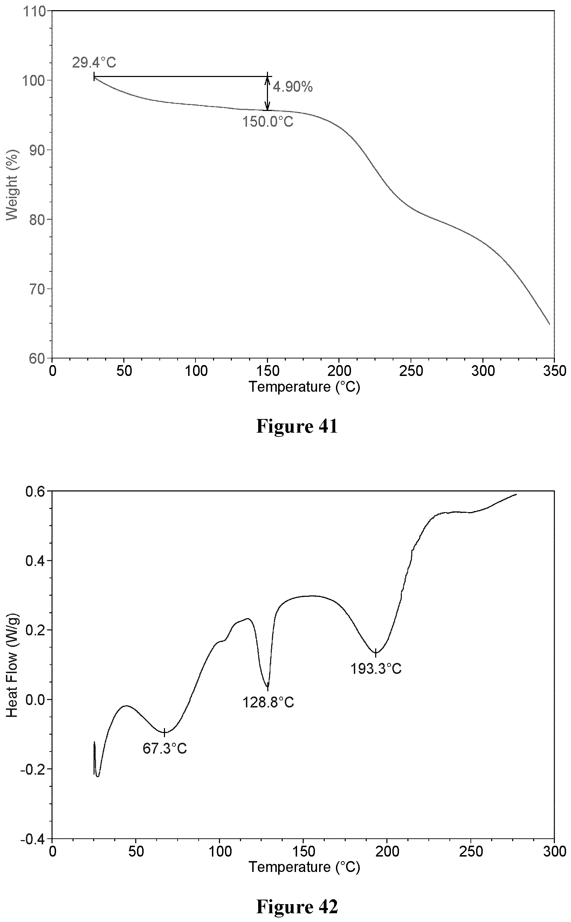

The present invention provides the crystalline form B of the tartrate of the compound of formula (I), and its differential scanning calorimetry (DSC) thermogram is shown in Figure 42.

The present invention provides the crystalline form B of the tartrate of the compound of formula (I), and its thermogravimetric analysis (TGA) thermogram shows 4.90% weight loss below 150°C.

The present invention provides the crystalline form B of the tartrate of the compound of formula (I), and its thermogravimetric analysis (TGA) thermogram is shown in Figure 41.

The present invention provides the crystalline form B of the tartrate of the compound of formula (I), which is a hydrate.

The present invention also provides a tartrate of the compound of formula (I), which is in crystalline form (crystalline form C of the tartrate), and its X-ray powder diffraction pattern has characteristic diffraction peaks at the following 2θ positions: 4.74°±0.2°, 10.82°±0.2°, 13.70°±0.2°, 14.37°±0.2°, 16.21°±0.2°, using Cu-Kα radiation.

The present invention provides the crystalline form C of the tartrate of the compound of formula (I), and its X-ray powder diffraction pattern also has characteristic diffraction peaks at the following 2θ positions: 5.85°±0.2°, 17.83°±0.2°, 18.97°±0.2°, 21.76°±0.2°, using Cu-Kα radiation.

The present invention provides the crystalline form C of the tartrate of the compound of formula (I), and the 2θ values and corresponding intensity in its X-ray powder diffraction pattern are shown in Table 13, and the error range of 2θ is ±0.2°.

**Table 13: 2θ values and corresponding intensity in XRPD pattern of the crystalline form C of the tartrate of the compound of formula (I)**

| Pos. [°2θ] | Height [cts] | FWHM Left [°2θ] | d-spacing [Å] | Rel. Int. [%] |
|---|---|---|---|---|
| 4.74 | 66.74 | 0.3070 | 18.64 | 65.75 |
| 5.85 | 33.36 | 0.3070 | 15.12 | 32.86 |
| 10.82 | 63.43 | 0.2047 | 8.17 | 62.49 |
| 13.70 | 101.51 | 0.1535 | 6.46 | 100.00 |
| 14.37 | 74.51 | 0.2047 | 6.16 | 73.40 |
| 16.21 | 89.95 | 0.2558 | 5.47 | 88.61 |
| 17.83 | 46.55 | 0.4093 | 4.98 | 45.86 |
| 18.97 | 41.74 | 0.3070 | 4.68 | 41.12 |
| 21.76 | 40.95 | 0.3070 | 4.09 | 40.34 |

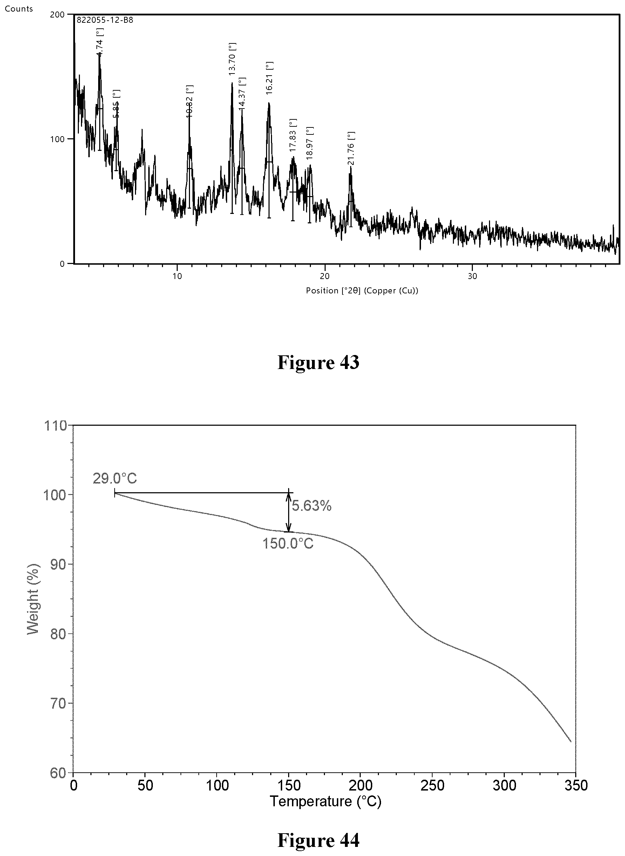

Further, the present invention provides the crystalline form C of the tartrate of the compound of formula (I), and its X-ray powder diffraction pattern is substantially shown in Figure 43.

The present invention provides the crystalline form C of the tartrate of the compound of formula (I), and its differential scanning calorimetry (DSC) shows 3 endothermic peaks at 79.5°C, 134.2°C and 189.7°C (peak temperature).

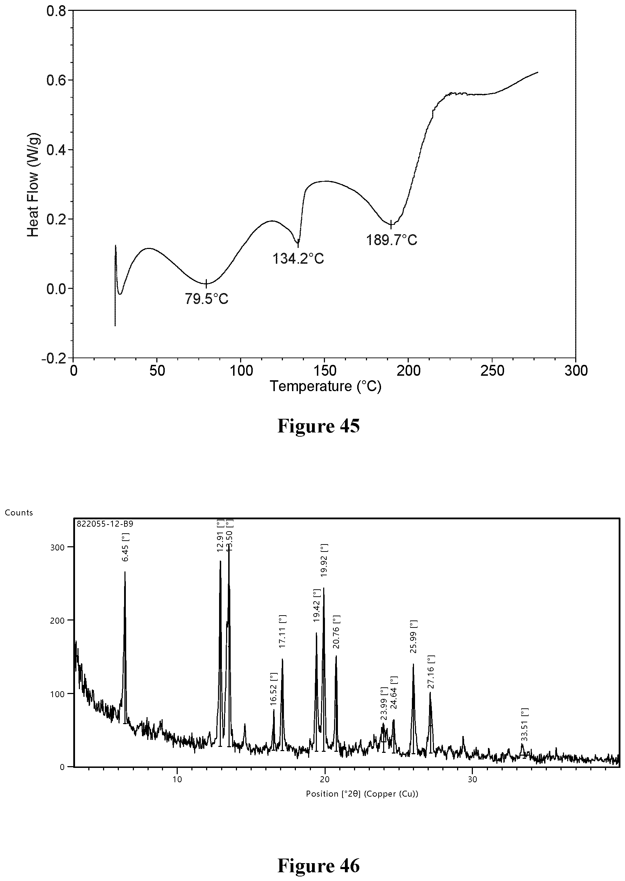

The present invention provides the crystalline form C of the tartrate of the compound of formula (I), and its differential scanning calorimetry (DSC) thermogram is shown in Figure 45.

The present invention provides the crystalline form C of the tartrate of the compound of formula (I), and its thermogravimetric analysis (TGA) thermogram shows 5.63% weight loss below 150°C.

The present invention provides the crystalline form C of the tartrate of the compound of formula (I), and its thermogravimetric analysis (TGA) thermogram is shown in Figure 44.

The present invention provides the crystalline form C of the tartrate of the compound of formula (I), which is a hydrate.

The present invention also provides a fumarate of the compound of formula (I), which is in crystalline form (crystalline form A of the fumarate), and its X-ray powder diffraction pattern has characteristic diffraction peaks at the following 2θ positions: 6.45°±0.2°, 12.91°±0.2°, 13.50°±0.2°, 17.11°±0.2°, 19.42°±0.2°, 19.92°±0.2°, 20.76°±0.2°, 25.99°±0.2°, using Cu-Kα radiation.

The present invention provides the crystalline form A of the fumarate of the compound of formula (I), and its X-ray powder diffraction pattern also has characteristic diffraction peaks at the following 2θ positions: 16.52°±0.2°, 23.99°±0.2°, 24.64°±0.2°, 27.16°±0.2°, using Cu-Kα radiation.

The present invention provides the crystalline form A of the fumarate of the compound of formula (I), and the 2θ values and corresponding intensity in its X-ray powder diffraction pattern are shown in Table 14, and the error range of 2θ is ±0.2°.

**Table 14: 2θ values and corresponding intensity in XRPD pattern of the crystalline form A of the fumarate of the compound of formula (I)**

| Pos. [°2θ] | Height [cts] | FWHM Left [°2θ] | d-spacing [Å] | Rel. Int. [%] |
|---|---|---|---|---|
| 6.45 | 207.24 | 0.1023 | 13.71 | 75.43 |
| 12.91 | 253.01 | 0.1023 | 6.86 | 92.09 |
| 13.50 | 274.75 | 0.1023 | 6.56 | 100.00 |
| 16.52 | 46.25 | 0.1535 | 5.37 | 16.83 |
| 17.11 | 125.12 | 0.1279 | 5.18 | 45.54 |
| 19.42 | 162.03 | 0.1023 | 4.57 | 58.97 |
| 19.92 | 223.23 | 0.1279 | 4.46 | 81.25 |
| 20.76 | 125.86 | 0.1279 | 4.28 | 45.81 |
| 23.99 | 29.41 | 0.3070 | 3.71 | 10.70 |
| 24.64 | 45.39 | 0.1535 | 3.61 | 16.52 |
| 25.99 | 122.53 | 0.1023 | 3.43 | 44.60 |
| 27.16 | 73.84 | 0.2047 | 3.28 | 26.88 |
| 33.51 | 8.22 | 0.6140 | 2.67 | 2.99 |

Further, the present invention provides the crystalline form A of the fumarate of the compound of formula (I), and its X-ray powder diffraction pattern is substantially shown in Figure 46.

The present invention provides the crystalline form A of the fumarate of the compound of formula (I), and its differential scanning calorimetry (DSC) shows 3 endothermic peaks at 145.9°C, 162.5°C and 192. 1°C (peak temperature).

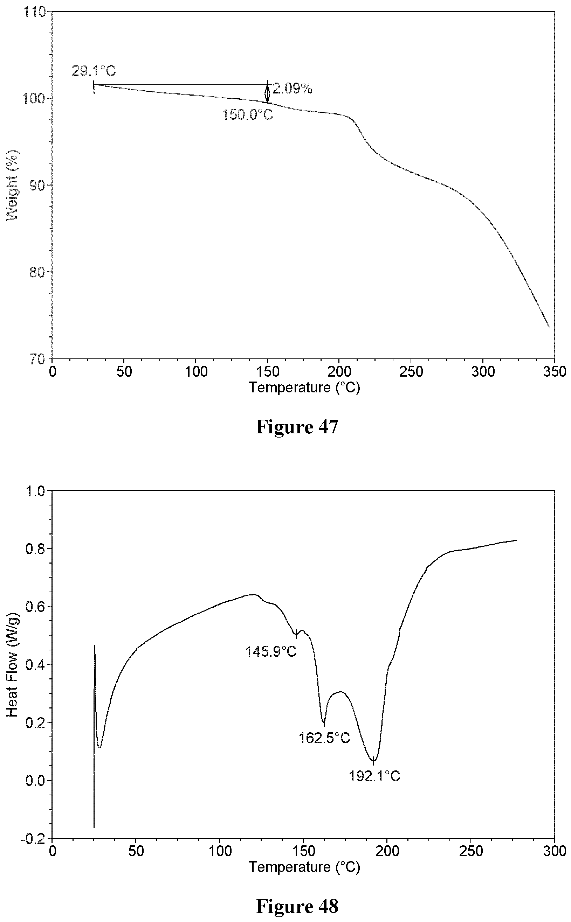

The present invention provides the crystalline form A of the fumarate of the compound of formula (I), and its differential scanning calorimetry (DSC) thermogram is shown in Figure 48.

The present invention provides the crystalline form A of the fumarate of the compound of formula (I), and its thermogravimetric analysis (TGA) thermogram shows 2.09% weight loss below 150°C.

The present invention provides the crystalline form A of the fumarate of the compound of formula (I), and its thermogravimetric analysis (TGA) thermogram is shown in Figure 47.

The present invention provides the crystalline form A of the fumarate of the compound of formula (I), which is an anhydrate.

The present invention also provides a fumarate of the compound of formula (I), which is in crystalline form (crystalline form B of the fumarate), and its X-ray powder diffraction pattern has characteristic diffraction peaks at the following 2θ positions: 12.23°±0.2°, 20.02°±0.2°, using Cu-Kα radiation.

The present invention provides the crystalline form B of the fumarate of the compound of formula (I), and its X-ray powder diffraction pattern also has characteristic diffraction peaks at the following 2θ positions: 16.09°±0.2°, 22.38°±0.2°, using Cu-Kα radiation.

The present invention provides the crystalline form B of the fumarate of the compound of formula (I), and the 2θ values and corresponding intensity in its X-ray powder diffraction pattern are shown in Table 15, and the error range of 2θ is ±0.2°.

**Table 15: 2θ values and corresponding intensity in XRPD pattern of the crystalline form B of the fumarate of the compound of formula (I)**

| Pos. [°2θ] | Height [cts] | FWHM Left [°2θ] | d-spacing [Å] | Rel. Int. [%] |
|---|---|---|---|---|
| 12.23 | 50.81 | 0.2007 | 7.24 | 57.88 |
| 16.09 | 44.30 | 0.2007 | 5.51 | 50.46 |
| 20.02 | 87.79 | 0.3011 | 4.44 | 100.00 |
| 22.38 | 12.70 | 0.8029 | 3.97 | 14.47 |

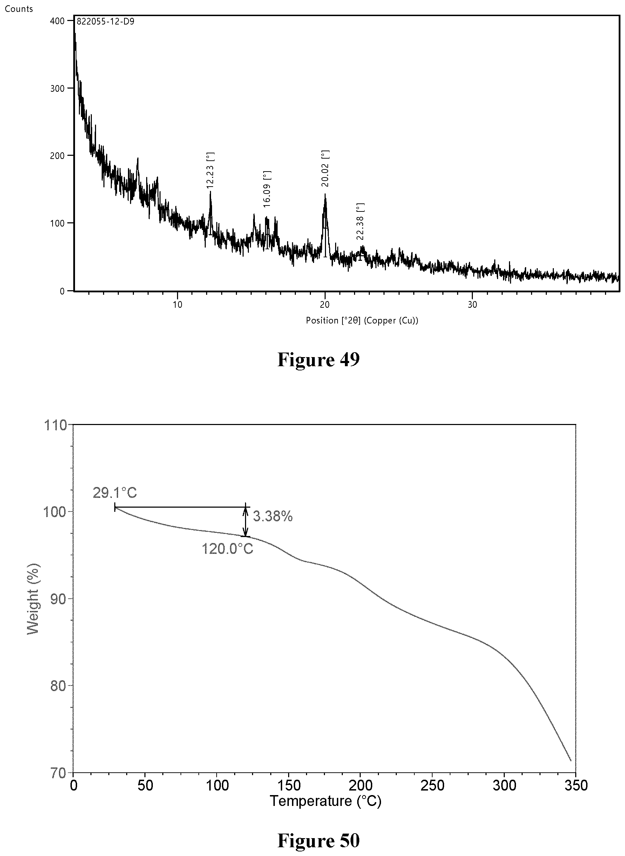

Further, the present invention provides the crystalline form B of the fumarate of the compound of formula (I), and its X-ray powder diffraction pattern is substantially shown in Figure 49.

The present invention provides the crystalline form B of the fumarate of the compound of formula (I), and its differential scanning calorimetry (DSC) shows 3 endothermic peaks at 62.8°C, 154.1°C and 190.9°C (peak temperature).

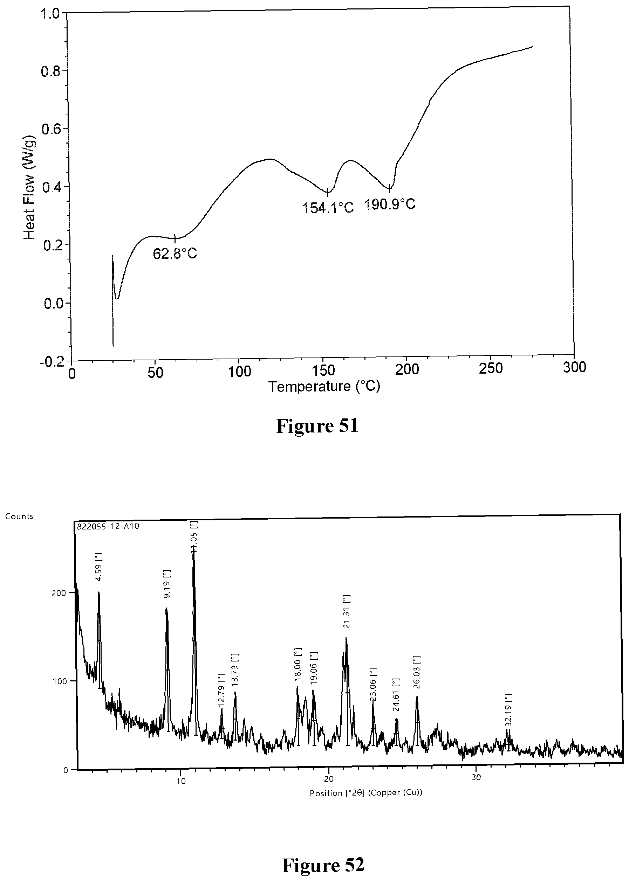

The present invention provides the crystalline form B of the fumarate of the compound of formula (I), and its differential scanning calorimetry (DSC) thermogram is shown in Figure 51.

The present invention provides the crystalline form B of the fumarate of the compound of formula (I), and its thermogravimetric analysis (TGA) thermogram shows 3.38% weight loss below 120°C.

The present invention provides the crystalline form B of the fumarate of the compound of formula (I), and its thermogravimetric analysis (TGA) thermogram is shown in Figure 50.

The present invention provides the crystalline form B of the fumarate of the compound of formula (I), which is a hydrate.

The present invention also provides a citrate of the compound of formula (I), which is in crystalline form (crystalline form A of the citrate), and its X-ray powder diffraction pattern has characteristic diffraction peaks at the following 2θ positions: 4.59°±0.2°, 9.19°±0.2°, 11.05°±0.2°, 18.00°±0.2°, 19.06°±0.2°, 21.31°±0.2°, using Cu-Kα radiation.

The present invention provides the crystalline form A of the citrate of the compound of formula (I), and its X-ray powder diffraction pattern also has characteristic diffraction peaks at the following 2θ positions: 12.79°±0.2°, 13.73°±0.2°, 23.06°±0.2°, 24.61°±0.2°, 26.03°±0.2°, using Cu-Kα radiation.

The present invention provides the crystalline form A of the citrate of the compound of formula (I), and the 2θ values and corresponding intensity in its X-ray powder diffraction pattern are shown in Table 16, and the error range of 2θ is ±0.2°.

**Table 16: 2θ values and corresponding intensity in XRPD pattern of the crystalline form A of the citrate of the compound of formula (I)**

| Pos. [°2θ] | Height [cts] | FWHM Left [°2θ] | d-spacing [Å] | Rel. Int. [%] |
|---|---|---|---|---|
| 4.59 | 108.78 | 0.2047 | 19.23 | 50.89 |
| 9.19 | 138.46 | 0.1791 | 9.63 | 64.77 |
| 11.05 | 213.76 | 0.1535 | 8.01 | 100.00 |
| 12.79 | 23.12 | 0.3070 | 6.92 | 10.82 |
| 13.73 | 48.29 | 0.2047 | 6.45 | 22.59 |
| 18.00 | 60.55 | 0.2047 | 4.93 | 28.33 |
| 19.06 | 56.16 | 0.2047 | 4.66 | 26.27 |
| 21.31 | 120.22 | 0.1535 | 4.17 | 56.24 |
| 23.06 | 37.77 | 0.3070 | 3.86 | 17.67 |
| 24.61 | 26.01 | 0.3070 | 3.62 | 12.17 |
| 26.03 | 51.74 | 0.2558 | 3.42 | 24.21 |
| 32.19 | 14.91 | 0.6140 | 2.78 | 6.97 |

Further, the present invention provides the crystalline form A of the citrate of the compound of formula (I), and its X-ray powder diffraction pattern is substantially shown in Figure 52.

The present invention provides the crystalline form A of the citrate of the compound of formula (I), and its differential scanning calorimetry (DSC) shows 2 endothermic peaks at 113.6°C and 174.6°C (peak temperature).

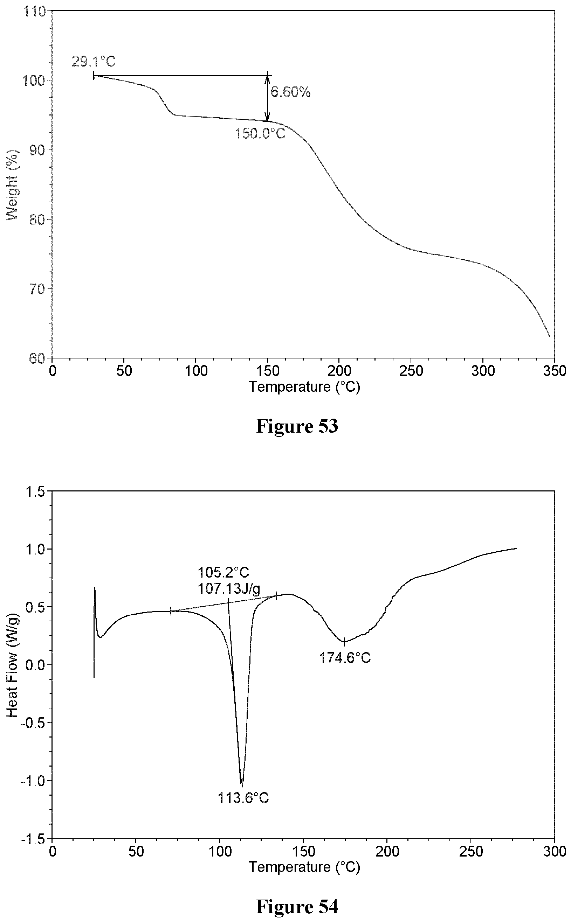

The present invention provides the crystalline form A of the citrate of the compound of formula (I), and its differential scanning calorimetry (DSC) thermogram is shown in Figure 54.

The present invention provides the crystalline form A of the citrate of the compound of formula (I), and its thermogravimetric analysis (TGA) thermogram shows 6.60% weight loss below 150°C.

The present invention provides the crystalline form A of the citrate of the compound of formula (I), and its thermogravimetric analysis (TGA) thermogram is shown in Figure 53.

The present invention provides the crystalline form A of the citrate of the compound of formula (I), which is a hydrate.

The present invention also provides a citrate of the compound of formula (I), which is in crystalline form (crystalline form B of the citrate), and its X-ray powder diffraction pattern has characteristic diffraction peaks at the following 2θ positions: 7.70°±0.2°, 8.74°±0.2°, 14.70°±0.2°, 16.42°±0.2°, 17.47°±0.2°, 28.70°±0.2°, using Cu-Kα radiation.

The present invention provides the crystalline form B of the citrate of the compound of formula (I), and its X-ray powder diffraction pattern also has characteristic diffraction peaks at the following 2θ positions: 13.20°±0.2°, 19.87°±0.2°, 22.23°±0.2°, 23.48°±0.2°, using Cu-Kα radiation.

The present invention provides the crystalline form B of the citrate of the compound of formula (I), and the 2θ values and corresponding intensity in its X-ray powder diffraction pattern are shown in Table 17, and the error range of 2θ is ±0.2°.

**Table 17: 2θ values and corresponding intensity in XRPD pattern of the crystalline form B of the citrate of the compound of formula (I)**

| Pos. [°2θ] | Height [cts] | FWHM Left [°2θ] | d-spacing [Å] | Rel. Int. [%] |
|---|---|---|---|---|
| 7.70 | 130.69 | 0.1279 | 11.48 | 100.00 |
| 8.74 | 78.73 | 0.1023 | 10.12 | 60.24 |
| 13.20 | 37.28 | 0.4093 | 6.71 | 28.52 |
| 14.70 | 102.97 | 0.2047 | 6.03 | 78.79 |
| 16.42 | 91.29 | 0.1023 | 5.40 | 69.85 |
| 17.47 | 80.57 | 0.1535 | 5.08 | 61.65 |
| 19.87 | 42.96 | 0.2047 | 4.47 | 32.87 |
| 22.23 | 34.29 | 0.2047 | 4.00 | 26.24 |
| 23.48 | 14.55 | 0.8187 | 3.79 | 11.13 |
| 28.70 | 54.44 | 0.2047 | 3.11 | 41.66 |

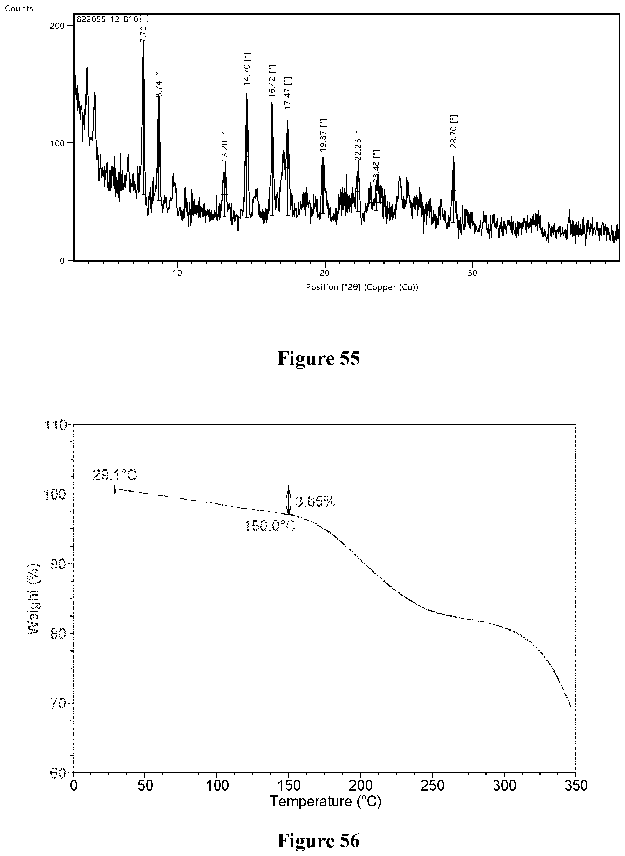

Further, the present invention provides the crystalline form B of the citrate of the compound of formula (I), and its X-ray powder diffraction pattern is substantially shown in Figure 55.

The present invention provides the crystalline form B of the citrate of the compound of formula (I), and its differential scanning calorimetry (DSC) shows 3 endothermic peaks at 98.9°C, 159.6°C and 183.1°C (peak temperature).

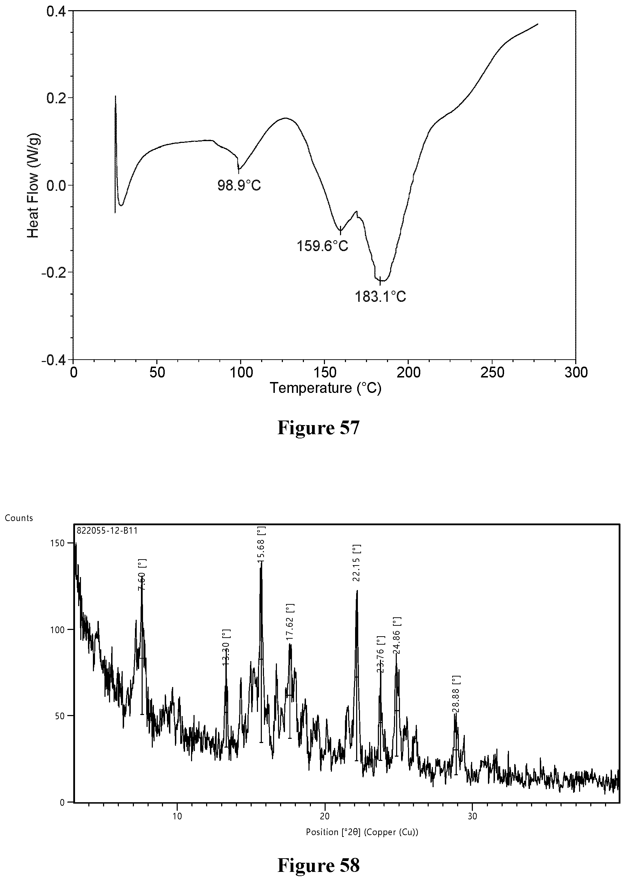

The present invention provides the crystalline form B of the citrate of the compound of formula (I), and its differential scanning calorimetry (DSC) thermogram is shown in Figure 57.

The present invention provides the crystalline form B of the citrate of the compound of formula (I), and its thermogravimetric analysis (TGA) thermogram shows 3.85% weight loss below 150°C.

The present invention provides the crystalline form B of the citrate of the compound of formula (I), and its thermogravimetric analysis (TGA) thermogram is shown in Figure 56.

The present invention provides the crystalline form B of the citrate of the compound of formula (I), which is an anhydrate.

The present invention also provides a malate of the compound of formula (I), which is in crystalline form (crystalline form A of the malate), and its X-ray powder diffraction pattern has characteristic diffraction peaks at the following 2θ positions: 8.75°±0.2°, 9.82°±0.2°, 15.08°±0.2°, 16.65°±0.2°, 20.89°±0.2°, 21.89°±0.2°, 23.75°±0.2°, using Cu-Kα radiation.

The present invention provides the crystalline form A of the malate of the compound of formula (I), and its X-ray powder diffraction pattern also has characteristic diffraction peaks at the following 2θ positions: 11.80°±0.2°, 14.04°±0.2°, 14.69°±0.2°, 24.81°±0.2°, 25.91°±0.2°, using Cu-Kα radiation.

The present invention provides the crystalline form A of the malate of the compound of formula (I), and the 2θ values and corresponding intensity in its X-ray powder diffraction pattern are shown in Table 18, and the error range of 2θ is ±0.2°.

**Table 18: 2θ values and corresponding intensity in XRPD pattern of the crystalline form A of the malate of the compound of formula (I)**

| Pos. [°2θ] | Height [cts] | FWHM Left [°2θ] | d-spacing [Å] | Rel. Int. [%] |
|---|---|---|---|---|
| 8.75 | 61.67 | 0.1535 | 10.11 | 9.53 |
| 9.82 | 86.81 | 0.2047 | 9.01 | 13.42 |
| 11.80 | 37.18 | 0.2047 | 7.50 | 5.75 |
| 14.04 | 37.01 | 0.1535 | 6.31 | 5.72 |
| 14.69 | 49.66 | 0.1535 | 6.03 | 7.68 |
| 15.08 | 94.25 | 0.1279 | 5.87 | 14.57 |
| 16.65 | 646.92 | 0.1279 | 5.32 | 100.00 |
| 20.89 | 184.11 | 0.1279 | 4.25 | 28.46 |
| 21.89 | 177.08 | 0.1279 | 4.06 | 27.37 |
| 23.75 | 68.95 | 0.1535 | 3.75 | 10.66 |
| 24.81 | 41.53 | 0.2047 | 3.59 | 6.42 |
| 25.91 | 49.02 | 0.1535 | 3.44 | 7.58 |
| 28.30 | 26.07 | 0.3070 | 3.15 | 4.03 |
| 29.78 | 14.25 | 0.6140 | 3.00 | 2.20 |

Further, the present invention provides the crystalline form A of the malate of the compound of formula (I), and its X-ray powder diffraction pattern is substantially shown in Figure 4.

The present invention provides the crystalline form A of the malate of the compound of formula (I), and its differential scanning calorimetry (DSC) shows an endothermic curve, in which Tₛₜₐᵣₜ = 176.2°C, Tₚₑₐₖ = 183.6°C, and ΔH = 78.06 J/g.

The present invention provides the crystalline form A of the malate of the compound of formula (I), and its differential scanning calorimetry (DSC) shows 2 endothermic peaks at 183.6°C and 207.1°C (peak temperature).

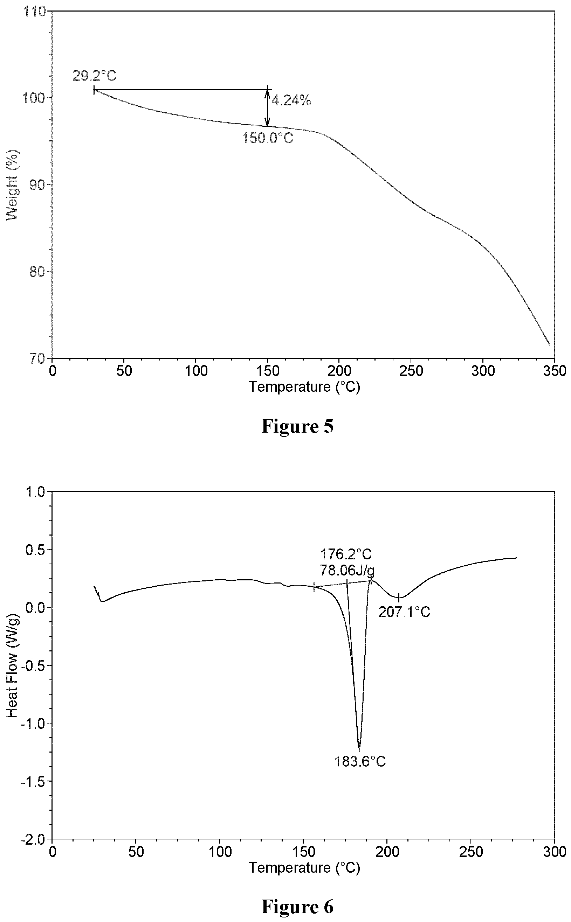

The present invention provides the crystalline form A of the malate of the compound of formula (I), and its differential scanning calorimetry (DSC) thermogram is shown in Figure 6.

The present invention provides the crystalline form A of the malate of the compound of formula (I), and its thermogravimetric analysis (TGA) thermogram shows 4.24% weight loss below 150°C, and the decomposition temperature is 200°C.

The present invention provides the crystalline form A of the malate of the compound of formula (I), and its thermogravimetric analysis (TGA) thermogram is shown in Figure 5.

The present invention provides the crystalline form A of the malate of the compound of formula (I), which is a hydrate.

The present invention also provides a malate of the compound of formula (I), which is in crystalline form (crystalline form B of the malate), and its X-ray powder diffraction pattern has characteristic diffraction peaks at the following 2θ positions: 7.60°±0.2°, 15.68°±0.2°, 22.15°±0.2°, 24.86°±0.2°, using Cu-Kα radiation.

The present invention provides the crystalline form B of the malate of the compound of formula (I), and its X-ray powder diffraction pattern also has characteristic diffraction peaks at the following 2θ positions: 13.30°±0.2°, 17.62°±0.2°, 23.76°±0.2°, 28.88°±0.2°, using Cu-Kα radiation.

The present invention provides the crystalline form B of the malate of the compound of formula (I), and the 2θ values and corresponding intensity in its X-ray powder diffraction pattern are shown in Table 19, and the error range of 2θ is ±0.2°.

**Table 19: 2θ values and corresponding intensity in XRPD pattern of the crystalline form B of the malate of the compound of formula (I)**

| Pos. [°2θ] | Height [cts] | FWHM Left [°2θ] | d-spacing [Å] | Rel. Int. [%] |
|---|---|---|---|---|
| 7.60 | 66.15 | 0.3070 | 11.63 | 67.01 |
| 13.30 | 40.61 | 0.2047 | 6.66 | 41.13 |
| 15.68 | 98.40 | 0.2047 | 5.65 | 99.68 |
| 17.62 | 50.40 | 0.5117 | 5.03 | 51.05 |
| 22.15 | 98.71 | 0.2047 | 4.01 | 100.00 |
| 23.76 | 44.54 | 0.2047 | 3.75 | 45.12 |
| 24.86 | 53.07 | 0.2558 | 3.58 | 53.76 |
| 28.88 | 29.71 | 0.3070 | 3.09 | 30.10 |

Further, the present invention provides the crystalline form B of the malate of the compound of formula (I), and its X-ray powder diffraction pattern is substantially shown in Figure 58.

The present invention provides the crystalline form B of the malate of the compound of formula (I), and its differential scanning calorimetry (DSC) shows an endothermic curve, in which Tₛₜₐᵣₜ = 172.3°C, Tₚₑₐₖ = 180.3°C, and ΔH = 59.25 J/g.

The present invention provides the crystalline form B of the malate of the compound of formula (I), and its differential scanning calorimetry (DSC) shows that its melting point is 180.3°C.

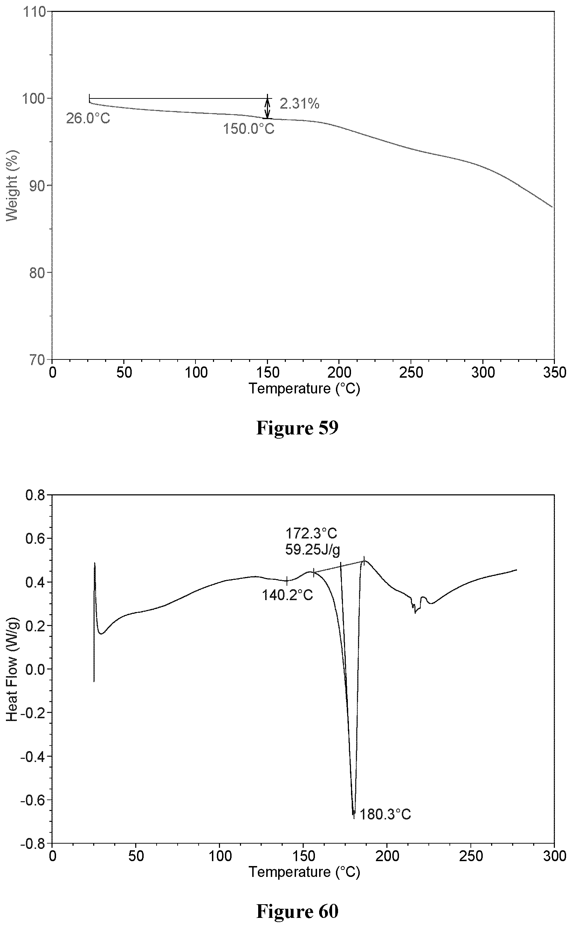

The present invention provides the crystalline form B of the malate of the compound of formula (I), and its differential scanning calorimetry (DSC) thermogram is shown in Figure 60.

The present invention provides the crystalline form B of the malate of the compound of formula (I), and its thermogravimetric analysis (TGA) thermogram shows 2.31% weight loss below 150°C.

The present invention provides the crystalline form B of the malate of the compound of formula (I), and its thermogravimetric analysis (TGA) thermogram is shown in Figure 59.

The present invention provides the crystalline form B of the malate of the compound of formula (I), which is an anhydrate.

The present invention also provides a hippurate of the compound of formula (I), which is in crystalline form (crystalline form A of the hippurate), and its X-ray powder diffraction pattern has characteristic diffraction peaks at the following 2θ positions: 7.64°±0.2°, 9.13°±0.2°, 15.33°±0.2°, 21.78°±0.2°, 25.22°±0.2°, using Cu-Kα radiation.

Further, the present invention provides the crystalline form A of the hippurate of the compound of formula (I), and its X-ray powder diffraction pattern also has characteristic diffraction peaks at the following 2θ positions: 11.43°±0.2°, 14.10°±0.2°, 16.05°±0.2°, 17.50°±0.2°, 18.31°±0.2°, 18.83°±0.2°, 22.45°±0.2°, 27.71°±0.2°, using Cu-Kα radiation.

The present invention provides the crystalline form A of the hippurate of the compound of formula (I), and the 2θ values and corresponding intensity in its X-ray powder diffraction pattern are shown in Table 20, and the error range of 2θ is ±0.2°.

**Table 20: 2θ values and corresponding intensity in XRPD pattern of the crystalline form A of the hippurate of the compound of formula (I)**

| Pos. [°2θ] | Height [cts] | FWHM Left [°2θ] | d-spacing [Å] | Rel. Int. [%] |
|---|---|---|---|---|
| 7.64 | 127.30 | 0.1535 | 11.57 | 41.08 |
| 9.13 | 116.09 | 0.2047 | 9.68 | 37.47 |
| 11.43 | 56.73 | 0.2047 | 7.74 | 18.31 |
| 14.10 | 43.93 | 0.3070 | 6.28 | 14.18 |
| 15.33 | 309.85 | 0.2047 | 5.78 | 100.00 |
| 16.05 | 90.55 | 0.2047 | 5.52 | 29.22 |
| 17.50 | 36.87 | 0.1535 | 5.07 | 11.90 |
| 18.31 | 66.44 | 0.2047 | 4.84 | 21.44 |
| 18.83 | 41.69 | 0.2047 | 4.71 | 13.45 |
| 21.78 | 204.06 | 0.1791 | 4.08 | 65.86 |
| 22.45 | 41.12 | 0.2047 | 3.96 | 13.27 |
| 25.22 | 84.64 | 0.2047 | 3.53 | 27.32 |
| 27.71 | 31.65 | 0.3070 | 3.22 | 10.21 |

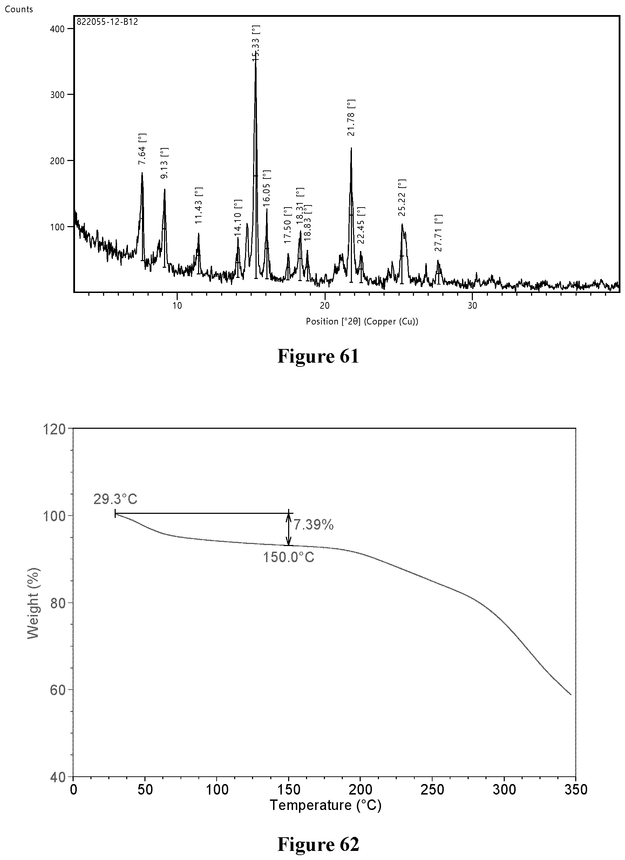

Further, the present invention provides the crystalline form A of the hippurate of the compound of formula (I), and its X-ray powder diffraction pattern is substantially shown in Figure 61.

The present invention provides the crystalline form A of the hippurate of the compound of formula (I), and its differential scanning calorimetry (DSC) shows 2 endothermic peaks at 88.7°C and 141.8°C (peak temperature).

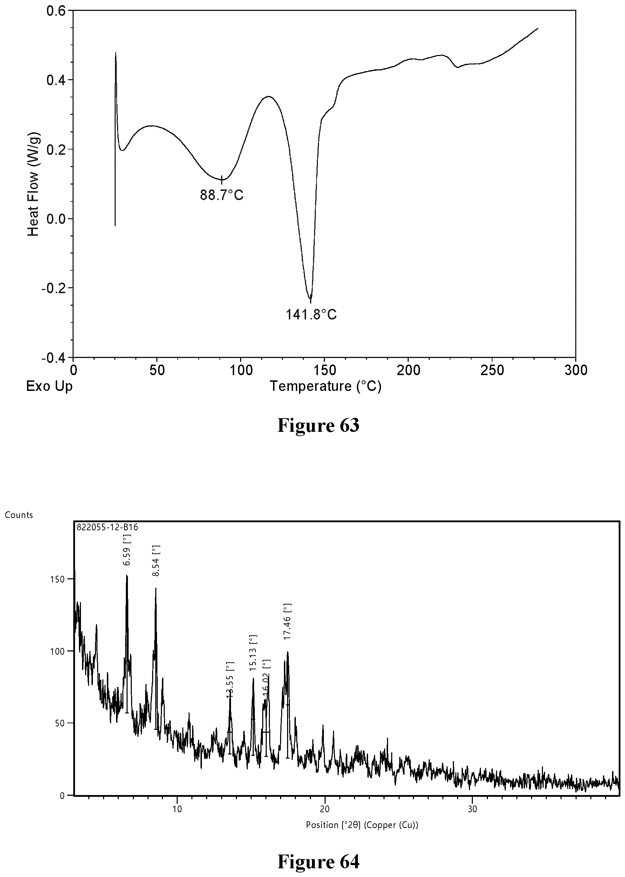

The present invention provides the crystalline form A of the hippurate of the compound of formula (I), and its differential scanning calorimetry (DSC) thermogram is shown in Figure 63.

The present invention provides the crystalline form A of the hippurate of the compound of formula (I), and its thermogravimetric analysis (TGA) thermogram shows 7.39% weight loss below 150°C.

The present invention provides the crystalline form A of the hippurate of the compound of formula (I), and its thermogravimetric analysis (TGA) thermogram is shown in Figure 62.

The present invention provides the crystalline form A of the hippurate of the compound of formula (I), which is a hydrate.

The present invention also provides an adipate of the compound of formula (I), which is in crystalline form (crystalline form A of the adipate), and its X-ray powder diffraction pattern has characteristic diffraction peaks at the following 2θ positions: 6.59°±0.2°, 8.54°±0.2°, 17.46°±0.2°, using Cu-Kα radiation.

Further, the present invention provides the crystalline form A of the adipate of the compound of formula (I), and its X-ray powder diffraction pattern also has characteristic diffraction peaks at the following 2θ positions: 13.55°±0.2°, 15.13°±0.2°, 16.02°±0.2°, using Cu-Kα radiation.

The present invention provides the crystalline form A of the adipate of the compound of formula (I), and the 2θ values and corresponding intensity in its X-ray powder diffraction pattern are shown in Table 21, and the error range of 2θ is ±0.2°.

**Table 21: 2θ values and corresponding intensity in XRPD pattern of the crystalline form A of the adipate of the compound of formula (I)**

| Pos. [°2θ] | Height [cts] | FWHM Left [°2θ] | d-spacing [Å] | Rel. Int. [%] |
|---|---|---|---|---|
| 6.59 | 94.48 | 0.1535 | 13.42 | 96.40 |
| 8.54 | 98.01 | 0.1023 | 10.36 | 100.00 |
| 13.55 | 29.90 | 0.3070 | 6.54 | 30.51 |
| 15.13 | 50.06 | 0.2047 | 5.86 | 51.08 |
| 16.02 | 33.63 | 0.4093 | 5.53 | 34.31 |
| 17.46 | 73.61 | 0.1535 | 5.08 | 75.11 |

Further, the present invention provides the crystalline form A of the adipate of the compound of formula (I), and its X-ray powder diffraction pattern is substantially shown in Figure 64.

The present invention provides the crystalline form A of the adipate of the compound of formula (I), and its differential scanning calorimetry (DSC) shows 2 endothermic peaks at 132.5°C and 153.0°C (peak temperature).

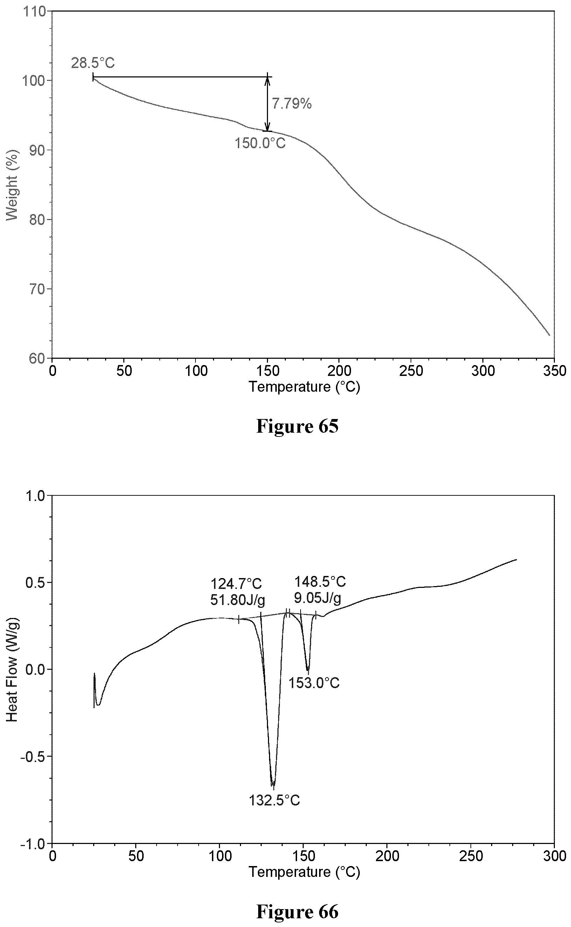

The present invention provides the crystalline form A of the adipate of the compound of formula (I), and its differential scanning calorimetry (DSC) thermogram is shown in Figure 66.

The present invention provides the crystalline form A of the adipate of the compound of formula (I), and its thermogravimetric analysis (TGA) thermogram shows 7.79% weight loss below 150°C.

The present invention provides the crystalline form A of the adipate of the compound of formula (I), and its thermogravimetric analysis (TGA) thermogram is shown in Figure 65.

The present invention provides the crystalline form A of the adipate of the compound of formula (I), which is a hydrate.

The present invention also provides an adipate of the compound of formula (I), which is in crystalline form (crystalline form B of the adipate), and its X-ray powder diffraction pattern has characteristic diffraction peaks at the following 2θ positions: 8.10°±0.2°, 12.18°±0.2°, 16.24°±0.2°, 17.68°±0.2°, 20.33°±0.2°, using Cu-Kα radiation.

Further, the present invention provides the crystalline form B of the adipate of the compound of formula (I), and its X-ray powder diffraction pattern also has characteristic diffraction peaks at the following 2θ positions: 10.68°±0.2°, 14.02°±0.2°, 19.60°±0.2°, 20.95°±0.2°, using Cu-Kα radiation.

The present invention provides the crystalline form B of the adipate of the compound of formula (I), and the 2θ values and corresponding intensity in its X-ray powder diffraction pattern are shown in Table 22, and the error range of 2θ is ±0.2°.

**Table 22: 2θ values and corresponding intensity in XRPD pattern of the crystalline form B of the adipate of the compound of formula (I)**

| Pos. [°2θ] | Height [cts] | FWHM Left [°2θ] | d-spacing [Å] | Rel. Int. [%] |
|---|---|---|---|---|
| 8.10 | 301.14 | 0.2047 | 10.92 | 71.14 |
| 10.68 | 50.18 | 0.1535 | 8.28 | 11.86 |
| 12.18 | 423.31 | 0.2303 | 7.27 | 100.00 |
| 14.02 | 46.75 | 0.1535 | 6.32 | 11.04 |
| 16.24 | 166.27 | 0.1535 | 5.46 | 39.28 |
| 17.68 | 182.19 | 0.1535 | 5.02 | 43.04 |
| 19.60 | 58.72 | 0.2558 | 4.53 | 13.87 |
| 20.33 | 86.95 | 0.1535 | 4.37 | 20.54 |
| 20.95 | 59.32 | 0.2047 | 4.24 | 14.01 |
| 21.26 | 24.43 | 0.8187 | 4.18 | 5.77 |
| 24.33 | 19.61 | 0.4093 | 3.66 | 4.63 |

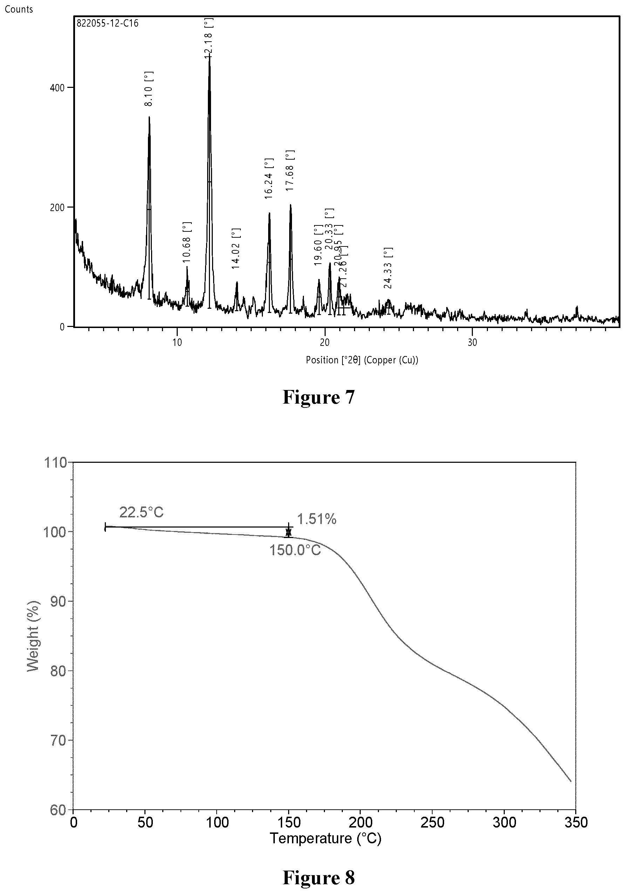

Further, the present invention provides the crystalline form B of the adipate of the compound of formula (I), and its X-ray powder diffraction pattern is substantially shown in Figure 7.

The present invention provides the crystalline form B of the adipate of the compound of formula (I), and its differential scanning calorimetry (DSC) shows an endothermic curve, in which Tₛₜₐᵣₜ = 152.0°C, Tₚₑₐₖ = 154.3°C, and ΔH = 87.83 J/g.

The present invention provides the crystalline form B of the adipate of the compound of formula (I), and its differential scanning calorimetry (DSC) shows that its melting point is 154.3°C.

The present invention provides the crystalline form B of the adipate of the compound of formula (I), and its differential scanning calorimetry (DSC) thermogram is shown in Figure 9.

The present invention provides the crystalline form B of the adipate of the compound of formula (I), and its thermogravimetric analysis (TGA) thermogram shows 0.51% weight loss below 150°C, and the decomposition temperature is 160°C.

The present invention provides the crystalline form B of the adipate of the compound of formula (I), and its thermogravimetric analysis (TGA) thermogram is shown in Figure 8.

The present invention provides the crystalline form B of the adipate of the compound of formula (I), which is an anhydrate.

The present invention also provides a sebacate of the compound of formula (I), which is in crystalline form (crystalline form A of the sebacate), and its X-ray powder diffraction pattern has characteristic diffraction peaks at the following 2θ positions: 8.91°±0.2°, 12.12°±0.2°, 20.39°±0.2°, 25.08°±0.2°, using Cu-Kα radiation.

Further, the present invention provides the crystalline form A of the sebacate of the compound of formula (I), and its X-ray powder diffraction pattern also has characteristic diffraction peaks at the following 2θ positions: 14.14°±0.2°, 16.01°±0.2°, 18.96°±0.2°, 23.90°±0.2°, using Cu-Kα radiation.

The present invention provides the crystalline form A of the sebacate of the compound of formula (I), and the 2θ values and corresponding intensity in its X-ray powder diffraction pattern are shown in Table 23, and the error range of 2θ is ±0.2°.

**Table 23: 2θ values and corresponding intensity in XRPD pattern of the crystalline form A of the sebacate of the compound of formula (I)**

| Pos. [°2θ] | Height [cts] | FWHM Left [°2θ] | d-spacing [Å] | Rel. Int. [%] |
|---|---|---|---|---|
| 8.91 | 70.41 | 0.1535 | 9.92 | 37.97 |
| 12.12 | 174.51 | 0.1279 | 7.30 | 94.11 |
| 14.14 | 64.93 | 0.2047 | 6.27 | 35.01 |
| 16.01 | 52.13 | 0.2558 | 5.53 | 28.11 |
| 18.96 | 37.05 | 0.4093 | 4.68 | 19.98 |
| 20.39 | 185.44 | 0.1279 | 4.36 | 100.00 |
| 23.90 | 55.83 | 0.1535 | 3.72 | 30.11 |
| 25.08 | 133.35 | 0.1023 | 3.55 | 71.91 |

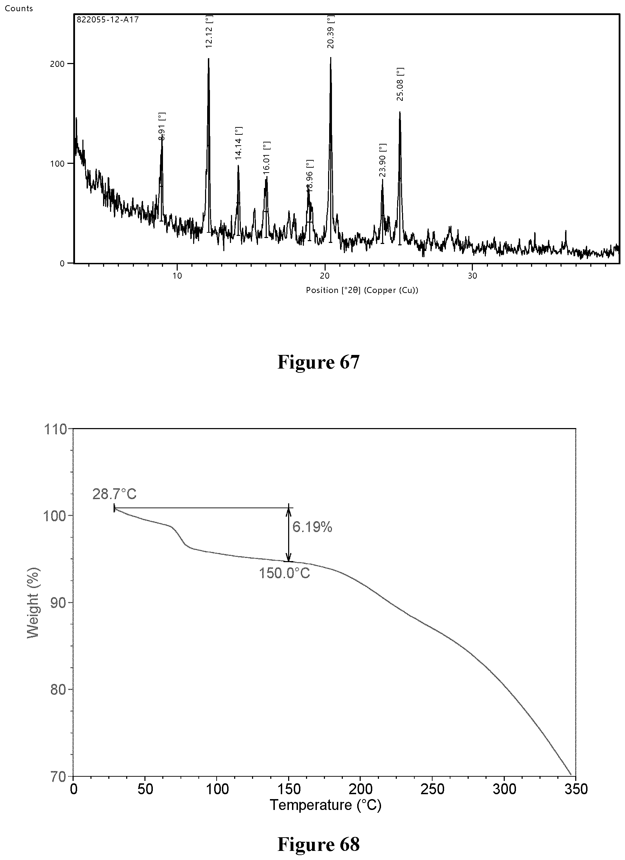

Further, the present invention provides the crystalline form A of the sebacate of the compound of formula (I), and its X-ray powder diffraction pattern is substantially shown in Figure 67.

The present invention provides the crystalline form A of the sebacate of the compound of formula (I), and its differential scanning calorimetry (DSC) shows 3 endothermic peaks at 102.6°C, 160.1°C and 173.7°C (peak temperature).

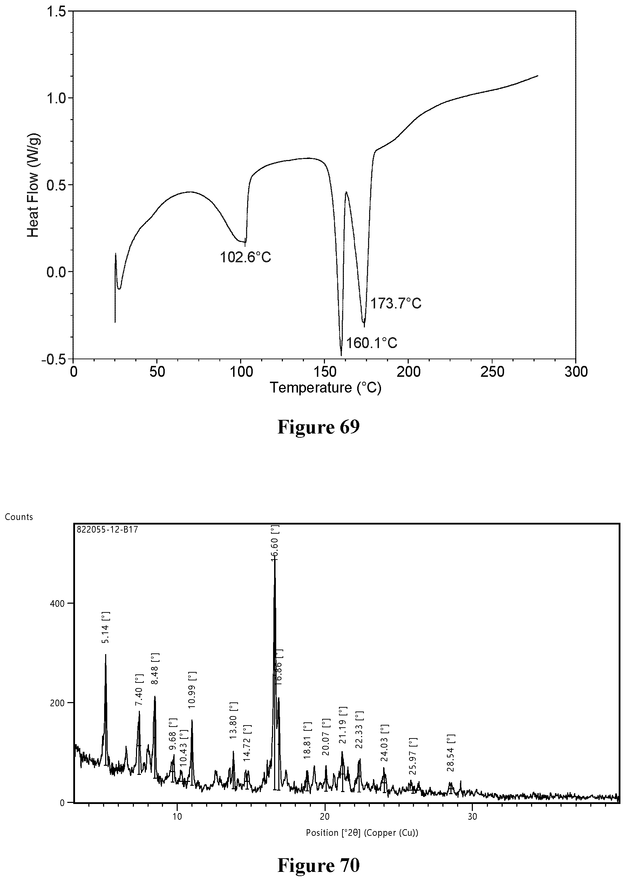

The present invention provides the crystalline form A of the sebacate of the compound of formula (I), and its differential scanning calorimetry (DSC) thermogram is shown in Figure 69.

The present invention provides the crystalline form A of the sebacate of the compound of formula (I), and its thermogravimetric analysis (TGA) thermogram shows 6.19% weight loss below 150°C.

The present invention provides the crystalline form A of the sebacate of the compound of formula (I), and its thermogravimetric analysis (TGA) thermogram is shown in Figure 68.

The present invention provides the crystalline form A of the sebacate of the compound of formula (I), which is a hydrate.

The present invention also provides a sebacate of the compound of formula (I), which is in crystalline form (crystalline form B of the sebacate), and its X-ray powder diffraction pattern has characteristic diffraction peaks at the following 2θ positions: 5.14°±0.2°, 7.40°±0.2°, 8.48°±0.2°, 10.99°±0.2°, 16.60°±0.2°, 16.86°±0.2°, using Cu-Kα radiation.

Further, the present invention provides the crystalline form B of the sebacate of the compound of formula (I), and its X-ray powder diffraction pattern also has characteristic diffraction peaks at the following 2θ positions: 13.80°±0.2°, 20.07°±0.2°, 21.19°±0.2°, 22.33°±0.2°, using Cu-Kα radiation.

The present invention provides the crystalline form B of the sebacate of the compound of formula (I), and the 2θ values and corresponding intensity in its X-ray powder diffraction pattern are shown in Table 24, and the error range of 2θ is ±0.2°.

**Table 24: 2θ values and corresponding intensity in XRPD pattern of the crystalline form B of the sebacate of the compound of formula (I)**

| Pos. [°2θ] | Height [cts] | FWHM Left [°2θ] | d-spacing [Å] | Rel. Int. [%] |
|---|---|---|---|---|
| 5.14 | 221.36 | 0.1023 | 17.19 | 48.12 |
| 7.40 | 113.83 | 0.2303 | 11.94 | 24.74 |
| 8.48 | 164.11 | 0.1279 | 10.43 | 35.67 |
| 9.68 | 40.69 | 0.2558 | 9.13 | 8.84 |
| 10.43 | 9.85 | 0.8187 | 8.48 | 2.14 |
| 10.99 | 129.41 | 0.1023 | 8.05 | 28.13 |
| 13.80 | 75.59 | 0.1023 | 6.42 | 16.43 |
| 14.72 | 30.97 | 0.3070 | 6.02 | 6.73 |
| 16.60 | 460.05 | 0.1535 | 5.34 | 100.00 |
| 16.86 | 184.40 | 0.1023 | 5.26 | 40.08 |
| 18.81 | 39.07 | 0.1535 | 4.72 | 8.49 |
| 20.07 | 47.78 | 0.2047 | 4.42 | 10.39 |
| 21.19 | 71.90 | 0.1535 | 4.19 | 15.63 |
| 22.33 | 62.80 | 0.1535 | 3.98 | 13.65 |
| 24.03 | 38.06 | 0.2558 | 3.70 | 8.27 |
| 25.97 | 12.71 | 0.8187 | 3.43 | 2.76 |
| 28.54 | 20.08 | 0.3070 | 3.13 | 4.36 |

Further, the present invention provides the crystalline form B of the sebacate of the compound of formula (I), and its X-ray powder diffraction pattern is substantially shown in Figure 70.

The present invention provides the crystalline form B of the sebacate of the compound of formula (I), and its differential scanning calorimetry (DSC) shows 3 endothermic peaks at 53.2°C, 110.8°C and 157.8°C (peak temperature).

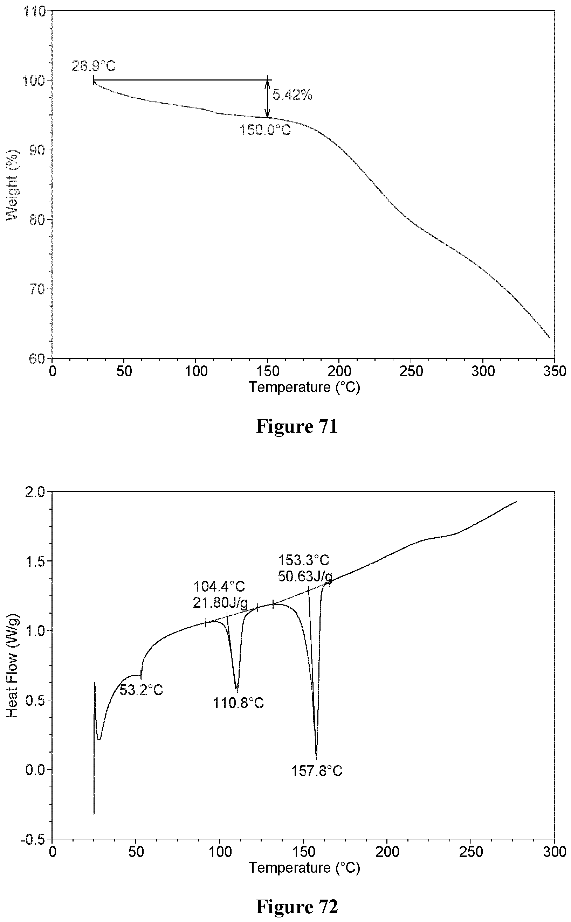

The present invention provides the crystalline form B of the sebacate of the compound of formula (I), and its differential scanning calorimetry (DSC) thermogram is shown in Figure 72.

The present invention provides the crystalline form B of the sebacate of the compound of formula (I), and its thermogravimetric analysis (TGA) thermogram shows 5.4% weight loss below 150°C.

The present invention provides the crystalline form B of the sebacate of the compound of formula (I), and its thermogravimetric analysis (TGA) thermogram is shown in Figure 71.

The present invention provides the crystalline form B of the sebacate of the compound of formula (I), which is a hydrate.

The present invention also provides a sebacate of the compound of formula (I), which is in crystalline form (crystalline form C of the sebacate), and its X-ray powder diffraction pattern has characteristic diffraction peaks at the following 2θ positions: 5.02°±0.2°, 7.52°±0.2°, 15.08°±0.2°, 19.91°±0.2°, using Cu-Kα radiation.

Further, the present invention provides the crystalline form C of the sebacate of the compound of formula (I), and its X-ray powder diffraction pattern also has characteristic diffraction peaks at the following 2θ positions: 12.37°±0.2°, 18.68°±0.2°, 21.52°±0.2°, 22.85°±0.2°, 24.07°±0.2°, using Cu-Kα radiation.

The present invention provides the crystalline form C of the sebacate of the compound of formula (I), and the 2θ values and corresponding intensity in its X-ray powder diffraction pattern are shown in Table 25, and the error range of 2θ is ±0.2°.

**Table 25: 2θ values and corresponding intensity in XRPD pattern of the crystalline form C of the sebacate of the compound of formula (I)**

| Pos. [°2θ] | Height [cts] | FWHM Left [°2θ] | d-spacing [Å] | Rel. Int. [%] |
|---|---|---|---|---|
| 5.02 | 265.03 | 0.1279 | 17.59 | 100.00 |
| 7.52 | 121.51 | 0.2047 | 11.76 | 45.85 |
| 12.37 | 86.77 | 0.2047 | 7.15 | 32.74 |
| 15.08 | 103.57 | 0.2558 | 5.88 | 39.08 |
| 18.68 | 44.98 | 0.1535 | 4.75 | 16.97 |
| 19.91 | 99.56 | 0.2558 | 4.46 | 37.56 |
| 21.52 | 44.94 | 0.2047 | 4.13 | 16.95 |
| 22.85 | 28.62 | 0.3070 | 3.89 | 10.80 |
| 24.07 | 54.43 | 0.3070 | 3.70 | 20.54 |

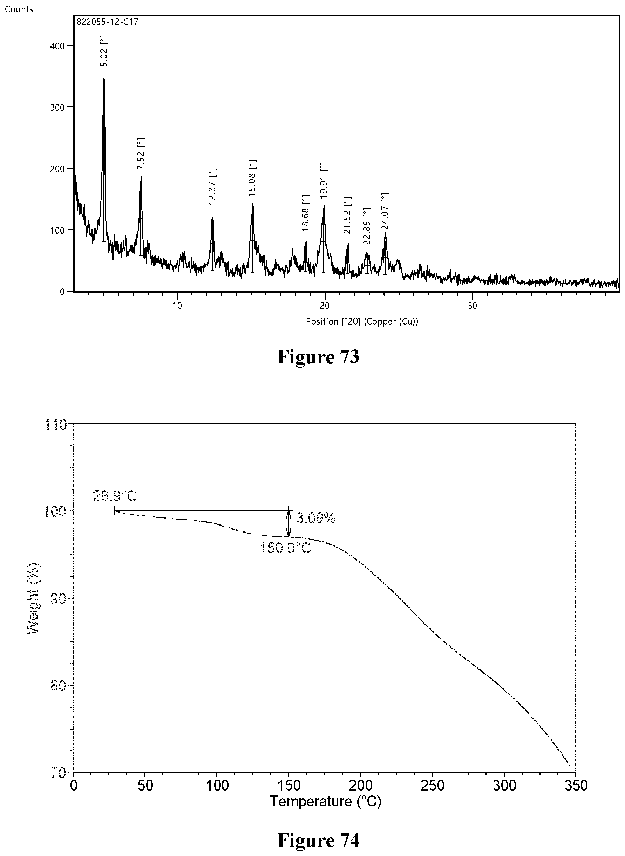

Further, the present invention provides the crystalline form C of the sebacate of the compound of formula (I), and its X-ray powder diffraction pattern is substantially shown in Figure 73.

The present invention provides the crystalline form C of the sebacate of the compound of formula (I), and its differential scanning calorimetry (DSC) shows 3 endothermic peaks at 111.4°C, 125.1°C and 156.4°C (peak temperature).

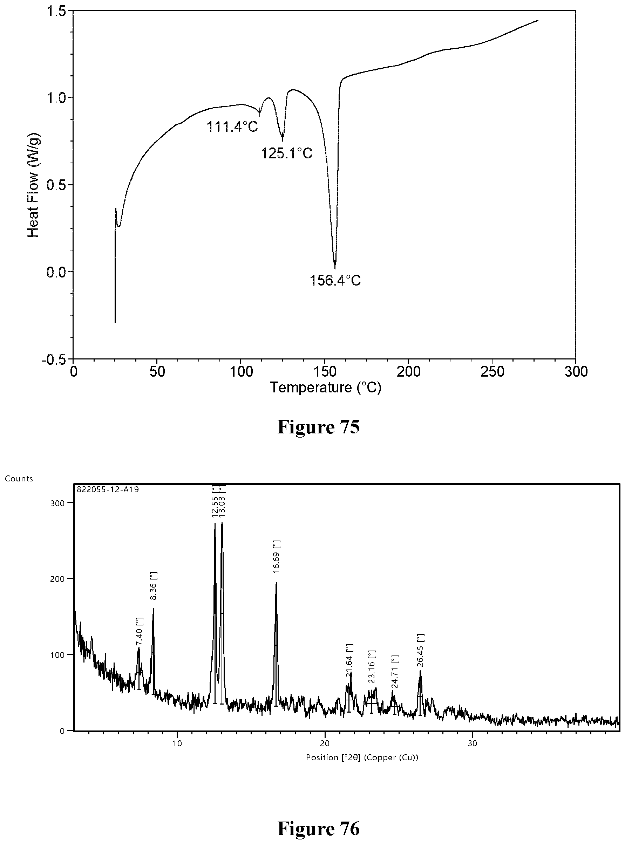

The present invention provides the crystalline form C of the sebacate of the compound of formula (I), and its differential scanning calorimetry (DSC) thermogram is shown in Figure 75.

The present invention provides the crystalline form C of the sebacate of the compound of formula (I), and its thermogravimetric analysis (TGA) thermogram shows 3.1% weight loss below 150°C.

The present invention provides the crystalline form C of the sebacate of the compound of formula (I), and its thermogravimetric analysis (TGA) thermogram is shown in Figure 74.

The present invention provides the crystalline form C of the sebacate of the compound of formula (I), which is an anhydrate.

The present invention also provides a 1,5-naphthalenedisulfonate of the compound of formula (I), which is in crystalline form (crystalline form A of the 1,5-naphthalenedisulfonate), and its X-ray powder diffraction pattern has characteristic diffraction peaks at the following 2θ positions: 8.36°±0.2°, 12.55°±0.2°, 13.03°±0.2°, and 16.69°±0.2°, using Cu-Kα radiation.

Further, the present invention provides the crystalline form A of the 1,5-naphthalenedisulfonate of the compound of formula (I), and its X-ray powder diffraction pattern also has characteristic diffraction peaks at the following 2θ positions: 7.40°±0.2°, 21.64°±0.2°, 23.16°±0.2°, 26.45°±0.2°, using Cu-Kα radiation.

The present invention provides the crystalline form A of the 1,5-naphthalenedisulfonate of the compound of formula (I), and the 2θ values and corresponding intensity in its X-ray powder diffraction pattern are shown in Table 26, and the error range of 2θ is ±0.2°.

**Table 26: 2θ values and corresponding intensity in XRPD pattern of the crystalline form A of the 1,5-naphthalenedisulfonate of the compound of formula (I)**

| Pos. [°2θ] | Height [cts] | FWHM Left [°2θ] | d-spacing [Å] | Rel. Int. [%] |
|---|---|---|---|---|
| 7.40 | 44.06 | 0.3070 | 11.94 | 18.46 |
| 8.36 | 110.29 | 0.1023 | 10.57 | 46.20 |
| 12.55 | 237.88 | 0.1023 | 7.05 | 99.64 |
| 13.03 | 238.73 | 0.1791 | 6.80 | 100.00 |
| 16.69 | 160.65 | 0.1791 | 5.31 | 67.29 |
| 21.64 | 31.89 | 0.5117 | 4.11 | 13.36 |
| 23.16 | 24.97 | 0.6140 | 3.84 | 10.46 |
| 24.71 | 19.89 | 0.6140 | 3.60 | 8.33 |
| 26.45 | 49.12 | 0.2558 | 3.37 | 20.57 |

Further, the present invention provides the crystalline form A of the 1,5-naphthalenedisulfonate of the compound of formula (I), and its X-ray powder diffraction pattern is substantially shown in Figure 76.

The present invention provides the crystalline form A of the 1,5-naphthalenedisulfonate of the compound of formula (I), and its differential scanning calorimetry (DSC) shows 2 endothermic peaks at 65.4°C and 231.1°C (peak temperature).

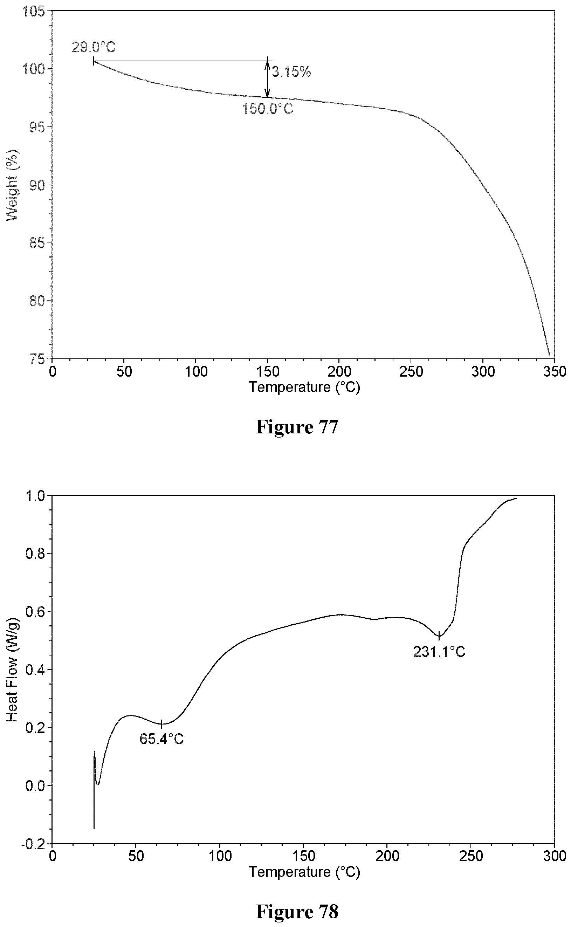

The present invention provides the crystalline form A of the 1,5-naphthalenedisulfonate of the compound of formula (I), and its differential scanning calorimetry (DSC) thermogram is shown in Figure 78.

The present invention provides the crystalline form A of the 1,5-naphthalenedisulfonate of the compound of formula (I), and its thermogravimetric analysis (TGA) thermogram shows 3.2% weight loss below 150°C.

The present invention provides the crystalline form A of the 1,5-naphthalenedisulfonate of the compound of formula (I), and its thermogravimetric analysis (TGA) thermogram is shown in Figure 77.

The present invention provides the crystalline form A of the 1,5-naphthalenedisulfonate of the compound of formula (I), which is a hydrate.

The present invention also provides a methanesulfonate of the compound of formula (I), which is in crystalline form (crystalline form A of the methanesulfonate), and its X-ray powder diffraction pattern has characteristic diffraction peaks at the following 2θ positions: 8.41°±0.2°, 13.22±0.2°, 16.95°±0.2°, 20.50°±0.2°, 20.86°±0.2°, 21.89°±0.2°, 22.48°±0.2°, 24.38°±0.2°, using Cu-Kα radiation.

Further, the present invention provides the crystalline form A of the methanesulfonate of the compound of formula (I), and its X-ray powder diffraction pattern also has characteristic diffraction peaks at the following 2θ positions: 10.39°±0.2°, 12.32°±0.2°, 18.06°±0.2°, 25.31°±0.2°, 26.15°±0.2°, 32.19°±0.2°, using Cu-Kα radiation.

The present invention provides the crystalline form A of the methanesulfonate of the compound of formula (I), and the 2θ values and corresponding intensity in its X-ray powder diffraction pattern are shown in Table 27, and the error range of 2θ is ±0.2°.

**Table 27: 2θ values and corresponding intensity in XRPD pattern of the crystalline form A of the methanesulfonate of the compound of formula (I)**

| Pos. [°2θ] | Height [cts] | FWHM Left [°2θ] | d-spacing [Å] | Rel. Int. [%] |
|---|---|---|---|---|
| 8.41 | 252.03 | 0.0768 | 10.52 | 55.68 |
| 10.39 | 64.99 | 0.1023 | 8.52 | 14.36 |
| 12.32 | 84.99 | 0.1023 | 7.18 | 18.78 |
| 13.22 | 125.94 | 0.0768 | 6.70 | 27.82 |
| 14.17 | 44.95 | 0.1535 | 6.25 | 9.93 |
| 16.95 | 403.14 | 0.0768 | 5.23 | 89.06 |
| 18.06 | 50.47 | 0.2047 | 4.91 | 11.15 |
| 20.50 | 452.66 | 0.1023 | 4.33 | 100.00 |
| 20.86 | 299.89 | 0.1023 | 4.26 | 66.25 |
| 21.89 | 100.02 | 0.1023 | 4.06 | 22.09 |
| 22.48 | 110.50 | 0.1279 | 3.96 | 24.41 |
| 24.38 | 118.20 | 0.1023 | 3.65 | 26.11 |
| 24.77 | 40.15 | 0.1535 | 3.60 | 8.87 |
| 25.31 | 94.63 | 0.1791 | 3.52 | 20.91 |
| 26.15 | 64.57 | 0.1023 | 3.41 | 14.26 |
| 27.00 | 32.89 | 0.2047 | 3.30 | 7.27 |
| 28.53 | 22.09 | 0.2047 | 3.13 | 4.88 |
| 32.19 | 48.55 | 0.1279 | 2.78 | 10.73 |

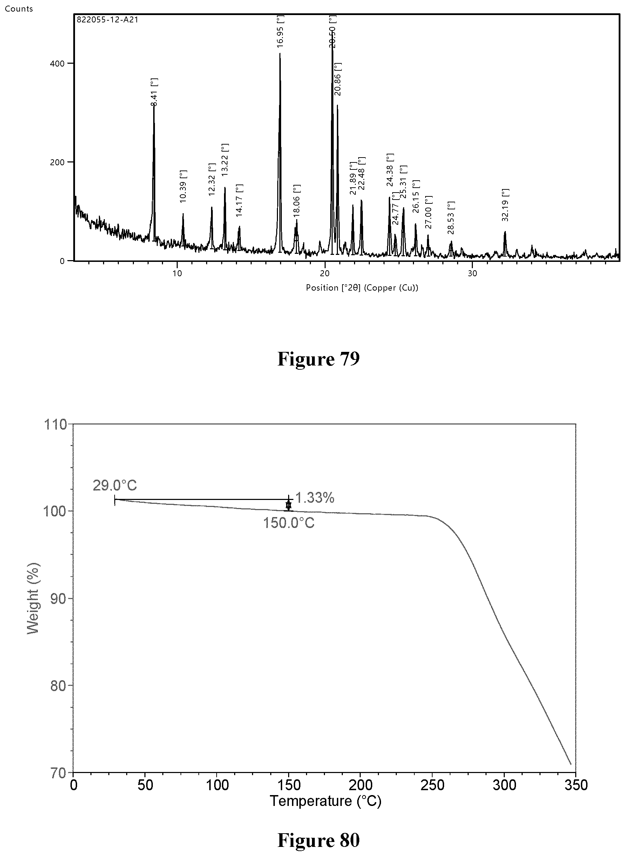

Further, the present invention provides the crystalline form A of the methanesulfonate of the compound of formula (I), and its X-ray powder diffraction pattern is substantially shown in Figure 79.

The present invention provides the crystalline form A of the methanesulfonate of the compound of formula (I), and its differential scanning calorimetry (DSC) shows an endothermic curve, in which Tₛₜₐᵣₜ = 238.9°C and Tₚₑₐₖ = 242.5°C.

The present invention provides the crystalline form A of the methanesulfonate of the compound of formula (I), and its differential scanning calorimetry (DSC) shows that its melting point is 242.5°C.

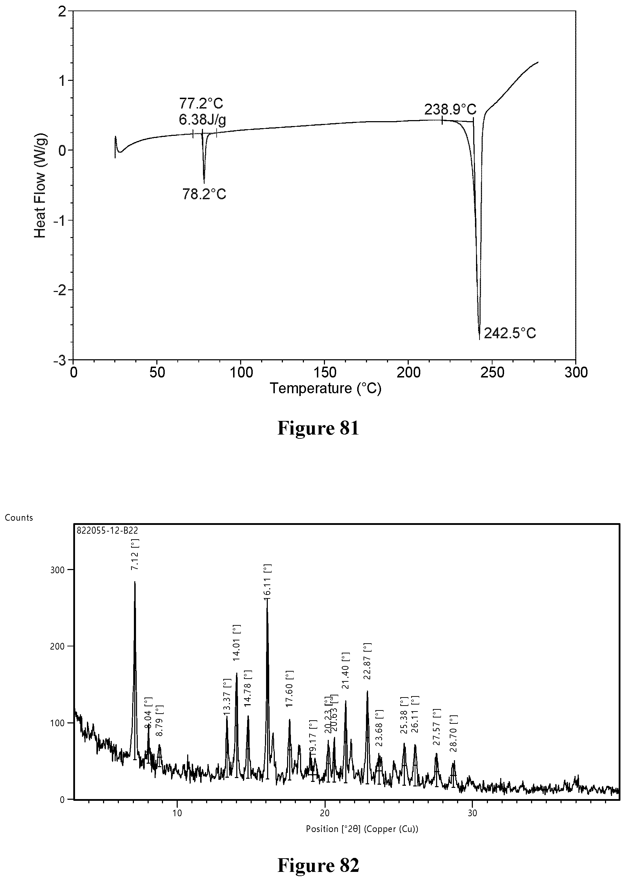

The present invention provides the crystalline form A of the methanesulfonate of the compound of formula (I), and its differential scanning calorimetry (DSC) thermogram is shown in Figure 81.

The present invention provides the crystalline form A of the methanesulfonate of the compound of formula (I), and its thermogravimetric analysis (TGA) thermogram shows 1.3% weight loss below 150°C, and the decomposition temperature is 250°C.

The present invention provides the crystalline form A of the methanesulfonate of the compound of formula (I), and its thermogravimetric analysis (TGA) thermogram is shown in Figure 80.

The present invention provides the crystalline form A of the methanesulfonate of the compound of formula (I), which is an anhydrate.

The present invention also provides a benzenesulfonate of the compound of formula (I), which is in crystalline form (crystalline form A of the benzenesulfonate), and its X-ray powder diffraction pattern has characteristic diffraction peaks at the following 2θ positions: 7.12°±0.2°, 14.01°±0.2°, 16.11°±0.2°, 21.40°±0.2°, 22.87°±0.2°, using Cu-Kα radiation.

Further, the present invention provides the crystalline form A of the benzenesulfonate of the compound of formula (I), and its X-ray powder diffraction pattern also has characteristic diffraction peaks at the following 2θ positions: 13.37°±0.2°, 14.78°±0.2°, 17.60°±0.2°, 20.23°±0.2°, 20.63°±0.2°, 25.38°±0.2°, 26.11°±0.2°, 27.57°±0.2°, using Cu-Kα radiation.

The present invention provides the crystalline form A of the benzenesulfonate of the compound of formula (I), and the 2θ values and corresponding intensity in its X-ray powder diffraction pattern are shown in Table 28, and the error range of 2θ is ±0.2°.

**Table 28: 2θ values and corresponding intensity in XRPD pattern of the crystalline form A of the benzenesulfonate of the compound of formula (I)**

| Pos. [°2θ] | Height [cts] | FWHM Left [°2θ] | d-spacing [Å] | Rel. Int. [%] |
|---|---|---|---|---|
| 7.12 | 232.47 | 0.1023 | 12.41 | 100.00 |
| 8.04 | 30.27 | 0.3070 | 11.00 | 13.02 |
| 8.79 | 24.56 | 0.3070 | 10.06 | 10.56 |
| 13.37 | 64.95 | 0.1535 | 6.62 | 27.94 |
| 14.01 | 136.62 | 0.1279 | 6.32 | 58.77 |
| 14.78 | 77.33 | 0.1535 | 5.99 | 33.26 |
| 16.11 | 218.12 | 0.1535 | 5.50 | 93.83 |
| 17.60 | 79.40 | 0.1791 | 5.04 | 34.16 |
| 19.17 | 16.57 | 0.6140 | 4.63 | 7.13 |
| 20.23 | 47.81 | 0.2047 | 4.39 | 20.57 |
| 20.63 | 51.04 | 0.1535 | 4.31 | 21.96 |
| 21.40 | 106.62 | 0.1279 | 4.15 | 45.86 |
| 22.87 | 120.54 | 0.1535 | 3.89 | 51.85 |
| 23.68 | 34.09 | 0.3070 | 3.76 | 14.66 |
| 25.38 | 51.70 | 0.2047 | 3.51 | 22.24 |
| 26.11 | 53.67 | 0.2047 | 3.41 | 23.09 |
| 27.57 | 38.76 | 0.3070 | 3.23 | 16.67 |
| 28.70 | 31.20 | 0.4093 | 3.11 | 13.42 |

Further, the present invention provides the crystalline form A of the benzenesulfonate of the compound of formula (I), and its X-ray powder diffraction pattern is substantially shown in Figure 82.

The present invention provides the crystalline form A of the benzenesulfonate of the compound of formula (I), and its differential scanning calorimetry (DSC) shows 2 endothermic peaks at 66.9°C and 151.0°C (peak temperature).

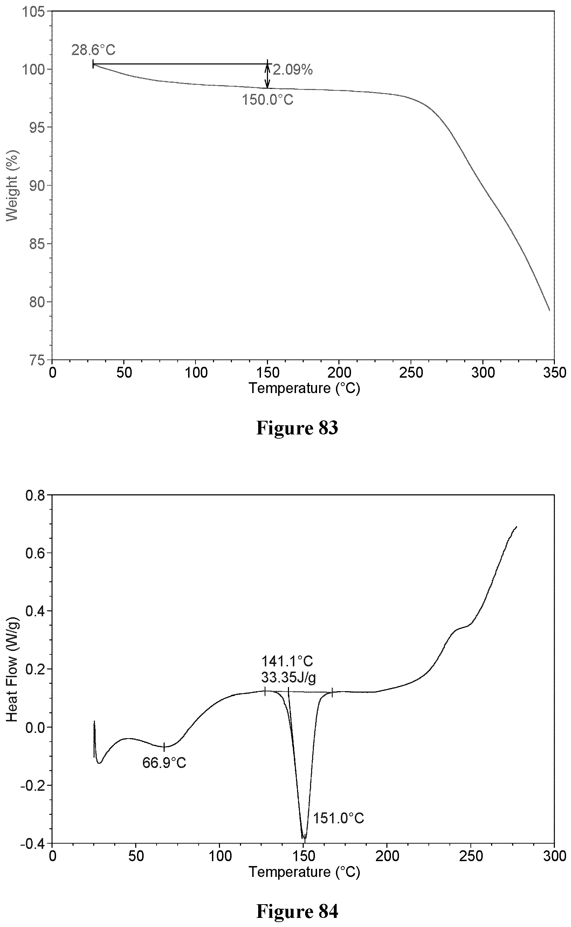

The present invention provides the crystalline form A of the benzenesulfonate of the compound of formula (I), and its differential scanning calorimetry (DSC) thermogram is shown in Figure 84.

The present invention provides the crystalline form A of the benzenesulfonate of the compound of formula (I), and its thermogravimetric analysis (TGA) thermogram shows 2.1% weight loss below 150°C.

The present invention provides the crystalline form A of the benzenesulfonate of the compound of formula (I), and its thermogravimetric analysis (TGA) thermogram is shown in Figure 83.

The present invention provides the crystalline form A of the benzenesulfonate of the compound of formula (I), which is an anhydrate.

The present invention also provides a benzenesulfonate of the compound of formula (I), which is in crystalline form (crystalline form B of the benzenesulfonate), and its X-ray powder diffraction pattern has characteristic diffraction peaks at the following 2θ positions: 13.87°±0.2°, 16.56°±0.2°, 17.78°±0.2°, 26.39°±0.2°, using Cu-Kα radiation.

Further, the present invention provides the crystalline form B of the benzenesulfonate of the compound of formula (I), and its X-ray powder diffraction pattern also has characteristic diffraction peaks at the following 2θ positions: 7.37°±0.2°, 22.47°±0.2°, 24.85°±0.2°, using Cu-Kα radiation.

The present invention provides the crystalline form B of the benzenesulfonate of the compound of formula (I), and the 2θ values and corresponding intensity in its X-ray powder diffraction pattern are shown in Table 29, and the error range of 2θ is ±0.2°.

**Table 29: 2θ values and corresponding intensity in XRPD pattern of the crystalline form B of the benzenesulfonate of the compound of formula (I)**

| Pos. [°2θ] | Height [cts] | FWHM Left [°2θ] | d-spacing [Å] | Rel. Int. [%] |
|---|---|---|---|---|
| 7.37 | 30.65 | 0.6140 | 12.00 | 35.67 |
| 13.87 | 56.39 | 0.2558 | 6.38 | 65.62 |
| 16.56 | 85.94 | 0.2047 | 5.35 | 100.00 |
| 17.78 | 61.53 | 0.2047 | 4.99 | 71.60 |
| 22.47 | 39.18 | 0.2047 | 3.96 | 45.59 |
| 24.85 | 57.55 | 0.2047 | 3.58 | 66.97 |
| 26.39 | 69.06 | 0.2558 | 3.38 | 80.36 |

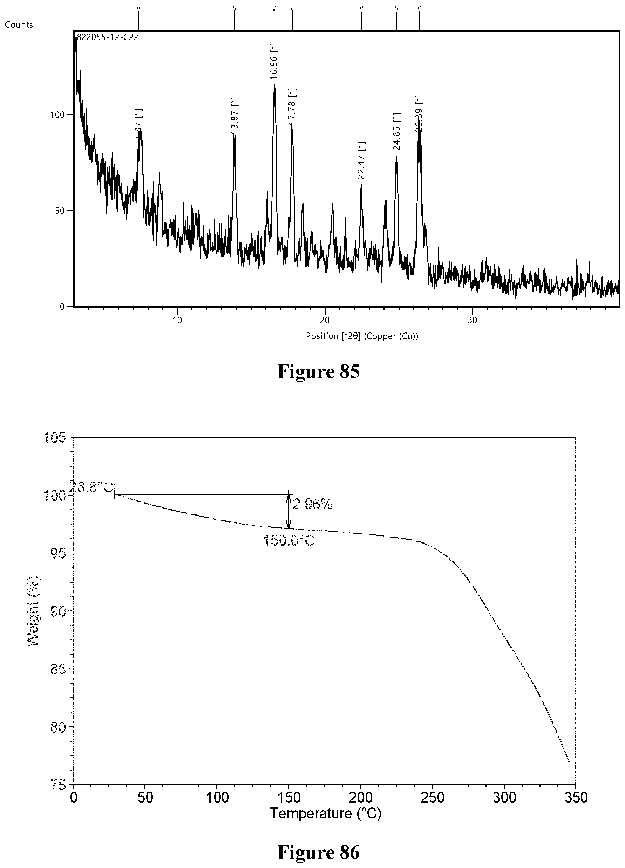

Further, the present invention provides the crystalline form B of the benzenesulfonate of the compound of formula (I), and its X-ray powder diffraction pattern is substantially shown in Figure 85.

The present invention provides the crystalline form B of the benzenesulfonate of the compound of formula (I), and its differential scanning calorimetry (DSC) shows an endothermic curve, in which Tₛₜₐᵣₜ = 134.3°C, Tₚₑₐₖ = 147.7°C, and ΔH = 43.82 J/g.

The present invention provides the crystalline form B of the benzenesulfonate of the compound of formula (I), and its differential scanning calorimetry (DSC) shows that its melting point is 147.7°C.

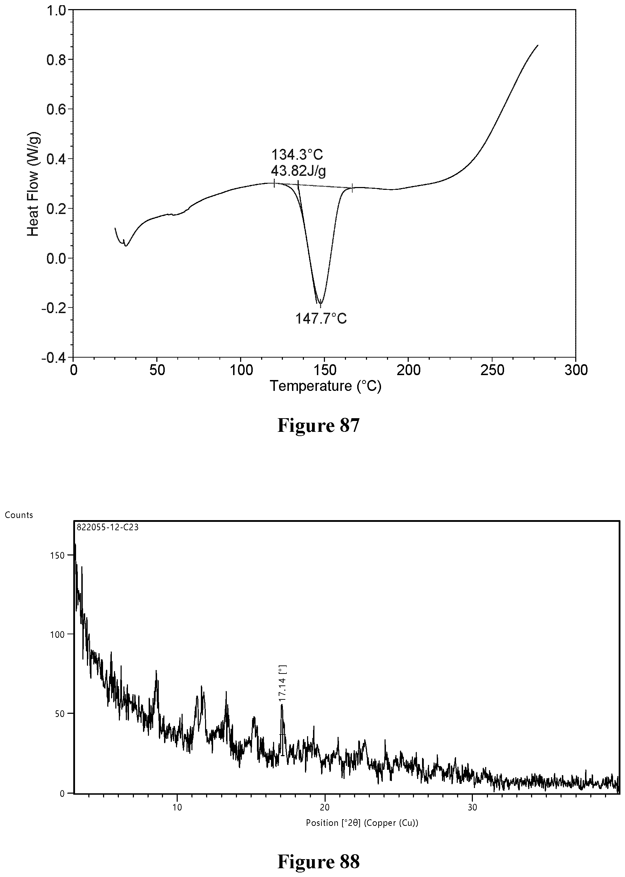

The present invention provides the crystalline form B of the benzenesulfonate of the compound of formula (I), and its differential scanning calorimetry (DSC) thermogram is shown in Figure 87.

The present invention provides the crystalline form B of the benzenesulfonate of the compound of formula (I), and its thermogravimetric analysis (TGA) thermogram shows 3.0% weight loss below 150°C.

The present invention provides the crystalline form B of the benzenesulfonate of the compound of formula (I), and its thermogravimetric analysis (TGA) thermogram is shown in Figure 86.

The present invention provides the crystalline form B of the benzenesulfonate of the compound of formula (I), which is a hydrate.

The present invention also provides an oxalate of the compound of formula (I), which is in crystalline form (crystalline form A of the oxalate), and its X-ray powder diffraction pattern has a characteristic diffraction peak at the following 2θ position: 17.14°±0.2°, using Cu-Kα radiation.

The present invention provides the crystalline form A of the oxalate of the compound of formula (I), and the 2θ values and corresponding intensity in its X-ray powder diffraction pattern are shown in Table 30, and the error range of 2θ is ±0.2°.

**Table 30: 2θ values and corresponding intensity in XRPD pattern of the crystalline form A of the oxalate of the compound of formula (I)**

| Pos. [°2θ] | Height [cts] | FWHM Left [°2θ] | d-spacing [Å] | Rel. Int. [%] |
|---|---|---|---|---|
| 17.14 | 26.28 | 0.3744 | 5.17 | 100.00 |

Further, the present invention provides the crystalline form A of the oxalate of the compound of formula (I), and its X-ray powder diffraction pattern is substantially shown in Figure 88.

The present invention provides the crystalline form A of the oxalate of the compound of formula (I), and its differential scanning calorimetry (DSC) shows 2 endothermic peaks at 199.6°C and 211.6°C (peak temperature).

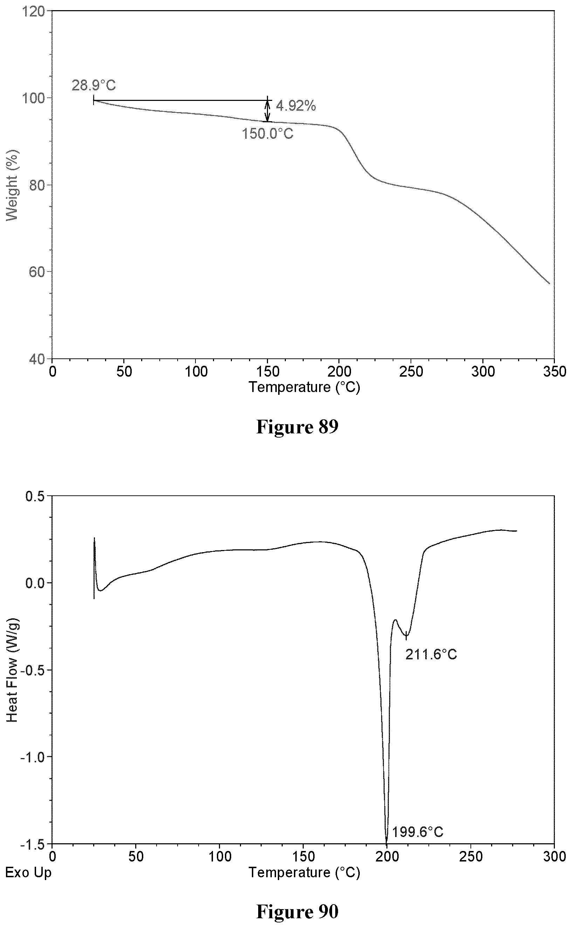

The present invention provides the crystalline form A of the oxalate of the compound of formula (I), and its differential scanning calorimetry (DSC) thermogram is shown in Figure 90.

The present invention provides the crystalline form A of the oxalate of the compound of formula (I), and its thermogravimetric analysis (TGA) thermogram shows 4.9% weight loss below 150°C.

The present invention provides the crystalline form A of the oxalate of the compound of formula (I), and its thermogravimetric analysis (TGA) thermogram is shown in Figure 89.

The present invention provides the crystalline form A of the oxalate of the compound of formula (I), which is a hydrate.

The present invention also provides a benzoate of the compound of formula (I), which is in crystalline form (crystalline form A of the benzoate), and its X-ray powder diffraction pattern has characteristic diffraction peaks at the following 2θ positions: 8.23°±0.2°, 13.48°±0.2°, 14.86°±0.2°, 15.13°±0.2°, using Cu-Kα radiation.

Further, the present invention provides the crystalline form A of the benzoate of the compound of formula (I), and its X-ray powder diffraction pattern also has characteristic diffraction peaks at the following 2θ positions: 6.62°±0.2°, 7.30°±0.2°, 12.11°±0.2°, 20.37°±0.2°, 22.37°±0.2°, using Cu-Kα radiation.

The present invention provides the crystalline form A of the benzoate of the compound of formula (I), and the 2θ values and corresponding intensity in its X-ray powder diffraction pattern are shown in Table 31, and the error range of 2θ is ±0.2°.

**Table 31: 2θ values and corresponding intensity in XRPD pattern of the crystalline form A of the benzoate of the compound of formula (I)**

| Pos. [°2θ] | Height [cts] | FWHM Left [°2θ] | d-spacing [Å] | Rel. Int. [%] |
|---|---|---|---|---|
| 6.62 | 64.17 | 0.1535 | 13.34 | 4.77 |
| 7.30 | 66.21 | 0.1535 | 12.11 | 4.92 |
| 8.23 | 520.82 | 0.1023 | 10.74 | 38.70 |
| 8.91 | 47.54 | 0.1535 | 9.93 | 3.53 |
| 12.11 | 87.07 | 0.1279 | 7.31 | 6.47 |
| 13.48 | 114.23 | 0.1023 | 6.57 | 8.49 |
| 14.86 | 244.97 | 0.0768 | 5.96 | 18.20 |
| 15.13 | 1345.62 | 0.1023 | 5.86 | 100.00 |
| 20.37 | 70.44 | 0.1791 | 4.36 | 5.23 |
| 22.37 | 44.21 | 0.1535 | 3.97 | 3.29 |
| 25.08 | 28.43 | 0.3070 | 3.55 | 2.11 |
| 26.72 | 10.39 | 0.5117 | 3.34 | 0.77 |

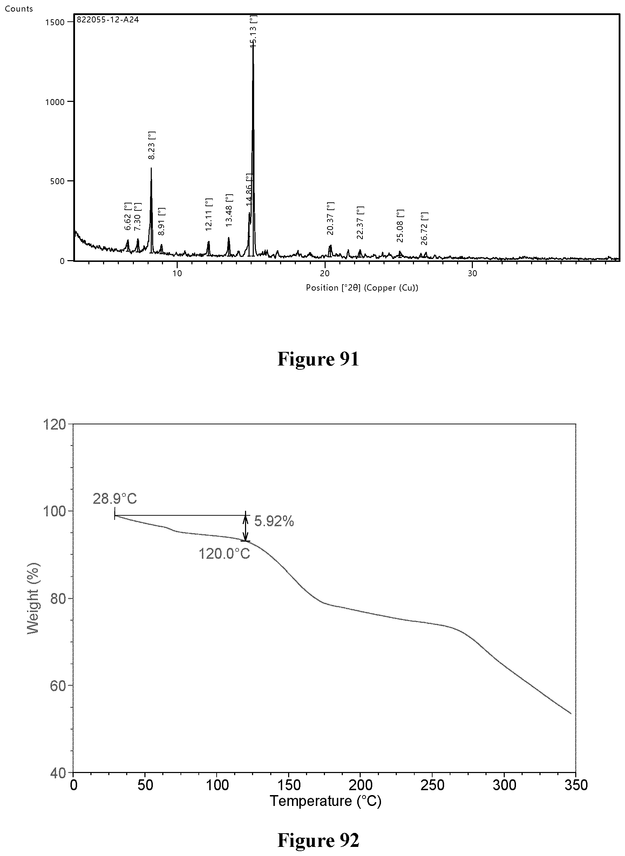

Further, the present invention provides the crystalline form A of the benzoate of the compound of formula (I), and its X-ray powder diffraction pattern is substantially shown in Figure 91.

The present invention provides the crystalline form A of the benzoate of the compound of formula (I), and its differential scanning calorimetry (DSC) shows 4 endothermic peaks at 67.5°C, 100.4°C, 118.6°C and 157.9°C (peak temperature).

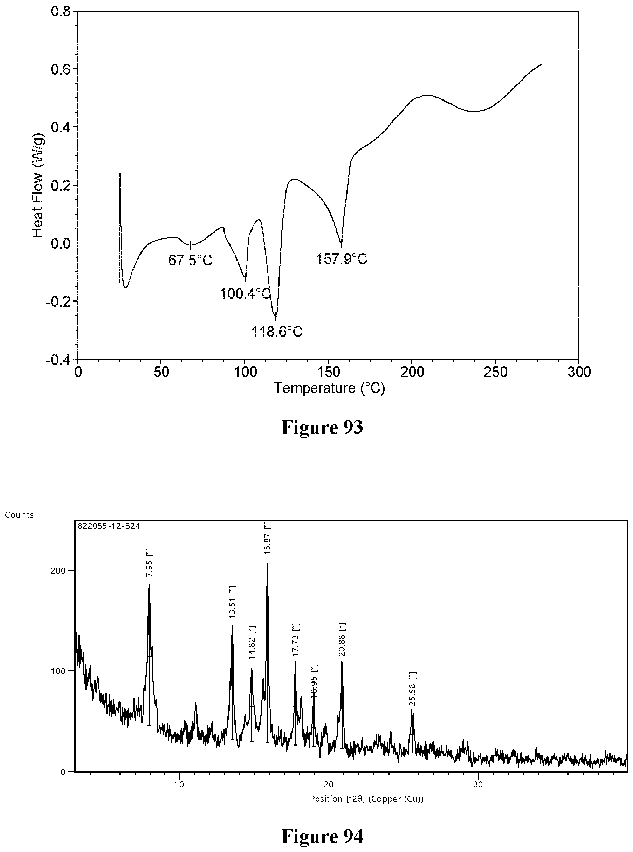

The present invention provides the crystalline form A of the benzoate of the compound of formula (I), and its differential scanning calorimetry (DSC) thermogram is shown in Figure 93.

The present invention provides the crystalline form A of the benzoate of the compound of formula (I), and its thermogravimetric analysis (TGA) thermogram shows 5.9% weight loss below 120°C.

The present invention provides the crystalline form A of the benzoate of the compound of formula (I), and its thermogravimetric analysis (TGA) thermogram is shown in Figure 92.

The present invention provides the crystalline form A of the benzoate of the compound of formula (I), which is a hydrate.

The present invention also provides a benzoate of the compound of formula (I), which is in crystalline form (crystalline form B of the benzoate), and its X-ray powder diffraction pattern has characteristic diffraction peaks at the following 2θ positions: 7.95°±0.2°, 13.51°±0.2°, 15.87°±0.2°, 20.88°±0.2°, using Cu-Kα radiation.

Further, the present invention provides the crystalline form B of the benzoate of the compound of formula (I), and its X-ray powder diffraction pattern also has characteristic diffraction peaks at the following 2θ positions: 14.82°±0.2°, 17.73°±0.2°, 18.95°±0.2°, 25.58°±0.2°, using Cu-Kα radiation.

The present invention provides the crystalline form B of the benzoate of the compound of formula (I), and the 2θ values and corresponding intensity in its X-ray powder diffraction pattern are shown in Table 32, and the error range of 2θ is ±0.2°.

**Table 32: 2θ values and corresponding intensity in XRPD pattern of the crystalline form B of the benzoate of the compound of formula (I)**

| Pos. [°2θ] | Height [cts] | FWHM Left [°2θ] | d-spacing [Å] | Rel. Int. [%] |
|---|---|---|---|---|
| 7.95 | 136.66 | 0.2047 | 11.12 | 76.54 |
| 13.51 | 109.11 | 0.2047 | 6.56 | 61.11 |
| 14.82 | 70.35 | 0.2047 | 5.98 | 39.40 |
| 15.87 | 178.54 | 0.1279 | 5.59 | 100.00 |
| 17.73 | 76.68 | 0.2047 | 5.00 | 42.95 |
| 18.95 | 37.14 | 0.3070 | 4.68 | 20.80 |
| 20.88 | 80.59 | 0.1535 | 4.26 | 45.14 |
| 25.58 | 36.47 | 0.2558 | 3.48 | 20.43 |

Further, the present invention provides the crystalline form B of the benzoate of the compound of formula (I), and its X-ray powder diffraction pattern is substantially shown in Figure 94.

The present invention provides the crystalline form B of the benzoate of the compound of formula (I), and its differential scanning calorimetry (DSC) shows 4 endothermic peaks at 111.6°C, 124.1°C, 129.1°C and 156.6°C (peak temperature).

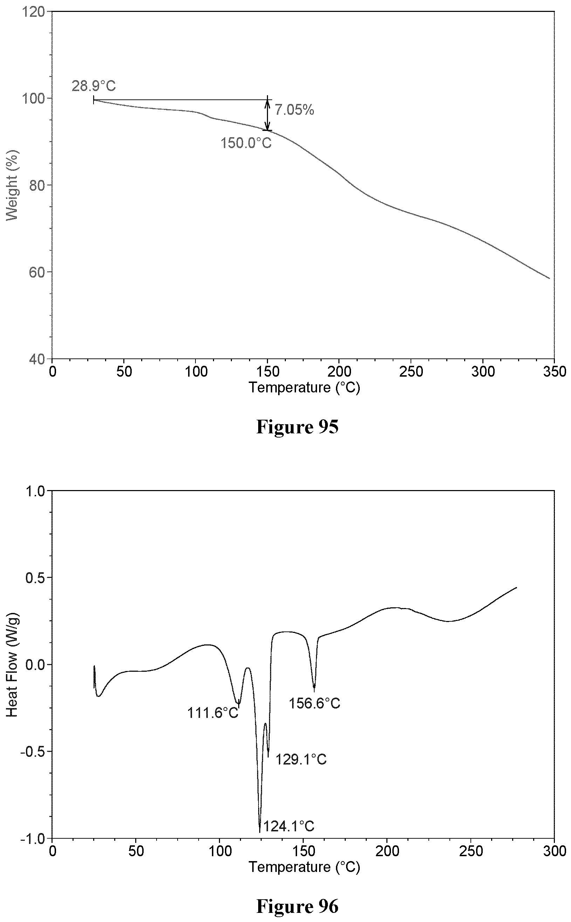

The present invention provides the crystalline form B of the benzoate of the compound of formula (I), and its differential scanning calorimetry (DSC) thermogram is shown in Figure 96.

The present invention provides the crystalline form B of the benzoate of the compound of formula (I), and its thermogravimetric analysis (TGA) thermogram shows 7.1% weight loss below 150°C.

The present invention provides the crystalline form B of the benzoate of the compound of formula (I), and its thermogravimetric analysis (TGA) thermogram is shown in Figure 95.

The present invention provides the crystalline form B of the benzoate of the compound of formula (I), which is a hydrate.

The present invention also provides a benzoate of the compound of formula (I), which is in crystalline form (crystalline form C of the benzoate), and its X-ray powder diffraction pattern has characteristic diffraction peaks at the following 2θ positions: 5.01°±0.2°, 7.24°±0.2°, 12.07°±0.2°, 14.51°±0.2°, 17.12°±0.2°, 18.62°±0.2°, using Cu-Kα radiation.

Further, the present invention provides the crystalline form C of the benzoate of the compound of formula (I), and its X-ray powder diffraction pattern also has characteristic diffraction peaks at the following 2θ positions: 15.84°±0.2°, 23.46°±0.2°, 24.42°±0.2°, 25.43°±0.2°, using Cu-Kα radiation.

The present invention provides the crystalline form C of the benzoate of the compound of formula (I), and the 2θ values and corresponding intensity in its X-ray powder diffraction pattern are shown in Table 33, and the error range of 2θ is ±0.2°.

**Table 33: 2θ values and corresponding intensity in XRPD pattern of the crystalline form C of the benzoate of the compound of formula (I)**

| Pos. [°2θ] | Height [cts] | FWHM Left [°2θ] | d-spacing [Å] | Rel. Int. [%] |
|---|---|---|---|---|
| 5.01 | 90.50 | 0.1535 | 17.63 | 54.90 |
| 7.24 | 124.59 | 0.2047 | 12.22 | 75.58 |
| 12.07 | 128.12 | 0.2047 | 7.34 | 77.72 |
| 14.51 | 111.90 | 0.2558 | 6.10 | 67.88 |
| 15.84 | 18.97 | 0.6140 | 5.60 | 11.51 |
| 17.12 | 164.84 | 0.2558 | 5.18 | 100.00 |
| 18.62 | 118.71 | 0.2047 | 4.77 | 72.01 |
| 23.46 | 38.25 | 0.2558 | 3.79 | 23.21 |
| 24.42 | 66.88 | 0.2047 | 3.65 | 40.57 |
| 25.43 | 25.06 | 0.4093 | 3.50 | 15.20 |

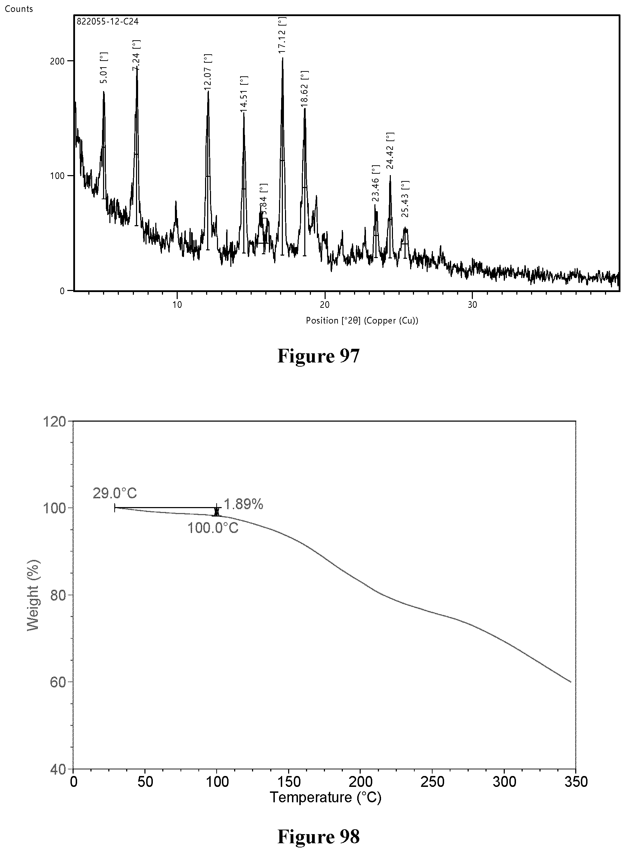

Further, the present invention provides the crystalline form C of the benzoate of the compound of formula (I), and its X-ray powder diffraction pattern is substantially shown in Figure 97.

The present invention provides the crystalline form C of the benzoate of the compound of formula (I), and its differential scanning calorimetry (DSC) shows 4 endothermic peaks at 106.6°C, 120.7°C, 126.4°C and 143.9°C (peak temperature).

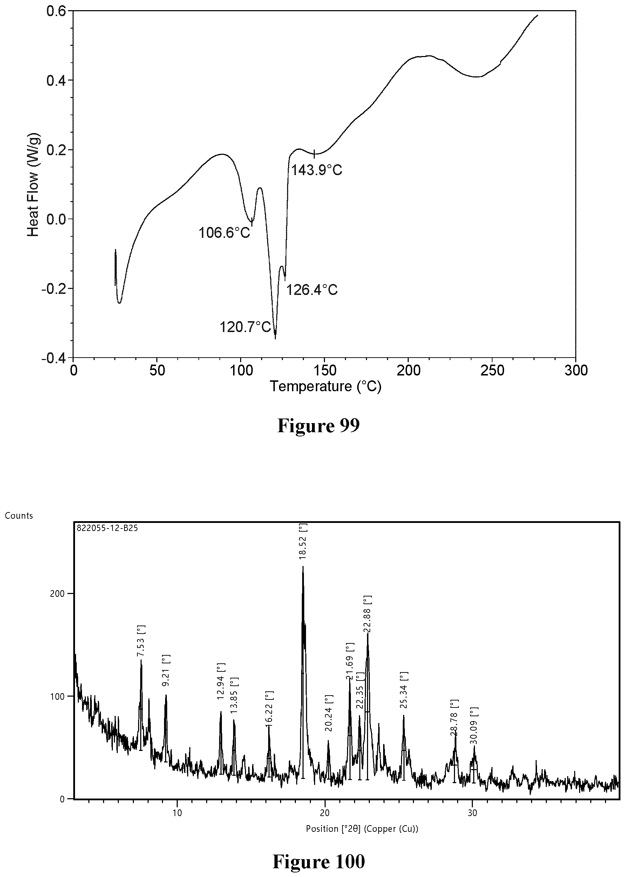

The present invention provides the crystalline form C of the benzoate of the compound of formula (I), and its differential scanning calorimetry (DSC) thermogram is shown in Figure 99.

The present invention provides the crystalline form C of the benzoate of the compound of formula (I), and its thermogravimetric analysis (TGA) thermogram shows 1.9% weight loss below 100°C.

The present invention provides the crystalline form C of the benzoate of the compound of formula (I), and its thermogravimetric analysis (TGA) thermogram is shown in Figure 98.

The present invention provides the crystalline form C of the benzoate of the compound of formula (I), which is an anhydrate.

The present invention also provides a hydrobromide of the compound of formula (I), which is in crystalline form (crystalline form A of the hydrobromide), and its X-ray powder diffraction pattern has characteristic diffraction peaks at the following 2θ positions: 7.53°±0.2°, 18.52°±0.2°, 21.69°±0.2°, 22.88°±0.2°, using Cu-Kα radiation.

Further, the present invention provides the crystalline form A of the hydrobromide of the compound of formula (I), and its X-ray powder diffraction pattern also has characteristic diffraction peaks at the following 2θ positions: 9.21°±0.2°, 12.94°±0.2°, 13.85°±0.2°, 22.35°±0.2°, 25.34°±0.2°, 30.09°±0.2°, using Cu-Kα radiation.

The present invention provides the crystalline form A of the hydrobromide of the compound of formula (I), and the 2θ values and corresponding intensity in its X-ray powder diffraction pattern are shown in Table 34, and the error range of 2θ is ±0.2°.

**Table 34: 2θ values and corresponding intensity in XRPD pattern of the crystalline form A of the hydrobromide of the compound of formula (I)**

| Pos. [°2θ] | Height [cts] | FWHM Left [°2θ] | d-spacing [Å] | Rel. Int. [%] |
|---|---|---|---|---|
| 7.53 | 80.04 | 0.2047 | 11.74 | 38.94 |
| 9.21 | 62.49 | 0.1535 | 9.60 | 30.41 |
| 12.94 | 60.82 | 0.1535 | 6.84 | 29.59 |
| 13.85 | 47.75 | 0.1535 | 6.40 | 23.23 |
| 16.22 | 36.59 | 0.2047 | 5.47 | 17.80 |
| 18.52 | 205.53 | 0.1023 | 4.79 | 100.00 |
| 20.24 | 34.96 | 0.1535 | 4.39 | 17.01 |
| 21.69 | 85.77 | 0.2047 | 4.10 | 41.73 |
| 22.35 | 57.28 | 0.1535 | 3.98 | 27.87 |
| 22.88 | 131.96 | 0.2558 | 3.89 | 64.20 |
| 25.34 | 57.98 | 0.1535 | 3.51 | 28.21 |
| 28.78 | 33.24 | 0.3070 | 3.10 | 16.17 |
| 30.09 | 26.07 | 0.2047 | 11.74 | 38.94 |

Further, the present invention provides the crystalline form A of the hydrobromide of the compound of formula (I), and its X-ray powder diffraction pattern is substantially shown in Figure 100.

The present invention provides the crystalline form A of the hydrobromide of the compound of formula (I), and its differential scanning calorimetry (DSC) shows an endothermic curve, in which Tₛₜₐᵣₜ = 220.2°C, Tₚₑₐₖ = 225.7°C, and ΔH = 11.29 J/g.

The present invention provides the crystalline form A of the hydrobromide of the compound of formula (I), and its differential scanning calorimetry (DSC) shows that its melting point is 225.7C.

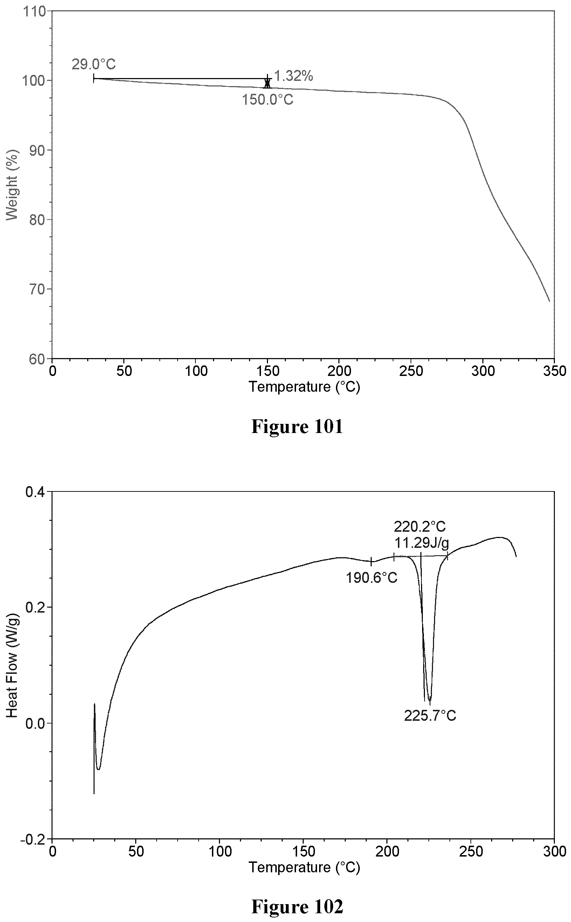

The present invention provides the crystalline form A of the hydrobromide of the compound of formula (I), and its differential scanning calorimetry (DSC) thermogram is shown in Figure 102.

The present invention provides the crystalline form A of the hydrobromide of the compound of formula (I), and its thermogravimetric analysis (TGA) thermogram shows 1.3% weight loss below 150°C, and the decomposition temperature is 250°C.

The present invention provides the crystalline form A of the hydrobromide of the compound of formula (I), and its thermogravimetric analysis (TGA) thermogram is shown in Figure 101.

The present invention provides the crystalline form A of the hydrobromide of the compound of formula (I), which is an anhydrate.

The salt or crystalline form of the invention is present at about 5% to about 100% by weight of the drug substance. In some embodiments, the salt or crystalline form of the invention is present at about 10% to about 100% by weight of the drug substance. In some embodiments, the salt or crystalline form of the invention is present at about 15% to about 100% by weight of the drug substance. In some embodiments, the salt or crystalline form of the invention is present at about 20% to about 100% by weight of the drug substance. In some embodiments, the salt or crystalline form of the invention is present at about 25% to about 100% by weight of the drug substance. In some embodiments, the salt or crystalline form of the invention is present at about 30% to about 100% by weight of the drug substance. In some embodiments, the salt or crystalline form of the invention is present at about 35% to about 100% by weight of the drug substance. In some embodiments, the salt or crystalline form of the invention is present at about 40% to about 100% by weight of the drug substance. In some embodiments, the salt or crystalline form of the invention is present at about 45% to about 100% by weight of the drug substance. In some embodiments, the salt or crystalline form of the invention is present at about 50% to about 100% by weight of the drug substance. In some embodiments, the salt or crystalline form of the invention is present at about 55% to about 100% by weight of the drug substance. In some embodiments, the salt or crystalline form of the invention is present at about 60% to about 100% by weight of the drug substance. In some embodiments, the salt or crystalline form of the invention is present at about 65% to about 100% by weight of the drug substance. In some embodiments, the salt or crystalline form of the invention is present at about 70% to about 100% by weight of the drug substance. In some embodiments, the salt or crystalline form of the invention is present at about 75% to about 100% by weight of the drug substance. In some embodiments, the salt or crystalline form of the invention is present at about 80% to about 100% by weight of the drug substance. In some embodiments, the salt or crystalline form of the invention is present at about 85% to about 100% by weight of the drug substance. In some embodiments, the salt or crystalline form of the invention is present at about 90% to about 100% by weight of the drug substance. In some embodiments, the salt or crystalline form of the invention is present at about 95% to about 100% by weight of the drug substance. In some embodiments, the salt or crystalline form of the invention is present at about 98% by weight to about 100% by weight of the drug substance. In some embodiments, the salt or crystalline form of the invention is present at about 99% to about 100% by weight of the drug substance. In certain embodiments, substantially all of the drug substance is the salt or crystalline form of the invention, i.e. the drug substance is substantially a phase pure salt or a phase pure crystal.

The structure of the crystalline form of the present invention can be analyzed using various analytical techniques known to those skilled in the art, including, but not limited to, X-ray powder diffraction (XRD), differential scanning calorimetry (DSC) and/or thermogravimetry (TG).

Thermogravimetric Analysis (TGA), also known as thermogravimetry (TG).

It can be understood that, as is well known in the field of differential scanning calorimetry (DSC), the height of the melting peak in DSC curve depends on many factors related to sample preparation and instrument geometry, while the peak position is relatively insensitive to experimental details. Thus, in some embodiments, the crystalline compounds of the invention are characterized by a DSC thermogram having characteristic peak positions with substantially the same properties as the DSC thermogram provided in the Figures of the invention with an error tolerance of ±3°C.

The substance which has substantially the same X-ray powder diffraction pattern, DSC thermogram or TGA thermogram as those disclosed in the invention, also belongs to the scope of the invention.

The present invention also relates to a pharmaceutical composition comprising a therapeutically effective amount of a salt of the compound of formula (I) and a hydrate, a solvate thereof and their crystalline forms according to the invention, and a pharmaceutically acceptable carrier or excipient.

The present invention also relates to a use of a salt of the compound of formula (I) and a hydrate, a solvate thereof and their crystalline forms according to the invention, as well as the pharmaceutical compositions in the manufacture of a medicament for treating the diseases mediated by DPP1.

Further, for the use according to the invention, the disease mediated by DPP1 is selected from the group consisting of non-cystic fibrosis bronchiectasis, cystic fibrosis bronchiectasis, acute lung injury, airway obstructive disease, bronchiectasis, cystic fibrosis, asthma, emphysema and chronic obstructive pulmonary disease.

Compared with the prior art, the invention has the following beneficial effects:
The advantages of the salt or the crystalline of the salt according to the invention include but are not limited to higher solubility, better pharmacokinetic properties and good stability, and are suitable for the preparation of pharmaceutical preparations, and the preparation method of the crystalline form is simple and effective, and easy to scale up for production.

The salt or the crystalline of the salt according to the invention has excellent physical properties, including but not limited to solubility, dissolution rate, light resistance, low hygroscopicity, high temperature resistance, high humidity resistance, fluidity and significantly improved viscosity. For example, the crystalline form according to the invention can significantly reduce the filtration time, shorten the production cycle, and save costs during the preparation process. The crystalline form according to the invention also has good light stability, thermal stability and moisture stability, which can ensure the reliability of the crystalline form during storage and transportation, thereby ensuring the safety of the preparation, and the crystalline form does not require special packaging to protect against the effects of light, temperature and humidity, and thus reduces costs. The crystalline form will not be degraded due to the influence of light, high temperature and high humidity, which improves the safety of the preparation and its effectiveness after long-term storage. Patients taking the crystalline form will not be concerned about photosensitivity reactions to the preparation due to exposure to sunlight.

The salt or the crystalline of the salt according to the invention has little or less degradation when stored or transported at ambient temperature, has good thermal stability, can be stably maintained for a long time, and is suitable for standard preparation production processes.

The salt or the crystalline of the salt according to the invention has good chemical stability and physical stability, is easy to prepare, and is more suitable for the preparation of preparations. In particular, the crystalline form according to the invention has better milling stability.

The salt or the crystalline of the salt according to the invention has good fluidity, good compressibility, high bulk density, low hygroscopicity and uniform particle size distribution.

The salt or the crystalline of the salt according to the invention is suitable and convenient for large-scale preparation. The preparation prepared by using the above crystalline form can reduce irritation and improve absorption, so that the problem of metabolic speed can be solved, the toxicity can be significantly reduced, and the safety can be improved, and thus effectively ensure the quality and efficacy of preparations.

Unless defined otherwise, all technical and scientific terms used herein have the same meaning as commonly understood by one of ordinary skill in the art to which this invention belongs. In the event of a conflict, the definitions provided in this application shall control. When an amount, concentration, or other value or parameter is expressed in the form of a range, a preferred range, or preferred numerical upper limit and preferred numerical lower limit, it should be understood that it specifically discloses any range by combining any range upper limit or preferred numerical value with any range lower limit or preferred numerical value, regardless of whether the range is specifically disclosed. Unless otherwise indicated, numerical ranges set forth herein are intended to include the endpoints of the range and all integers and fractions (decimals) within the range.

The terms "about" and "approximately" when used with numerical variables generally mean that the value of the variable and all values of the variable are within experimental error (for example, within a 95% confidence interval for the mean) or within ±10% of the specified value, or within a wider range.

"Effective dosage" means that amount of a compound that causes physiological or medical reaction in a tissue, system, or subject for which such amount is sought, including the amount of a compound that, when administered to a subject, is sufficient to prevent the occurance of one or more symptoms of the disease or condition being treated, or to reduce them to a certain extent.

"IC₅₀" refers to the half-inhibitory concentration, which is the concentration at which half of the maximum inhibitory effect is achieved.

Unless otherwise stated, percentages, parts, etc. herein are by weight.

The term "amorphous" refers to any solid substance that is not ordered in three dimensions. In some cases, amorphous solids can be characterized by known techniques including XRPD crystal diffraction analysis, differential scanning calorimetry (DSC), solid state nuclear magnetic resonance (ssNMR), or a combination thereof. As explained below, the XRPD pattern produced by amorphous solids has no obvious diffraction characteristic peaks.

As used herein, the term "crystalline form" or "crystal" refers to any solid substance exhibiting a three-dimensional order, which, in contrast to amorphous solid substance, produces a characteristic XRPD pattern with well-defined peaks.

As used herein, the term "crystal seed" refers to the formation of crystal nuclei through the addition of insoluble additives in a crystallization process that accelerates or promotes the growth of enantiomeric crystals of the same crystalline form or stereoconfiguration.

As used herein, the term "X-ray powder diffraction pattern (XRPD pattern)" refers to an experimentally observed diffraction pattern or parameters, data or values derived therefrom. XRPD pattern is usually characterized by peak position (abscissa) and/or peak intensity (ordinate).

As used herein, the term "2θ" refers to the peak position expressed in degrees (°) based on settings in X-ray diffraction experiments, and is typically the abscissa unit in a diffraction pattern. If the reflection is diffracted when the incident beam forms an angle θ with a lattice plane, the experimental setup requires recording the reflected beam in an angle 2θ. It should be understood that reference herein to a specific 2θ value for a particular crystalline form is intended to mean the 2θ value (expressed in degrees) measured using the X-ray diffraction experimental conditions described herein.

As used herein, the term "substantially the same" for an X-ray diffraction peak means taking into account representative peak positions and intensity variations. For example, those skilled in the art will understand that the peak position (2θ) will show some variation, typically as much as 0.1-0.2 degrees, and that the instrument used to measure diffraction will also cause some variation. Additionally, those skilled in the art will understand that relative peak intensity will vary due to inter-instrument variation as well as the degree of crystallinity, preferred orientation, prepared sample surface, and other factors known to those skilled in the art, and should be considered as merely for qualitative measurement.

"Pharmaceutical composition" means a mixture of one or more compounds described herein, or physiologically/pharmaceutically acceptable salts thereof, with other ingredients, wherein the other ingredients include physiologically/pharmaceutically acceptable carriers and excipients.

"Carrier" refers to a carrier or diluent that does not cause significant irritation to the organism and does not eliminate the biological activity and properties of the compound to be administered.

"Excipient" refers to an inert substance added to a pharmaceutical composition to further facilitate administration of the compound. Examples of excipients include, but are not limited to, calcium carbonate, calcium phosphate, various sugars and different types of starch, cellulose derivatives (including microcrystalline cellulose), gelatin, vegetable oils, polyethylene glycols, diluents, granulating agents, lubricants, binders, disintegrants, etc.

It is understood that the numerical values described and claimed herein are approximations. Variations within values may be attributed to equipment calibration, equipment errors, crystal purity, crystal size, sample size, and other factors.

### Description of figures

Figure 1 is an X-ray powder diffraction pattern of the crystalline form A of the hydrochloride of the compound represented by formula (I).
Figure 2 is a thermogravimetric analysis thermogram of the crystalline form A of the hydrochloride of the compound represented by formula (I).
Figure 3 is a differential scanning calorimetry analysis curve of the crystalline form A of the hydrochloride of the compound represented by formula (I).
Figure 4 is an X-ray powder diffraction pattern of the crystalline form A of malate of the compound represented by formula (I).
Figure 5 is a thermogravimetric analysis thermogram of the crystalline form A of malate of the compound represented by formula (I).
Figure 6 is a differential scanning calorimetry analysis curve of the crystalline form A of malate of the compound represented by formula (I).
Figure 7 is an X-ray powder diffraction pattern of the crystalline form B of the adipate of the compound represented by formula (I).
Figure 8 is a thermogravimetric analysis thermogram of the crystalline form B of the adipate of the compound represented by formula (I).
Figure 9 is a differential scanning calorimetry analysis curve of the crystalline form B of the adipate of the compound represented by formula (I).
Figure 10 is an X-ray powder diffraction pattern of the crystalline form A of the sulfate of the compound represented by formula (I).
Figure 11 is a thermogravimetric analysis thermogram of the crystalline form A of the sulfate of the compound represented by formula (I).
Figure 12 a differential scanning calorimetry analysis curve of the crystalline form A of the sulfate of the compound represented by formula (I).
Figure 13 is an X-ray powder diffraction pattern of the crystalline form B of the sulfate of the compound represented by formula (I).
Figure 14 is a thermogravimetric analysis thermogram of the crystalline form B of the sulfate of the compound represented by formula (I).
Figure 15 a differential scanning calorimetry analysis curve of the crystalline form B of the sulfate of the compound represented by formula (I).
Figure 16 is an X-ray powder diffraction pattern of the crystalline form A of the maleate of the compound represented by formula (I).
Figure 17 is a thermogravimetric analysis thermogram of the crystalline form A of the maleate of the compound represented by formula (I).
Figure 18 a differential scanning calorimetry analysis curve of the crystalline form A of the maleate of the compound represented by formula (I).
Figure 19 is an X-ray powder diffraction pattern of the crystalline form B of the maleate of the compound represented by formula (I).
Figure 20 is a thermogravimetric analysis thermogram of the crystalline form B of the maleate of the compound represented by formula (I).
Figure 21 is differential scanning calorimetry analysis curve of the crystalline form B of the maleate of the compound represented by formula (I).
Figure 22 is an X-ray powder diffraction pattern of the crystalline form C of the maleate of the compound represented by formula (I).
Figure 23 is a thermogravimetric analysis thermogram of the crystalline form C of the maleate of the compound represented by formula (I).
Figure 24 is differential scanning calorimetry analysis curve of the crystalline form C of the maleate of the compound represented by formula (I).
Figure 25 is an X-ray powder diffraction pattern of the crystalline form A of the phosphate of the compound represented by formula (I).
Figure 26 is a thermogravimetric analysis thermogram of the crystalline form A of the phosphate of the compound represented by formula (I).
Figure 27 is differential scanning calorimetry analysis curve of the crystalline form A of the phosphate of the compound represented by formula (I).
Figure 28 is an X-ray powder diffraction pattern of the crystalline form B of the phosphate of the compound represented by formula (I).
Figure 29 is a thermogravimetric analysis thermogram of the crystalline form B of the phosphate of the compound represented by formula (I).
Figure 30 is differential scanning calorimetry analysis curve of the crystalline form B of the phosphate of the compound represented by formula (I).
Figure 31 is an X-ray powder diffraction pattern of the crystalline form C of the phosphate of the compound represented by formula (I).
Figure 32 is a thermogravimetric analysis thermogram of the crystalline form C of the phosphate of the compound represented by formula (I).
Figure 33 is differential scanning calorimetry analysis curve of the crystalline form C of the phosphate of the compound represented by formula (I).
Figure 34 is an X-ray powder diffraction pattern of the crystalline form A of the mucate of the compound represented by formula (I).
Figure 35 is a thermogravimetric analysis thermogram of the crystalline form A of the mucate of the compound represented by formula (I).
Figure 36 is differential scanning calorimetry analysis curve of the crystalline form A of the mucate of the compound represented by formula (I).
Figure 37 is an X-ray powder diffraction pattern of the crystalline form A of the tartrate of the compound represented by formula (I).
Figure 38 is a thermogravimetric analysis thermogram of the crystalline form A of the tartrate of the compound represented by formula (I).
Figure 39 is differential scanning calorimetry analysis curve of the crystalline form A of the tartrate of the compound represented by formula (I).
Figure 40 is an X-ray powder diffraction pattern of the crystalline form B of the tartrate of the compound represented by formula (I).
Figure 41 is a thermogravimetric analysis thermogram of the crystalline form B of the tartrate of the compound represented by formula (I).
Figure 42 is differential scanning calorimetry analysis curve of the crystalline form B of the tartrate of the compound represented by formula (I).
Figure 43 is an X-ray powder diffraction pattern of the crystalline form C of the tartrate of the compound represented by formula (I).
Figure 44 is a thermogravimetric analysis thermogram of the crystalline form C of the tartrate of the compound represented by formula (I).
Figure 45 is differential scanning calorimetry analysis curve of the crystalline form C of the tartrate of the compound represented by formula (I).
Figure 46 is an X-ray powder diffraction pattern of the crystalline form A of the fumarate of the compound represented by formula (I).
Figure 47 is a thermogravimetric analysis thermogram of the crystalline form A of the fumarate of the compound represented by formula (I).
Figure 48 is differential scanning calorimetry analysis curve of the crystalline form A of the fumarate of the compound represented by formula (I).
Figure 49 is an X-ray powder diffraction pattern of the crystalline form B of the fumarate of the compound represented by formula (I).
Figure 50 is a thermogravimetric analysis thermogram of the crystalline form B of the fumarate of the compound represented by formula (I).
Figure 51 is differential scanning calorimetry analysis curve of the crystalline form B of the fumarate of the compound represented by formula (I).
Figure 52 is an X-ray powder diffraction pattern of the crystalline form A of the citrate of the compound represented by formula (I).
Figure 53 is a thermogravimetric analysis thermogram of the crystalline form A of the citrate of the compound represented by formula (I).
Figure 54 is differential scanning calorimetry analysis curve of the crystalline form A of the citrate of the compound represented by formula (I).
Figure 55 is an X-ray powder diffraction pattern of the crystalline form B of the citrate of the compound represented by formula (I).
Figure 56 is a thermogravimetric analysis thermogram of the crystalline form B of the citrate of the compound represented by formula (I).
Figure 57 is differential scanning calorimetry analysis curve of the crystalline form B of the citrate of the compound represented by formula (I).
Figure 58 is an X-ray powder diffraction pattern of the crystalline form B of the malate of the compound represented by formula (I).
Figure 59 is a thermogravimetric analysis thermogram of the crystalline form B of the malate of the compound represented by formula (I).
Figure 60 is differential scanning calorimetry analysis curve of the crystalline form B of the malate of the compound represented by formula (I).
Figure 61 is an X-ray powder diffraction pattern of the crystalline form A of the hippurate of the compound represented by formula (I).
Figure 62 is a thermogravimetric analysis thermogram of the crystalline form A of the hippurate of the compound represented by formula (I).
Figure 63 is differential scanning calorimetry analysis curve of the crystalline form A of the hippurate of the compound represented by formula (I).
Figure 64 is an X-ray powder diffraction pattern of the crystalline form A of the adipate of the compound represented by formula (I).
Figure 65 is a thermogravimetric analysis thermogram of the crystalline form A of the adipate of the compound represented by formula (I).
Figure 66 is differential scanning calorimetry analysis curve of the crystalline form A of the adipate of the compound represented by formula (I).
Figure 67 is an X-ray powder diffraction pattern of the crystalline form A of the sebacate of the compound represented by formula (I).
Figure 68 is a thermogravimetric analysis thermogram of the crystalline form A of the sebacate of the compound represented by formula (I).
Figure 69 is differential scanning calorimetry analysis curve of the crystalline form A of the sebacate of the compound represented by formula (I).
Figure 70 is an X-ray powder diffraction pattern of the crystalline form B of the sebacate of the compound represented by formula (I).
Figure 71 is a thermogravimetric analysis thermogram of the crystalline form B of the sebacate of the compound represented by formula (I).
Figure 72 is differential scanning calorimetry analysis curve of the crystalline form B of the sebacate of the compound represented by formula (I).
Figure 73 is an X-ray powder diffraction pattern of the crystalline form C of the sebacate of the compound represented by formula (I).
Figure 74 is a thermogravimetric analysis thermogram of the crystalline form C of the sebacate of the compound represented by formula (I).
Figure 75 is differential scanning calorimetry analysis curve of the crystalline form C of the sebacate of the compound represented by formula (I).
Figure 76 is an X-ray powder diffraction pattern of the crystalline form A of the 1,5-naphthalenedisulfonate of the compound represented by formula (I).
Figure 77 is a thermogravimetric analysis thermogram of the crystalline form A of the 1,5-naphthalenedisulfonate of the compound represented by formula (I).
Figure 78 is differential scanning calorimetry analysis curve of the crystalline form A of the 1,5-naphthalenedisulfonate of the compound represented by formula (I).
Figure 79 is an X-ray powder diffraction pattern of the crystalline form A of the methanesulfonate of the compound represented by formula (I).
Figure 80 is a thermogravimetric analysis thermogram of the crystalline form A of the methanesulfonate of the compound represented by formula (I).
Figure 81 is differential scanning calorimetry analysis curve of the crystalline form A of the methanesulfonate of the compound represented by formula (I).
Figure 82 is an X-ray powder diffraction pattern of the crystalline form A of the benzenesulfonate of the compound represented by formula (I).
Figure 83 is a thermogravimetric analysis thermogram of the crystalline form A of the benzenesulfonate of the compound represented by formula (I).
Figure 84 is differential scanning calorimetry analysis curve of the crystalline form A of the benzenesulfonate of the compound represented by formula (I).
Figure 85 is an X-ray powder diffraction pattern of the crystalline form B of the benzenesulfonate of the compound represented by formula (I).
Figure 86 is a thermogravimetric analysis thermogram of the crystalline form B of the benzenesulfonate of the compound represented by formula (I).
Figure 87 is differential scanning calorimetry analysis curve of the crystalline form B of the benzenesulfonate of the compound represented by formula (I).
Figure 88 is an X-ray powder diffraction pattern of the crystalline form A of the oxalate of the compound represented by formula (I).
Figure 89 is a thermogravimetric analysis thermogram of the crystalline form A of the oxalate of the compound represented by formula (I).
Figure 90 is differential scanning calorimetry analysis curve of the crystalline form A of the oxalate of the compound represented by formula (I).
Figure 91 is an X-ray powder diffraction pattern of the crystalline form A of the benzoate of the compound represented by formula (I).
Figure 92 is a thermogravimetric analysis thermogram of the crystalline form A of the benzoate of the compound represented by formula (I).
Figure 93 is differential scanning calorimetry analysis curve of the crystalline form A of the benzoate of the compound represented by formula (I).
Figure 94 is an X-ray powder diffraction pattern of the crystalline form B of the benzoate of the compound represented by formula (I).
Figure 95 is a thermogravimetric analysis thermogram of the crystalline form B of the benzoate of the compound represented by formula (I).
Figure 96 is differential scanning calorimetry analysis curve of the crystalline form B of the benzoate of the compound represented by formula (I).
Figure 97 is an X-ray powder diffraction pattern of the crystalline form C of the benzoate of the compound represented by formula (I).
Figure 98 is a thermogravimetric analysis thermogram of the crystalline form C of the benzoate of the compound represented by formula (I).
Figure 99 is differential scanning calorimetry analysis curve of the crystalline form C of the benzoate of the compound represented by formula (I).
Figure 100 is an X-ray powder diffraction pattern of the crystalline form A of the hydrobromide of the compound represented by formula (I).
Figure 101 is a thermogravimetric analysis thermogram of the crystalline form A of the hydrobromide of the compound represented by formula (I).
Figure 102 is differential scanning calorimetry analysis curve of the crystalline form A of the hydrobromide of the compound represented by formula (I).
Figure 103 is a dynamic vapour sorption spectrum of the crystalline form A of the hydrochloride of the compound represented by formula (I).
Figure 104 is a dynamic vapour sorption spectrum of the crystalline form A of the malate of the compound represented by formula (I).
Figure 105 is a dynamic vapour sorption spectrum of the crystalline form B of the adipate of the compound represented by formula (I).
Figure 106 is a PLM characterization spectrum of the crystalline form A of the hydrochloride of the compound represented by formula (I).
Figure 107 is a PLM characterization spectrum of the crystalline form A of the malate of the compound represented by formula (I).
Figure 108 is a PLM characterization spectrum of the crystalline form B of the adipate of the compound represented by formula (I).

### Specific mode for carrying out the invention

The implementation process and beneficial effects of the present invention are described in detail below through specific examples, which are intended to help readers better understand the essence and characteristics of the invention, and are not intended to limit the scope of the invention.

Compound structures were determined by nuclear magnetic resonance (NMR) and/or mass spectroscopy (MS).

NMR shifts (δ) are given in units of 10⁻⁶ (ppm). NMR was measured using Bruker Avance III 400 and Bruker Avance 300 nuclear magnetic instruments. The measurement solvents were deuterated dimethyl sulfoxide (DMSO-d₆), deuterated chloroform (CDCl₃), deuterated methanol (CD₃OD), and the internal standard was tetramethylsilane (TMS).

The measurement of MS was by Agilent 6120B (ESI) and Agilent 6120B (APCI).

The measurement of HPLC was by Agilent 1260DAD high pressure liquid chromatography (Zorba × SB-C18 100 × 4.6 mm).

Column chromatography generally uses Yantai Huanghai silica gel 200-300 mesh silica gel as the carrier.

The measurement of XRD was analyzed using X-ray powder diffractometer Panalytical EMPYREAN. The scanning parameters are shown in Table 35:

**Table 35: XRPD test parameters**

| Parameters | Instrument 1 | Instrument 2 | Instrument 3 |
|---|---|---|---|
| Model | Empyrean | X' Pert3 | X' Pert3 |
| X-ray | Cu, Kα, | Cu, Kα, | Cu, Kα, |
| | Kα1 (Å): 1.540598, | Kα1 (Å): 1.540598, | Kα1 (Å): 1.540598, |
| | Kα2 (Å): 1.544426 | Kα2 (Å): 1.544426 | Kα2 (Å): 1.544426 |
| | Kα2/Kα1 intensity ratio:0.50 | Kα2/Kα1 intensity ratio:0.50 | Kα2/Kα1 intensity ratio:0.50 |
| X-ray tube setting | 45 kV, 40 mA | 45 kV, 40 mA | 45 kV, 40 mA |
| Divergent slit | 1/8° | 1/8° | 1/8° |
| Scanning mode | Continuous | Continuous | Continuous |
| Scanning range (°2Theta) | 3-40 | 3-40 | 3-40 |
| Scanning time per step (s) | 17.8 | 46.7 | 46.7 |
| Scanning step size (°2Theta) | 0.0167 | 0.0263 | 0.0263 |
| Testing time | ∼5 min 30 s | ~5 min | ~5 min |

The measurements of TGA and DSC were collected on TA Q5000/5500 thermogravimetric analyzer and TA 2500 differential scanning calorimeter, respectively. The test parameters are shown in Table 36:

**Table 36: DSC and TGA test parameters**

| Paremeters | TGA | DSC |
|---|---|---|
| Method | Linear heating | Linear heating |
| Sample tray | Aluminum tray, open | aluminum tray, capped /uncapped |
| Temperature range | Room temperature - setting endpoint temperature | 25°C - setting endpoint temperature |
| Scanning rate (°C/min) | 10 | 10 |
| Protective gas | Nitrogen | Nitrogen |

The known starting materials of the invention can be synthesized by methods known in the art, or can be purchased from Titan Scientific, Energy Chemical, Shang Hai Demo, Chengdu Kelon Chemical, Accela ChemBio, and J&K Scientific, etc.

The solution refers to an aqueous solution if there is no special indication in the examples.

The reaction temperature is room temperature if there is no special indication in the examples.

In the examples, 1 M represents a concentration of 1 mol/L.

Room temperature is 10°C~30°C.

### Example 1: Preparation of compounds represented by formula (I)

### Step 1: Preparation of Compound a

### Tert-butyl(S)-(1-cyano-2-(2-fluoro-4-(3-methyl-2-oxo-2,3-dihydrobenzo[d]oxazol-5-yl)phenyl) ethyl)carbamate (Compound a)

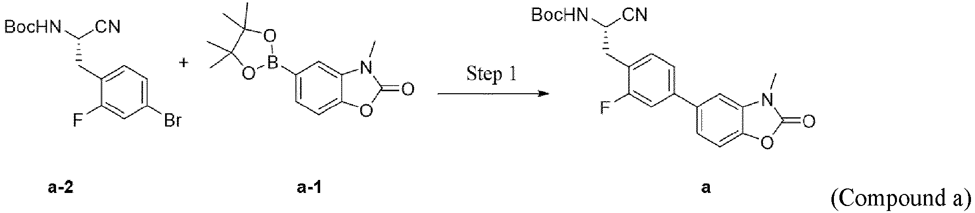

In the 50 L reactor, 10.005 kg 1,4-dioxane, 1.600 kg Compound a-1 and 2.000 kg Compound a-2 were added under stirring, and then 6.605 kg aqueous solution of potassium carbonate (1.600 kg potassium carbonate dissolved in 5.005 kg purified water) was added. After the addition is completed, nitrogen replacement was performed three times. 100.0 g [1,1'-bis(diphenylphosphine)ferrocene]palladium dichloride dichloromethane complex was added, and nitrogen replacement was performed once. Under nitrogen protection, the reaction solution was heated to 80±5°C and reacted for about 2 hours, then the sample was taken for HPLC monitoring. When the control target value of Compound a-2 content is ≤1.0%, the reaction is stopped. 5.000 kg purified water was added to the reaction solution, cooled to 10±5°C, and 10.005 kg purified water was added, stirred and crystallized at 10±5°C for about 1 hour, filtered, washed the filter cake with purified water (2.500 kg×2), and collected the filter cake. 12.605 kg absolute ethanol and filter cake were added into a 50 L reactor, stirred at 20±5°C for about 0.5 hours, filterred, washed the filter cake with absolute ethanol (1.000 kg×2), and collected the filter cake. The filter cake was dried at 55±5°C and vacuum ≤ -0.07 MPa for about 16 hours, and 2.143 kg Compound a was obtained, with a molar yield of 89.4%.

¹H NMR (400 MHz, DMSO) δ 7.90 (s, 1H), 7.72 - 7.30 (m, 6H), 4.72 (s, 1H), 3.41 (d, 3H), 3.09-3.21(m, 2H), 1.37 (s, 9H).

LCMS m/z = 356.1[M-56+H]⁺.

### Step 2: Preparation of Compound b

### (S)-2-amino-3-(2-fluoro-4-(3-methyl-2-oxo-2,3-dihydrobenzo[d]oxazol-5-yl)phenyl)propanenitrile 4-methylbenzenesulfonic acid (Compound b)

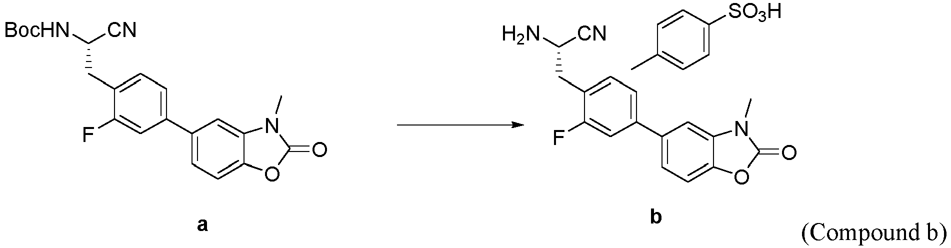

In a 50 L glass reactor, 16.785 kg acetonitrile, 2.1381 kg Compound a and 2.950 kg p-toluenesulfonic acid monohydrate were added under stirring. After the addition is completed, the temperature was controlled to 25±5°C and reacted for about 2 hours, then the sample was taken for HPLC monitoring. When the control target value of Compound a content is ≤1.0%, the reaction is stopped. The reaction solution was filtered, the filter cake was washed with 1.670 kg acetonitrile, and the filter cake was collected. 2.1381 kg acetonitrile and filter cake were added into the reactor, heated to 80±5°C, and stirred for 3 hours. Then it was cooled to 20±5°C and stirred for 1 hour. After filterring, the filter cake was washed with 1.670 kg acetonitrile, and collected the filter cake. The filter cake was dried at 55±5°C and vacuum ≤ -0.07 MPa for about 16 hours, and 2.141 kg Compound b was obtained, with a molar yield of 85.5%.

¹H NMR (400 MHz, DMSO) δ 9.04 (s, 3H), 7.70 - 7.37 (m, 8H), 7.13 (d, 2H), 4.90 (dd, 1H), 3.41 (s, 3H), 3.29 (t, 2H), 2.29 (s, 3H).

LCMS m/z =312.2 [M-172+H]⁺.

### Step 3: Preparation of Compound c

### Tert-butyl(S)-2-(((S)-1-cyano-2-(2-fluoro-4-(3-methyl-2-oxo-2,3-dihydrobenzo[d]oxazol-5-yl) phenyl)ethyl)carbamoyl)-1,4-oxazepane-4-carboxylate (Compound c)

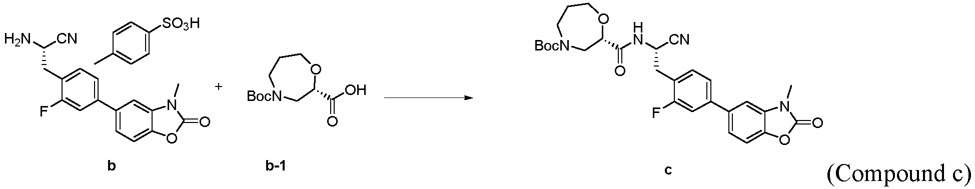

In a 100 L reactor, 19.060 kg ethyl acetate, 2.1413 kg Compound b and 1.1300 kg Compound b-1 were added under stirring, and then 1.725 kg N,N-diisopropylethylamine was added. After the addition is completed, under the protection of nitrogen, the the reaction solution was cooled to 5±5°C, and 4.240 kg propyl phosphoric anhydride was added dropwise at the controlled temperature of 10±5°C. After the addition is completed, the temperature was kept at 25±5°C and reacted for about 3-8 hours. The reaction solution was washed successively with sodium bicarbonate solution (1.070 kg sodium bicarbonate dissolved in 20.350 kg water), citric acid solution (2.150 kg citric acid monohydrate dissolved in 19.275 kg water), aqueous solution of sodium chloride (4.300 kg chloride sodium dissolved in 17.135 kg water). 0.210 kg medicinal charcoal was added to the organic phase, and stirred for about 0.5 hour. It was filtered by 0.540 kg diatomaceous earth pad, and the filter cake was washed with 1.905 kg ethyl acetate. 1.070 kg anhydrous sodium sulfate was added to the organic phase, and dried for about 0.5 hours. It was filterred, and the filter cake was washed with 1.905 kg ethyl acetate, the filtrate was combined and was concentrated under reduced pressure at 50±5°C until no obvious fraction flows out. 2.385 kg Compound c was obtained, which is directly used in the next reaction step.

¹H NMR (400 MHz, CDCl₃) δ 7.54 - 7.01 (m, 7H), 5.18 (s, 1H), 4.25 - 3.94 (m, 3H), 3.54 (dd, 2H), 3.46 (s, 3H), 3.39 - 3.04 (m, 4H), 1.99 (d, 2H), 1.54 - 1.39 (m, 9H).

LCMS m/z =483.2 [M-56+1]⁺.

### Step 4: (S)-N-((S)-1-cyano-2-(2-fluoro-4-(3-methyl-2-oxo-2,3-dihydrobenzo[d]oxazol-5-yl)phenyl) ethyl)-1,4-oxazepane-2-carboxamide (Formula I)

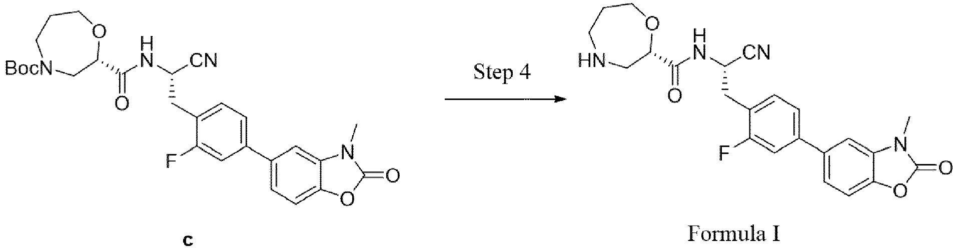

In a 50 L double-layer glass reactor containing the concentrated Compound c, 9.305 kg acetonitrile and 2.530 kg p-toluenesulfonic acid monohydrate were added under stirring. After the addition is completed, the temperature was kept at 25±5°C and reacted for about 2 hours, then the sample was taken for HPLC monitoring. When the control target value of Compound c content is ≤1.0%, the reaction was stopped. The reaction solution was cooled to 10±5°C, the diluted ammonia solution (1.075 kg ammonia solution mixed with 36.000 kg purified water) was added dropwise, and the temperature of the material was controlled below 25°C during the dropwise addition. After the addition was completed, it was cooled to 10±5°C and crystallized for 2 hours. It was filterred, and the filter cake was washed with 11.930 kg purified water. The filter cake and 14.890 kg ethanol were added into a 50 L double-layer glass reactor, and stirred at 20±5°C for 0.5 hours, filtered. The filter cake was washed with 1.860 kg ethanol, and collected the filter cake. The filter cake was dried at 55±5°C and vacuum degree ≤ -0.07 MPa for about 13 hours, and 1.6627 kg of crude compound (Formula I) was obtained, with a molar yield of 85.6%.

In a 100 L reactor, 9.100 kg acetonitrile, 9.220 kg absolute ethanol and 1.6627 kg crude compound (Formula I) were added under stirring. It was heated to an internal temperature of 75±5°C, stirred to dissolve to clear, and filterred while hot. The filtrate was transferred to a 100 L reactor (if the filtrate has product precipitation, it should be heated to dissolve to clear). It was cooled to 35±5°C under stirring, kept the temperature until obvious solids were precipitated, and then kept the temperature and stirred for about 0.5 hours. Then it was cooled to 5±5°C, kept the temperature and crystallized for 2 hours. It was filterred, and the filter cake was washed with 1.300 kg ethanol, collected the filter cake, and dried the filter cake at 55±5°C and vacuum degree ≤ -0.07 MPa for about 25 hours. 1.4060 kg of compound formula I was obtained, with a molar yield of 84.6%.

¹H NMR (400 MHz, DMSO) δ 8.69 (d, 1H), 7.64 (d, 1H), 7.61 - 7.51 (m, 2H), 7.46 (t, 2H), 7.39 (d, 1H), 5.06 (q, 1H), 4.01 (dd, 1H), 3.87 (ddd, 1H), 3.73 (ddd, 1H), 3.40 (s, 3H), 3.34 - 3.28 (m, 1H), 3.20 (dd, 1H), 3.06 (dd, 1H), 2.78 (ddd, 1H), 2.69 - 2.54 (m, 2H), 2.21 (s, 1H), 1.84 - 1.63 (m, 2H)_{∘} LCMS m/z =439.2 [M+1]⁺_{∘}

### Example 2: Preparation of the crystalline form A of the hydrochloride of the compound represented by formula (I)

200 mg of the compound represented by formula (I) was taken, and 5 mL of acetone and 1 eq 1 M hydrochloric acid were added. It was stirred at room temperature for 3 days, filterred and dried, and the crystalline form A of the hydrochloride was obtained. The crystalline form A of the hydrochloride of the compound represented by formula (I) is characterized by XRD, DSC and TGA, as shown in Figures 1-3 in sequence.

### Example 3: Preparation of the crystalline form A of the sulfate of the compound represented by formula (I)

200 mg of the compound represented by formula (I) was taken, and 5 mL of methanol and 1 eq 1 M sulfuric acid were added. It was stirred at room temperature for 3 days, filterred and dried, and the crystalline form A of the sulfate was obtained. The crystalline form A of the sulfate of the compound represented by formula (I) is characterized by XRD, DSC and TGA, as shown in Figures 10-12 in sequence.

### Example 4: Preparation of the crystalline form B of the sulfate of the compound represented by formula (I)

200 mg of the compound represented by formula (I) was taken, and 5 mL of acetone and 1 eq 1 M sulfuric acid were added. It was stirred at room temperature for 3 days, filterred and dried, and the crystalline form B of the sulfate was obtained. The crystalline form B of the sulfate of the compound represented by formula (I) is characterized by XRD, DSC and TGA, as shown in Figures 13-15 in sequence.

### Example 5: Preparation of the crystalline form A of the maleate of the compound represented by formula (I)

200 mg of the compound represented by formula (I) was taken, and 5 mL of methanol and 1 eq maleic acid were added. It was stirred at room temperature for 3 days, filterred and dried, and the crystalline form A of the maleate was obtained. The crystalline form A of the maleate of the compound represented by formula (I) is characterized by XRD, DSC and TGA, as shown in Figures 16-18 in sequence.

### Example 6: Preparation of the crystalline form B of the maleate of the compound represented by formula (I)

200 mg of the compound represented by formula (I) was taken, and 5 mL of acetone and 1 eq maleic acid were added. It was stirred at room temperature for 3 days, filterred and dried, and the crystalline form B of the maleate was obtained. The crystalline form B of the maleate of the compound represented by formula (I) is characterized by XRD, DSC and TGA, as shown in Figures 19-21 in sequence.

### Example 7: Preparation of the crystalline form C of the maleate of the compound represented by formula (I)

200 mg of the compound represented by formula (I) was taken, and 5 mL of 2-methyltetrahydrofuran and 1 eq maleic acid were added. It was stirred at room temperature for 3 days, filterred and dried, and the crystalline form C of the maleate was obtained. The crystalline form C of the maleate of the compound represented by formula (I) is characterized by XRD, DSC and TGA, as shown in Figures 22-24 in sequence.

### Example 8: Preparation of the crystalline form A of the phosphate of the compound represented by formula (I)

200 mg of the compound represented by formula (I) was taken, and 5 mL of methanol and 1 eq phosphoric acid were added. It was stirred at room temperature for 3 days, filterred and dried, and the crystalline form A of the phosphate was obtained. The crystalline form A of the phosphate of the compound represented by formula (I) is characterized by XRD, DSC and TGA, as shown in Figures 25-27 in sequence.

### Example 9: Preparation of the crystalline form B of the phosphate of the compound represented by formula (I)

200 mg of the compound represented by formula (I) was taken, and 5 mL of acetone and 1 eq phosphoric acid were added. It was stirred at room temperature for 3 days, filterred and dried, and the crystalline form B of the phosphate was obtained. The crystalline form B of the phosphate of the compound represented by formula (I) is characterized by XRD, DSC and TGA, as shown in Figures 28-30 in sequence.

### Example 10: Preparation of the crystalline form C of the phosphate of the compound represented by formula (I)

200 mg of the compound represented by formula (I) was taken, and 5 mL of 2-methyltetrahydrofuran and 1 eq phosphoric acid were added. It was stirred under suspension at room temperature for 3 days, filterred and dried, and the crystalline form C of the phosphate was obtained. The crystalline form C of the phosphate of the compound represented by formula (I) is characterized by XRD, DSC and TGA, as shown in Figures 31-33 in sequence.

### Example 11: Preparation of the crystalline form A of the mucate of the compound represented by formula (I)

200 mg of the compound represented by formula (I) was taken, and 5 mL of methanol and 1 eq mucic acid were added. It was stirred at room temperature for 3 days, filterred and dried, and the crystalline form A of the mucate was obtained. The crystalline form A of the mucate of the compound represented by formula (I) is characterized by XRD, DSC and TGA, as shown in Figures 34-36 in sequence.

### Example 12: Preparation of the crystalline form A of the tartrate of the compound represented by formula (I)

200 mg of the compound represented by formula (I) was taken, and 5 mL of methanol and 1 eq tartaric acid were added. It was stirred at room temperature for 3 days, filterred and dried, and the crystalline form A of the tartrate was obtained. The crystalline form A of the tartrate of the compound represented by formula (I) is characterized by XRD, DSC and TGA, as shown in Figures 37-39 in sequence.

### Example 13: Preparation of the crystalline form B of the tartrate of the compound represented by formula (I)

200 mg of the compound represented by formula (I) was taken, and 5 mL of acetone and 1 eq tartaric acid were added. It was stirred at room temperature for 3 days, filterred and dried, and the crystalline form B of the tartrate was obtained. The crystalline form B of the tartrate of the compound represented by formula (I) is characterized by XRD, DSC and TGA, as shown in Figures 40-42 in sequence.

### Example 14: Preparation of the crystalline form C of the tartrate of the compound represented by formula (I)

200 mg of the compound represented by formula (I) was taken, and 5 mL of 2-methyltetrahydrofuran and 1 eq tartaric acid were added. It was stirred at room temperature for 3 days, filterred and dried, and the crystalline form C of the tartrate was obtained. The crystalline form C of the tartrate of the compound represented by formula (I) is characterized by XRD, DSC and TGA, as shown in Figures 43-45 in sequence.

### Example 15: Preparation of the crystalline form A of the fumarate of the compound represented by formula (I)

200 mg of the compound represented by formula (I) was taken, and 5 mL of acetone and 1 eq fumaric acid were added. It was stirred at room temperature for 3 days, filterred and dried, and the crystalline form A of the fumarate was obtained. The crystalline form A of the fumarate of the compound represented by formula (I) is characterized by XRD, DSC and TGA, as shown in Figures 46-48 in sequence.

### Example 16: Preparation of the crystalline form B of the fumarate of the compound represented by formula (I)

200 mg of the compound represented by formula (I) was taken, and 5 mL of 2-methyltetrahydrofuran and 1 eq fumaric acid were added. It was stirred at room temperature for 3 days, filterred and dried, and the crystalline form B of the fumarate was obtained. The crystalline form B of the fumarate of the compound represented by formula (I) is characterized by XRD, DSC and TGA, as shown in Figures 49-51 in sequence.

### Example 17: Preparation of the crystalline form A of the citrate of the compound represented by formula (I)

200 mg of the compound represented by formula (I) was taken, and 5 mL of methanol and 1 eq citric acid were added. It was stirred at room temperature for 3 days, filterred and dried, and the crystalline form A of the citrate was obtained. The crystalline form A of the citrate of the compound represented by formula (I) is characterized by XRD, DSC and TGA, as shown in Figures 52-54 in sequence.

### Example 18: Preparation of the crystalline form B of the citrate of the compound represented by formula (I)

200 mg of the compound represented by formula (I) was taken, and 5 mL of acetone and 1 eq citric acid were added. It was stirred at room temperature for 3 days, filterred and dried, and the crystalline form B of the citrate was obtained. The crystalline form B of the citrate of the compound represented by formula (I) is characterized by XRD, DSC and TGA, as shown in Figures 55-57 in sequence.

### Example 19: Preparation of the crystalline form A of the malate of the compound represented by formula (I)

200 mg of the compound represented by formula (I) was taken, and 5 mL of methanol and 1 eq malic acid were added. It was stirred at room temperature for 3 days, filterred and dried, and the crystalline form A of the malate was obtained. The crystalline form A of the malate of the compound represented by formula (I) is characterized by XRD, DSC and TGA, as shown in Figures 4-6 in sequence.

### Example 20: Preparation of the crystalline form B of the malate of the compound represented by formula (I)

200 mg of the compound represented by formula (I) was taken, and 5 mL of acetone and 1 eq malic acid were added. It was stirred at room temperature for 3 days, filterred and dried, and the crystalline form B of the malate was obtained. The crystalline form B of the malate of the compound represented by formula (I) is characterized by XRD, DSC and TGA, as shown in Figures 58-60 in sequence.

### Example 21: Preparation of the crystalline form A of the hippurate of the compound represented by formula (I)

200 mg of the compound represented by formula (I) was taken, and 5 mL of acetone and 1 eq hippuric acid were added. It was stirred at room temperature for 3 days, filterred and dried, and the crystalline form A of the hippurate was obtained. The crystalline form A of the hippurate of the compound represented by formula (I) is characterized by XRD, DSC and TGA, as shown in Figures 61-63 in sequence.

### Example 22: Preparation of the crystalline form A of the adipate of the compound represented by formula (I)

200 mg of the compound represented by formula (I) was taken, and 5 mL of acetone and 1 eq adipic acid were added. It was stirred at room temperature for 3 days, filterred and dried, and the crystalline form A of the adipate was obtained. The crystalline form A of the adipate of the compound represented by formula (I) is characterized by XRD, DSC and TGA, as shown in Figures 64-66 in sequence.

### Example 23: Preparation of the crystalline form B of the adipate of the compound represented by formula (I)

200 mg of the compound represented by formula (I) was taken, and 5 mL of ethyl acetate and 1 eq adipic acid were added. It was stirred at room temperature for 3 days, filterred and dried, and the crystalline form B of the adipate was obtained. The crystalline form B of the adipate of the compound represented by formula (I) is characterized by XRD, DSC and TGA, as shown in Figures 7-9 in sequence.

### Example 24: Preparation of the crystalline form A of the sebacate of the compound represented by formula (I)

200 mg of the compound represented by formula (I) was taken, and 5 mL of methanol and 1 eq sebacic acid were added. It was stirred at room temperature for 3 days, filterred and dried, and the crystalline form A of the sebacate was obtained. The crystalline form A of the sebacate of the compound represented by formula (I) is characterized by XRD, DSC and TGA, as shown in Figures 67-69 in sequence.

### Example 25: Preparation of the crystalline form B of the sebacate of the compound represented by formula (I)

200 mg of the compound represented by formula (I) was taken, and 5 mL of acetone and 1 eq sebacic acid were added. It was stirred at room temperature for 3 days, filterred and dried, and the crystalline form B of the sebacate was obtained. The crystalline form B of the sebacate of the compound represented by formula (I) is characterized by XRD, DSC and TGA, as shown in Figures 70-72 in sequence.

### Example 26: Preparation of the crystalline form C of the sebacate of the compound represented by formula (I)

200 mg of the compound represented by formula (I) was taken, and 5 mL of ethyl acetate and 1 eq sebacic acid were added. It was stirred at room temperature for 3 days, filterred and dried, and the crystalline form C of the sebacate was obtained. The crystalline form C of the sebacate of the compound represented by formula (I) is characterized by XRD, DSC and TGA, as shown in Figures 73-75 in sequence.

### Example 27: Preparation of the crystalline form A of the 1,5-naphthalenedisulfonate of the compound represented by formula (I)

200 mg of the compound represented by formula (I) was taken, and 5 mL of methanol and 1 eq 1,5-naphthalenedisulfonic acid were added. It was stirred at room temperature for 3 days, filterred and dried, and the crystalline form A of the 1,5-naphthalenedisulfonate was obtained. The crystalline form A of the 1,5-naphthalenedisulfonate of the compound represented by formula (I) is characterized by XRD, DSC and TGA, as shown in Figures 76-78 in sequence.

### Example 28: Preparation of the crystalline form A of the methanesulfonate of the compound represented by formula (I)

200 mg of the compound represented by formula (I) was taken, and 5 mL of methanol and 1 eq methanesulfonic acid were added. It was stirred at room temperature for 3 days, filterred and dried, and the crystalline form A of the methanesulfonate was obtained. The crystalline form A of the methanesulfonate of the compound represented by formula (I) is characterized by XRD, DSC and TGA, as shown in Figures 79-81 in sequence.

### Example 29: Preparation of the crystalline form A of the benzenesulfonate of the compound represented by formula (I)

200 mg of the compound represented by formula (I) was taken, and 5 mL of acetone and 1 eq benzenesulfonic acid were added. It was stirred at room temperature for 3 days, filterred and dried, and the crystalline form A of the benzenesulfonate was obtained. The crystalline form A of the benzenesulfonate of the compound represented by formula (I) is characterized by XRD, DSC and TGA, as shown in Figures 82-84 in sequence.

### Example 30: Preparation of the crystalline form B of the benzenesulfonate of the compound represented by formula (I)

200 mg of the compound represented by formula (I) was taken, and 5 mL of ethyl acetate and 1 eq benzenesulfonic acid were added. It was stirred at room temperature for 3 days, filterred and dried, and the crystalline form B of the benzenesulfonate was obtained. The crystalline form B of the benzenesulfonate of the compound represented by formula (I) is characterized by XRD, DSC and TGA, as shown in Figures 85-87 in sequence.

### Example 31: Preparation of the crystalline form A of the oxalate of the compound represented by formula (I)

200 mg of the compound represented by formula (I) was taken, and 5 mL of ethyl acetate and 1 eq oxalic acid were added. It was stirred at room temperature for 3 days, filterred and dried, and the crystalline form A of the oxalate was obtained. The crystalline form A of the oxalate of the compound represented by formula (I) is characterized by XRD, DSC and TGA, as shown in Figures 88-90 in sequence.

### Example 32: Preparation of the crystalline form A of the benzoate of the compound represented by formula (I)

200 mg of the compound represented by formula (I) was taken, and 5 mL of methanol and 1 eq benzoic acid were added. It was stirred at room temperature for 3 days, filterred and dried, and the crystalline form A of the benzoate was obtained. The crystalline form A of the benzoate of the compound represented by formula (I) is characterized by XRD, DSC and TGA, as shown in Figures 91-93 in sequence.

### Example 33: Preparation of the crystalline form B of the benzoate of the compound represented by formula (I)

200 mg of the compound represented by formula (I) was taken, and 5 mL of acetone and 1 eq benzoic acid were added. It was stirred at room temperature for 3 days, filterred and dried, and the crystalline form B of the benzoate was obtained. The crystalline form B of the benzoate of the compound represented by formula (I) is characterized by XRD, DSC and TGA, as shown in Figures 94-96 in sequence.

### Example 34: Preparation of the crystalline form C of the benzoate of the compound represented by formula (I)

200 mg of the compound represented by formula (I) was taken, and 5 mL of ethyl acetate and 1 eq benzoic acid were added. It was stirred at room temperature for 3 days, filterred and dried, and the crystalline form C of the benzoate was obtained. The crystalline form C of the benzoate of the compound represented by formula (I) is characterized by XRD, DSC and TGA, as shown in Figures 97-99 in sequence.

### Example 35: Preparation of the crystalline form A of the hydrobromide of the compound represented by formula (I)

200 mg of the compound represented by formula (I) was taken, and 5 mL of ethyl acetate and 1 eq hydrobromic acid were added, and the seeds were added. It was stirred at room temperature for 3 days, filterred and dried, and the crystalline form A of the hydrobromide was obtained. The crystalline form A of the hydrobromide of the compound represented by formula (I) is characterized by XRD, DSC and TGA, as shown in Figures 100-102 in sequence.

### Evaluation test of crystalline form

1. After the crystalline A of hydrochloride, crystalline A of malate and crystalline B of adipate were placed at 25°C/60%RH and 40°C/75%RH, and their physical and chemical stability were tested by XRPD and HPLC respectively. The results are shown in Table 37.

**Table 37: Solid stability evaluation**

| Materials | Conditions | Time | HPLC purity (Area%) | Purity/Starting purity (%) | Crystalline form change |
|---|---|---|---|---|---|
| Crystalline A of hydrochloride | Starting | - | 99.63 | - | - |
| | 25°C/60%RH | 1 week | 99.60 | 100.0 | No |
| | | 3 weeks | 99.67 | 100.0 | N/A |
| | 40°C/75%RH | 1 week | 99.59 | 100.0 | No |
| | | 3 weeks | 99.17 | 99.5 | N/A |
| Crystalline A of malate | Starting | | 99.90 | | |
| | 25°C/60%RH | 1 week | 99.89 | 100.0 | No |
| | | 3 weeks | 99.91 | 100.0 | N/A |
| | 40°C/75%RH | 1 week | 99.92 | 100.0 | No |
| | | 3 weeks | 99.92 | 100.0 | N/A |
| Crystalline B of adipate | Starting | - | 99.74 | - | - |
| | 25°C/60%RH | 1 week | 99.71 | 100.0 | No |
| | | 3 weeks | 99.72 | 100.0 | N/A |
| | 40°C/75%RH | 1 week | 99.74 | 100.0 | No |
| | | 3 weeks | 99.72 | 100.0 | N/A |

The results showed that the crystalline form of each sample did not change after being placed under the conditions of 25°C/60%RH and 40°C/75%RH for 1 week to 1 month, and the purity did not decrease significantly, and the stability was good.

### 2. HPLC impurities of solid stability samples

After the crystalline A of hydrochloride, crystalline A of malate and crystalline B of adipate were placed at 25°C/60%RH and 40°C/75%RH for 1W and 3W respectively, impurities of their solid stability samples were tested by HPLC. HPLC test conditions are shown in Table 38, and the test results are shown in Table 39.

**Table 38: HPLC test conditions of solid stability evaluation**

| Liquid chromatograph | Waters H-Class with PDA detector | |
|---|---|---|
| Chromatographic column | ACQUITYUPLC BEH C18 2.1 mm/50 mm/1.7 µm | |
| Mobile phase | A: 0.05% TFA in H₂O | |
| | B: ACN | |
| Elution gradient | Time (min) | %B |
| | 0.00 | 5 |
| | 4.00 | 95 |
| | 5.00 | 95 |
| | 5.01 | 5 |
| | 7.00 | 5 |
| Running time | 7.0 min | |
| Flow velocity of mobile phase | 0.5 mL/min | |
| Injection volume | 3 µL | |
| Detection wavelength | UV at 254 nm / 210 nm | |
| Column temperature | 35°C | |
| Injector temperature | RT | |
| Diluent | MeOH or ACN/H₂O (1:1, v/v) | |

**Table 39: HPLC impurities of solid stability samples**

| No. | RRT | Starting | 25 °C/ 60%RH/1W | 25 °C/ 60%RH/3W | 40 °C/ 75%RH/1W | 40 °C/ 75%RH/3W |
|---|---|---|---|---|---|---|
| Crystalline A of hydrochloride | 1.00 | 99.63 | 99.60 | 99.67 | 99.59 | - |
| | 1.06 | 0.21 | 0.26 | 0.21 | 0.20 | - |
| | 1.12 | 0.16 | 0.14 | 0.13 | 0.12 | 0.18 |
| | 1.16 | - | - | - | - | 0.06 |
| | 1.28 | - | - | - | 0.09 | 0.17 |
| Crystalline A of malate | 1.00 | 99.90 | 99.89 | 99.91 | 99.92 | 99.92 |
| | 1.12 | 0.10 | 0.11 | 0.09 | 0.08 | 0.08 |
| Crystalline B of adipate | 1.00 | 99.74 | 99.71 | 99.72 | 99.74 | 99.72 |
| | 1.12 | 0.26 | 0.29 | 0.28 | 0.26 | 0.28 |

The results showed that the purity of each sample did not decrease significantly after being placed under the conditions of 25°C/60%RH and 40°C/75%RH for 1 week to 1 month, and the stability was good.

### 3. Dynamic solubility

About 20 mg of material (calculated as free base) was weighted into a 5 mL vial, 4 mL of solvent was added, rotated and mixed at 37°C for 1, 2, 4 and 24 hours (25 rpm). A sample of about 0.9 mL was taken, centrifuged and filterred, tested the solid by XRPD, and tested the liquid by HPLC concentration and pH. The test results are shown in Table 40.

**Table 40: Dynamic solubility**

| Materials | vehicles | 1 hour | | | 2 hours | | | 4 hours | | | 24 hours | | |
|---|---|---|---|---|---|---|---|---|---|---|---|---|---|
| | | Solubility | pH | Crystalline form change | Solubility | pH | Crystalline form change | Solubility | pH | Crystalline form change | Solubility | pH | Crystalline form change |
| Crystalline A of hydrochloride | SGF | 3.7 | 1.7 | No | 3.9 | 1.9 | No | 4.0 | 1.8 | No | 3.4 | 1.8 | No |
| | FaSSIF | 1.7 | 5.8 | C | 1.6 | 5.8 | C | 1.5 | 5.8 | C | 1.4 | 5.8 | C |
| | FeSSIF | 2.9 | 5.0 | No | 2.9 | 5.0 | G | 3.0 | 4.9 | G | 2.6 | 5.1 | G |
| | H₂O | >5.2 | 5.7 | N/A | >5.0 | 5.7 | N/A | >5.1 | 5.7 | N/A | >4.9 | 5.7 | N/A |
| Crystalline A of malate | SGF | >5.3 | 2.4 | N/A | >5.3 | 2.5 | N/A | >5.3 | 2.5 | N/A | >5.3 | 2.5 | N/A |
| | FaSSIF | 3.5 | 5.8 | A | 3.0 | 5.9 | C | 2.9 | 5.9 | C | 2.7 | 5.8 | C |
| | FeSSIF | 2.6 | 5.0 | G | 2.8 | 5.0 | G | 2.7 | 5.0 | G | 2.7 | 5.0 | G |
| | H₂O | >5.5 | 5.3 | N/A | >5.4 | 5.2 | N/A | >5.4 | 5.4 | N/A | >5.3 | 5.2 | N/A |
| Crystalline B of adipate | SGF | 4.4 | 2.3 | L | 4.5 | 2.4 | L | 4.5 | 2.5 | L | 4.4 | 2.5 | L |
| | FaSSIF | >4.9 | 5.5 | N/A | >5.0 | 5.5 | N/A | >5.0 | 5.5 | N/A | >5.0 | 5.5 | N/A |
| | FeSSIF | 2.8 | 4.9 | G | 2.8 | 4.9 | G | 2.9 | 4.8 | G | 2.7 | 4.9 | G |
| | H₂O | >5.0 | 4.8 | N/A | >4.9 | 4.9 | N/A | >4.9 | 4.9 | N/A | >4.9 | 4.9 | N/A |
| Free base | SGF | >5.2 | 2.7 | N/A | >5.2 | 2.8 | N/A | >5.3 | 2.8 | N/A | >5.2 | 2.7 | N/A |
| | FaSSIF | 0.37 | 6.5 | No | 0.36 | 6.5 | No | 0.35 | 6.5 | No | 0.29 | 6.5 | C |
| | FeSSIF | 2.7 | 5.2 | G | 2.7 | 5.3 | G | 2.7 | 5.2 | G | 2.3 | 5.4 | G |
| | H₂O | 0.035 | 8.4 | B+C | 0.030 | 8.2 | B+C | 0.016 | 8.4 | B+C | 0.016 | 8.4 | B+C |

Solubility: mg/mL. N/A: The sample is dissolved to clear and no XRPD data was collected.
L: The sample is nearly clear, the solid content is too small, and no XRPD data was collected.
G: The sample is gelled; A: Amorphous; C: Disproportionated into a free state.

Results: The water solubility of either salt is significantly improved compared to the free base.

### 4. Hygroscopicity

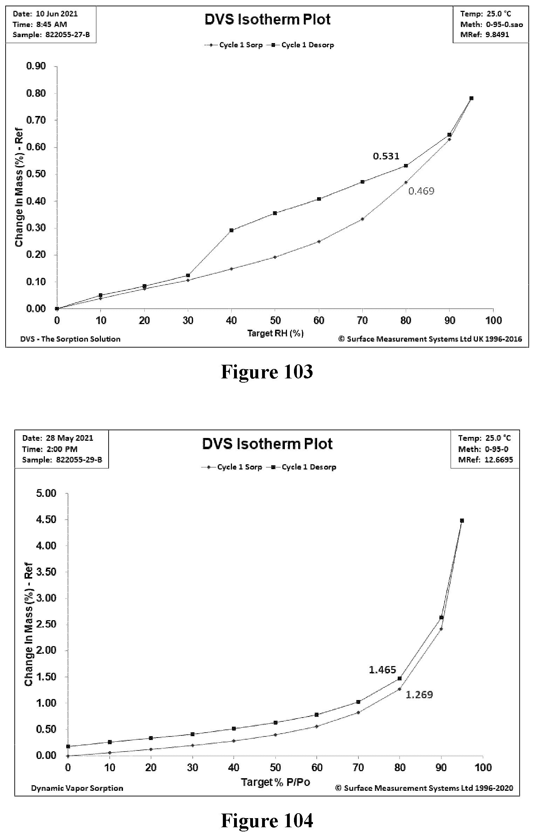

The hygroscopicity of the crystalline A of hydrochloride, crystalline A of malate and crystalline B of adipate was evaluated by a Dynamic Vapour Sorption (DVS) instrument. Crystalline form C starts with ambient humidity (-60% RH), and crystalline form B starts with 0% relative humidity (0% RH). The test collected mass change percentage of the sample under constant temperature conditions of 25°C, with changes in humidity (60%RH-95%RH-0%RH-95%RH or 0%RH-95%RH-0%RH). The test results are shown in Figures 103, 104 and 105 respectively. The results show that the water sorption of crystalline A of hydrochloride, crystalline A of malate and crystalline B of adipate at 25°C/80%RH is 0.47%, 1.27% and 0.24% respectively. The crystalline form of all samples did not change after DVS test. This shows that the crystalline form of the invention has low hygroscopicity and has low requirements on pharmaceutical packaging and storage conditions.

### 5. PLM

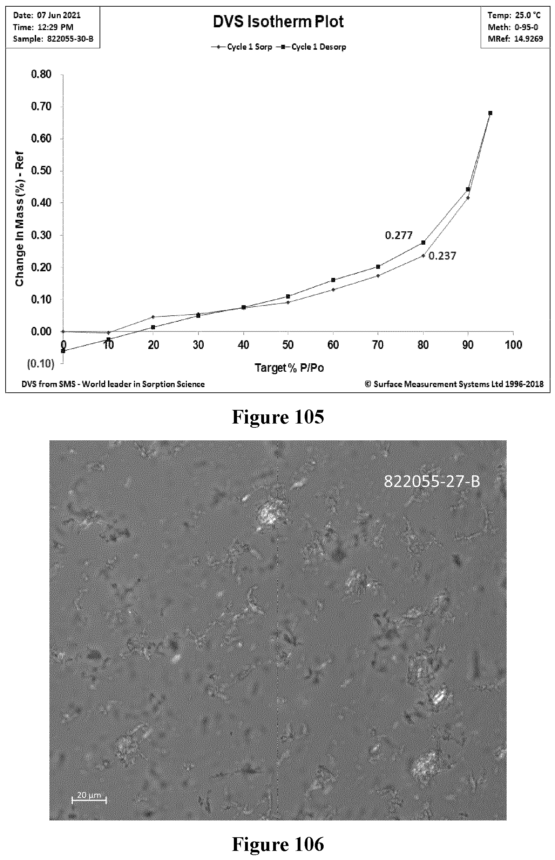

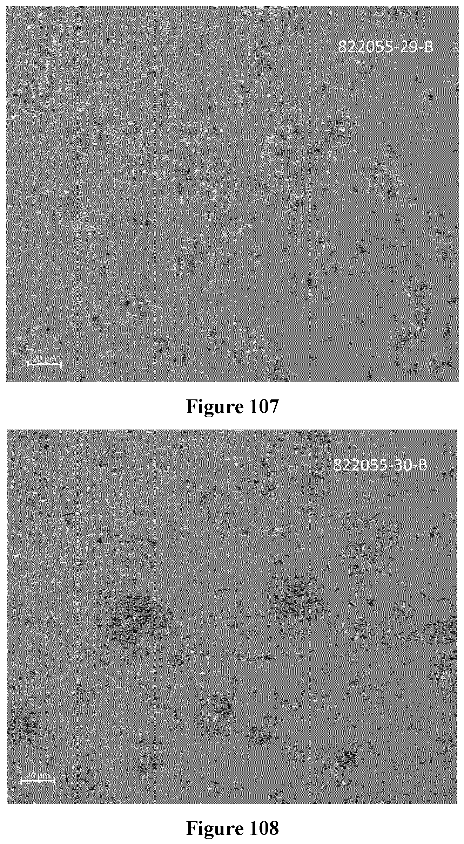

PLM characterization was performed on crystalline A of hydrochloride, crystalline A of malate and crystalline B of adipate. The results (Figure 106 to Figure 108) show that the particle size of each sample is less than 20 µm.

### Biological tests

### 1. Activity detection test of DPP1 enzyme in vitro

Recombinant human DPP1 enzyme (R&D Systems, Cat. No 1071-CY) with a final concentration of 100 µg/mL and recombinant human cathepsin L (R&D System, Cat. No 952-CY) with a final concentration of 20 µg/mL were mixed, and then incubated at room temperature for 1 hour, to activate DPP1 enzyme. The activated DPP1 enzyme was diluted 100 times, and 5 µL of different concentrations of compounds and 5 µL of the diluted DPP1 enzyme were added to a 384-well plate, and incubated at room temperature for 30 minutes. After adding 10 µL of the substrate Gly-Arg-AMC (bachem, Cat. No I-1215) with a concentration of 20 µM, it was continued to incubate at room temperature for 60 minutes. The fluorescence intensity was detected on a microplate reader, in which the excitation wavelength is 380 nm and the emission wavelength is 460 nm. The IC₅₀ value was calculated using the Origin2019 software DosResp function.

**Table 41: DPP1 inhibitory activity**

| Compound No. | IC₅₀/nM |
|---|---|
| Compound I | 1.6 |

Conclusion: Compound 1 shows high inhibitory activity against DPP1 receptor.

### 2. The pharmacokinetic test of rat

1.1 Test animals: male SD rats, about 220 g, 6 to 8 weeks old, 6 rats/compound. Purchased from Chengdu Senwell Experimental Animals Co., Ltd.

1.2 Test design: On the day of the test, 6 SD rats were randomly divided into groups according to body weight. Water and no food for 12 to 14 hours one day before administration, and food 4 hours after administration.

**Table 42: Administration Information**

| Groups | Number | Administration Information | | | | | |
|---|---|---|---|---|---|---|---|
| | Male | Test substance | Administation dosage (mg/kg) | Administration concentration (mg/mL) | Administration volume (mL/kg) | Collected sample | Administration manner |
| G1 | 3 | INS1007 | 1 | 0.2 | 5 | Plasma | Intravenous |
| G2 | 3 | Compound I | 1 | 0.2 | 5 | Plasma | |
| G3 | 3 | INS1007 | 3 | 0.3 | 10 | Plasma | Gavage |
| G4 | 3 | Compound I | 3 | 0.3 | 10 | Plasma | |

Vehicle for intravenous administration: 5%DMA+5%Solutol+90%Saline; vehicle for gavage administration: 0.5%MC; the reference compound INS1007, which is Compound 2 in patent WO2015110826A1, was prepared according to the method of the patent.

Before and after administration, 0.1 mL of blood was taken from the orbit under isoflurane anesthesia. It was placed in an EDTAK2 centrifuge tube, and centrifuged at 5000 rpm and 4°C for 10 min, and the plasma was collected. Time points of blood collection in the intravenous groups: 0, 5, 15, 30 min, 1, 2, 4, 6, 8, 24 h; time points of blood collection in the gavage groups: 0, 5, 15, 30 min, 1, 2, 4, 6, 8, 24 h. Before analysis and detection, all samples were stored at -80°C.

**Table 43: Pharmacokinetic parameters of test compounds in rat plasma**

| Test compounds | Administration manner | CL (mL/min/kg) | Vdₛₛ (L/kg) | AUC₀₋ₜ (hr*ng/mL) | F (%) |
|---|---|---|---|---|---|
| INS1007 | Intravenous (1 mg/kg) | 2.08 | 0.738 | 8396 | - |
| Compound 1 | | 1.66 | 0.753 | 9503 | - |
| INS1007 | Gavage (3 mg/kg) | - | - | 22201 | 88.1 |
| Compound 1 | | - | - | 31159 | >100 |

Conclusion: The compounds of the invention have good bioavailability and pharmacokinetic characteristics.

### 3. Toxicity test of 14-day oral repeated administration in rat

SD rats were randomly divided into groups according to body weight, including vehicle control group (0.5% MC), INS1007 (30, 100, 300 mg/kg) groups, and Compound I (30, 100, 300 mg/kg) groups. Each drug administration group includes 16 animals, and the vehicle control group includes 10 animals, half male and half female. Drugs of corresponding concentrations or vehicles were given by oral gavage every day for 14 consecutive days, and the recovery period was 7 days. During the administration period, the observation of general symptoms, and the measurement of body weight and food intake were taken in each group. At the end of the administration period and the recovery period, hematology, serum biochemistry and gross anatomy tests were conducted on each group.

Conclusion: At the same dosage, the compound of the invention is less toxic than INS1007 and has higher safety.

## Claims

1. Salts of a compound represented by formula (I) and hydrates and solvates thereof: 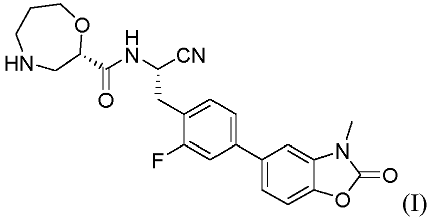 wherein, the salt is selected from the group consisting of hydrochloride, sulfate, maleate, phosphate, mucate, tartrate, fumarate, citrate, malate, hippurate, adipate, sebacate, 1,5-naphthalenedisulfonate, methanesulfonate, benzenesulfonate, oxalate, benzoate, hydrobromide, 2-naphthalenesulfonate, p-toluenesulfonate, hemi-1,5-naphthalenedisulfonate, or succinate.

2. The salts and hydrates and solvates thereof according to claim 1, wherein the salt is selected from the group consisting of hydrochloride, sulfate, maleate, phosphate, mucate, tartrate, fumarate, citrate, malate, hippurate, adipate, sebacate, 1,5-naphthalenedisulfonate, methanesulfonate, benzenesulfonate, oxalate, benzoate, or hydrobromide, preferably in crystalline form.

3. The salts and hydrates and solvates thereof according to claim 1, wherein the salt is selected from the group consisting of hydrochloride, malate or adipate, preferably in crystalline form.

4. The salts and hydrates and solvates thereof according to any one of claims 1-3, wherein the salt is selected from the group consisting of hydrochloride, wherein the hydrochloride is a crystalline form (crystalline form A of the hydrochloride), and its X-ray powder diffraction pattern has characteristic diffraction peaks at the following 2θ positions: 9.38°±0.2°, 16.40°±0.2°, 18.69°±0.2°, 22.04°±0.2°, 23.05°±0.2°, 23.90°±0.2°, using Cu-Kα radiation.

5. The salts and hydrates and solvates thereof according to claim 4, wherein its X-ray powder diffraction pattern has characteristic diffraction peaks at the following 2θ positions: 13.99°±0.2°, 14.75° ±0.2°, 17.92°±0.2°, 25.70°±0.2°, 30.32°±0.2°, using Cu-Kα radiation.

6. The salts and hydrates and solvates thereof according to claim 5, wherein its X-ray powder diffraction pattern is shown in Figure 1.

7. The salts and hydrates and solvates thereof according to claim 5, wherein its thermogravimetric analysis (TGA) thermogram is shown in Figure 2, and the differential scanning calorimetry (DSC) thermogram is shown in Figure 3.

8. The salts and hydrates and solvates thereof according to any one of claims 1 to 3, wherein the salt is selected from the group consisting of malate, wherein the malate is a crystalline form (crystalline form A of the malate), and its X-ray powder diffraction pattern has characteristic diffraction peaks at the following 2θ positions: 8.75°±0.2°, 9.82°±0.2°, 15.08°±0.2°, 16.65°±0.2°, 20.89°±0.2°, 21.89°±0.2 °, 23.75°±0.2°, using Cu-Kα radiation.

9. The salts and hydrates and solvates thereof according to claim 8, wherein its X-ray powder diffraction pattern has characteristic diffraction peaks at the following 2θ positions: 11.80°±0.2°, 14.04°±0.2°, 14.69°±0.2°, 24.81°±0.2°, 25.91°±0.2°, using Cu-Kα radiation.

10. The salts and hydrates and solvates thereof according to claim 9, wherein its X-ray powder diffraction pattern is shown in Figure 4.

11. The salts and hydrates and solvates thereof according to claim 9, wherein its thermogravimetric analysis (TGA) thermogram is shown in Figure 5, and its differential scanning calorimetry (DSC) thermogram is shown in Figure 6.

12. The salts and hydrates and solvates thereof according to any one of claims 1-3, wherein the salt is selected from the group consisting of adipate, wherein the adipate is a crystalline form (crystalline form B of the adipate), and its X-ray powder diffraction pattern has characteristic diffraction peaks at the following 2θ positions: 8.10°±0.2°, 12.18°±0.2°, 16.24°±0.2°, 17.68°±0.2°, 20.33°±0.2°, using Cu-Kα radiation.

13. The salts and hydrates and solvates thereof according to claim 12, wherein its X-ray powder diffraction pattern has characteristic diffraction peaks at the following 2θ positions: 10.68°±0.2°, 14.02°± 0.2°, 19.60°±0.2°, 20.95°±0.2°, using Cu-Kα radiation.

14. The salts and hydrates and solvates thereof according to claim 13, wherein its X-ray powder diffraction pattern is shown in Figure 7.

15. The salts and hydrates and solvates thereof according to claim 13, wherein its thermogravimetric analysis (TGA) thermogram is shown in Figure 8 and its differential scanning calorimetry (DSC) thermogram is shown in Figure 9.

16. A pharmaceutical composition comprising a therapeutically effective amount of the salts and hydrates and solvates thereof according to any one of claims 1 to 15, and a pharmaceutically acceptable carrier or excipient.

17. A use of the salts and hydrates and solvates thereof according to any one of claims 1 to 15, or the pharmaceutical composition according to claim 16, in the manufacture of a medicament for treating the diseases mediated by DPP1.

18. The use according to claim 17, wherein the disease mediated by DPP1 is selected from the group consisting of non-cystic fibrosis bronchiectasis, cystic fibrosis bronchiectasis, acute lung injury, airway obstructive disease, bronchiectasis, cystic fibrosis, asthma, emphysema and chronic obstructive pulmonary disease.
